(19) Europäisches Patentamt
European Patent Office
Office européen des brevets

(11) EP 2 070 914 B1

(12) # FASCICULE DE BREVET EUROPEEN

(45) Date de publication et mention de la délivrance du brevet:
**03.08.2011 Bulletin 2011/31**

(51) Int Cl.:
***C07D 239/48*** (2006.01) ***A61P 19/00*** (2006.01)
***A61K 31/505*** (2006.01)

(21) Numéro de dépôt: **09003174.1**

(22) Date de dépôt: **12.11.2003**

(54) **Nouveaux dérivés antagonistes du récepteur de la vitronectine, leur procédé de préparation, leur application comme médicaments et les compositions pharmaceutiques les renfermant**

Neue antagonistische Derivate des Vitronektinrezeptors, ihr Herstellungsverfahren, ihre Anwendung als Arzneimittel und die pharmazeutischen Zusammensetzungen, die sie enthalten

New antagonist derivatives of the vitronectin receptor, method for their preparation, their application as medicine and pharmaceutical compositions containing them

(84) Etats contractants désignés:
**AT BE BG CH CY CZ DE DK EE ES FI FR GB GR HU IE IT LI LU MC NL PT RO SE SI SK TR**

(30) Priorité: **19.11.2002 FR 0214429**

(43) Date de publication de la demande:
**17.06.2009 Bulletin 2009/25**

(62) Numéro(s) de document de la (des) demande(s) initiale(s) en application de l'article 76 CBE:
**03799611.3 / 1 565 467**

(73) Titulaire: **Galapagos SAS**
**93230 Romainville (FR)**

(72) Inventeurs:
- **Ruxer, Jean -Marie**
  **92130 Issy les Moulineaux (FR)**
- **Lefrancois, Jean-Michel**
  **93340 Le Raincy (FR)**
- **Heckmann, Bertrand**
  **91440 Bures sur Yvette (FR)**

(74) Mandataire: **Lord, Hilton David**
**Marks & Clerk LLP**
**90 Long Acre**
**London**
**WC2E 9RA (GB)**

(56) Documents cités:
**EP-A- 0 820 991** **EP-A- 0 933 367**
**WO-A-96/00574**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

Cette demande est une demande divisionnaire de la demande EP03799611.3.

La présente invention a pour objet de nouveaux dérivés antagonistes du récepteur de la vitronectine, leur procédé de préparation, leur application comme médicaments et les compositions pharmaceutiques les renfermant.

L'invention a pour objet les composés de formule (I) :

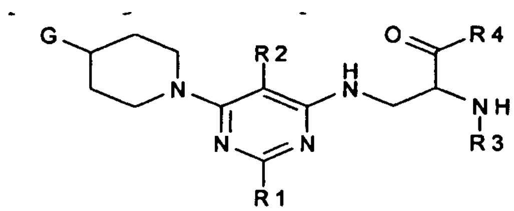

(I)

dans laquelle $R^1$, $R^2$, $R^3$, $R^4$ et G ont les significations indiquées plus bas ainsi que leurs sels physiologiquement acceptables. Les composés de formule (I) sont des composés ayant une activité pharmacologique et sont donc utilisables à titre de médicaments. Ce sont des antagonistes du récepteur de la vitronectine et des inhibiteurs de l'adhésion cellulaire et ils inhibent la résorption osseuse médiée par les ostéoclastes. Ils sont donc utiles pour le traitement thérapeutique ou prophylactique de maladies qui sont causées au moins en partie par une augmentation non désirée de la résorption osseuse, par exemple l'ostéoporose. L'invention de plus a pour objet les procédés de préparation des composés de formule (I), leur application, en particulier à titre de médicament et les compositions pharmaceutiques les renfermant.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. La majorité des désordres au niveau de l'os sont basés sur un équilibre perturbé entre la formation osseuse et la résorption osseuse. L'ostéoporose est caractérisée par une perte sèche de cette matrice osseuse. Un ostéoclaste mature activé résorbe l'os après adhésion à la matrice osseuse via la sécrétion d'enzyme protéolytique, et de protons à l'intérieur de la zone d'adhésion, aboutissant à des dépressions ou des creusements de la surface de l'os qui apparaissent au moment où l'ostéoclaste se détache de l'os.

Des études ont montré que la fixation de l'ostéoclaste sur l'os est médiée par des récepteurs : les intégrines. Les intégrines sont une superfamille de récepteurs médiant les processus d'adhésion cellule/cellule et plus particulièrement cellule/matrice, incluant notamment $\alpha_{IIb}\beta_3$ comme récepteur des plaquettes sanguines (fibrinogène) et $\alpha_v\beta_3$ comme récepteur de la vitronectine. Les peptides contenant le motif RGD ainsi que les anticorps anti $\alpha_v\beta_3$ sont connus pour leur capacité d'inhibition de la résorption de la dentine et d'empêchement de l'adhésion des ostéoclastes sur les matrices minéralisées (Horton et al. Exp. Cell. Res. (1991), 195, 368). Le peptide Echistatine, isolé du venin de serpent contenant également un motif RGD et décrit comme inhibiteur de l'adhésion des ostéoclastes à l'os est un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1411).

Le récepteur $\alpha_v\beta_3$ est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés de formule (I) selon l'invention.

En effet, les récepteurs $\alpha_v\beta_3$, exprimés au niveau de la membrane des ostéoclastes sont à la base du processus d'adhésion/résorption, contribuent à l'organisation du cytosquelette cellulaire, et sont impliqués dans l'ostéoporose. Les récepteurs $\alpha_v\beta_3$ exprimés au niveau des cellules du muscle lisse de l'aorte, stimulent leur migration vers la néointima, ce qui entraîne la formation de l'artériosclérose et la survenue de resténose post-angioplastique (Brown et al., cardiovascular Res. (1994), 28, 1815). Les cellules endothéliales secrètent des facteurs de croissance qui sont mitogènes pour l'endothélium et peuvent contribuer à la formation de nouveaux vaisseaux sanguins (Angiogenèse).

Les antagonistes de l'intégrine $\alpha_v\beta_3$ peuvent ainsi entraîner une régression des tumeurs cancéreuses en induisant l'apoptose des vaisseaux sanguins angiogéniques. (Brook et al. Cell (1994) 79, 1157).

Cheresh et al (Science 1995, 270, 1500) ont décrit des anticorps anti-$\alpha_v\beta_3$ ou des antagonistes du récepteur $\alpha_v\beta_3$ qui inhibent le processus d'angiogénèse induit par bFGF dans l'oeil de rat, une propriété qui peut être utilisée pour le traitement des rétinopathies, notamment du diabétique.

La demande de brevet WO-A-94/12181 décrit des systèmes aromatiques ou non aromatiques substitués et WO-A-94/08577 décrit des hétérocycles substitués comme antagonistes du récepteur de fibrinogène et inhibiteurs de l'agrégation plaquettaire. EP-A-528586 et EP-A-528587 décrivent des dérivés de la phénylalanine substitués par un aminoalkyle ou un hétérocycle et WO-A-95/32710 décrivent des dérivés aryliques comme inhibiteurs de la résorption

osseuse par les ostéoclastes. WO-A-96/00574 décrit des benzodiazépines et WO-A-96/00730 décrit des composés inhibiteurs du récepteur fibrinogène, en particulier benzodiazépines qui sont liés à un cycle à 5 chaînons azoté comme antagonistes du récepteur de la vitronectine. WO9800395 WO99/3245 et WO99/3762 décrivent des antagonistes du récepteur de la vitronectine dérivés de la tyrosine. EP0820991 revendique des dérivés de cycloalkyle comme antagonistes du récepteur de la vitronectine.

D'autres investigations ont permis de montrer que les dérivés de formule (I) présentent une forte activité comme antagonistes du récepteur de la vitronectine et de la résorption osseuse médiée via les ostéoclastes.

L'invention a pour objet les composés de formule (I) :

(I)

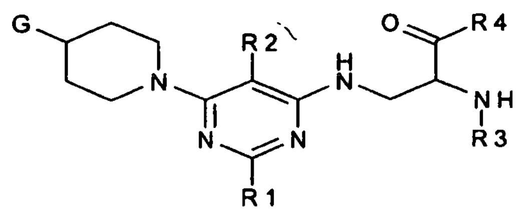

sous toutes leurs formes isomères, seules ou en mélange, ainsi que leurs sels d'addition physiologiquement acceptables, dans laquelle

- G représente 1,2,3,4-tétrahydro-1,8-naphtyridin-7-yle,
- $R^1$ représente méthyle,
- $R^2$ représente méthyle,
- $R^3$ représente benzyloxycarbonyle et
- $R^4$ représente OH ou t.butoxy.

Par halogène on entend fluor, chlore, brome ou iode.

Les atomes de carbone optiquement actifs contenus dans les composés de formule (I) peuvent indépendamment les uns des autres présenter la configuration R ou la configuration S.

Les composés de formule (I) peuvent avoir par exemple la formule :

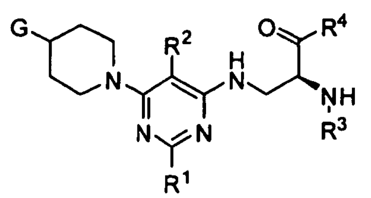

Les composés de formule (I) peuvent être sous la forme d'énantiomères purs ou de diastéréoisomères purs ou sous la forme d'un mélange d'énantiomères, par exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque.

L'invention comprend également toutes les formes tautomères des composés de formule (I).

Les diastéréoisomères, incluant les isomères E/Z peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, il s'agit par exemple des sels de métaux alcalins ou alcalino terreux tels que les sels de sodium, de potassium, de magnésium, de

calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine.

Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléfique, fumarique, tartarique, méthanesulfonique ou para toluène sulfonique.

Les composés de formule (I) qui comportent un groupe basique et un groupe acide peuvent être présents sous forme de Zwiterions (bétaines), qui sont également inclus dans la présente invention.

Le cas échéant un anion $Q^-$ physiologiquement acceptable peut être contenu dans les composés de formule (I) renfermant un groupement ammonium chargé. Il s'agit de préférence d'un anion monovalent ou d'un équivalent d'anion polyvalent d'un acide organique ou inorganique non toxique, physiologiquement acceptable et en particulier pharmaceutiquement acceptable, par exemple l'anion ou un équivalent d'anion d'un des acides cités plus haut utile pour la formation des sels d'addition.

$Q^-$ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et paratoluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation ou d'anion.

L'invention inclut également tous les sels des composés de formule (I) qui, à cause de leur faible acceptabilité physiologique, ne sont pas directement utilisables comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formés avec les alcools, et tous les dérivés des composés de formule (I), par exemple les esters, prodrogues et autres dérivés physiologiquement acceptables, ainsi que les métabolites des composés de formule (I).

L'invention a plus particulièrement pour objet les composés de formule (I) tels que définis plus haut dans laquelle $R_3$ est un groupement benzyloxycarbonyle, ainsi que leurs sels d'addition pharmaceutiquement acceptables.

L'invention a plus particulièrement pour objet les composés de formule (I) tels que définis plus haut dans laquelle $R_2$ est un radical méthyle ainsi que leurs sels d'addition pharmaceutiquement acceptables.

L'invention a plus particulièrement pour objet les composés de formule (I) tels que définis plus haut dans laquelle :

G représente :

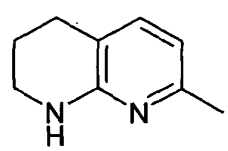

ainsi que leurs sels d'addition pharmaceutiquement acceptables.

L'invention a particulièrement pour objet les composés de formule (I) dont les noms suivent :

- 3-[[6-[4-(1,2,3,4-tétrahydro-1,8-naphthyridin-7-yl)-1-pipérdinyl]-2,5-diméthyl-4-pyrimidinyl]amino]-N-[(phénylméthoxy)carbonyl]alaninate de (1,1-diméthyléthyle)
- 3-[[6-[4-(1,2,3,4-tétrahydro-1,8-naphthyridin-7-yl)-1-pipérdinyl]-2,5-diméthyl-4-pyrimidinyl]amino]-N-[(phénylméthoxy)carbonyl]alanine

sous la configuration (R) ou la configuration (S) ou leurs mélanges, ainsi que leurs sels d'addition.

La présente invention a également pour objet un procédé de préparation des composés de formule (I). Les composés peuvent généralement être préparés, par exemple au cours d'une synthèse convergente par couplage de deux ou plusieurs fragments qui peuvent être dérivés par rétrosynthèse des composés de formule (I). Afin d'éviter que les groupes fonctionnels puissent mener à des réactions indésirables ou secondaires au cours de chaque étape de

synthèse, il peut être avantageux ou nécessaire au cours de la synthèse des composés de formule (I), d'introduire les groupes fonctionnels sous forme de précurseurs qui sont par la suite convertis en groupes fonctionnels désirés ou de bloquer temporairement ces groupes fonctionnels en mettant en oeuvre une stratégie de groupe protecteur appropriée à la synthèse qui est connue par l'homme de l'art (Greene, Wuts protective Group in Organic Synthesis, Wiley 1991).

Ainsi les composés de formule (I) peuvent être préparés, selon le schéma suivant :

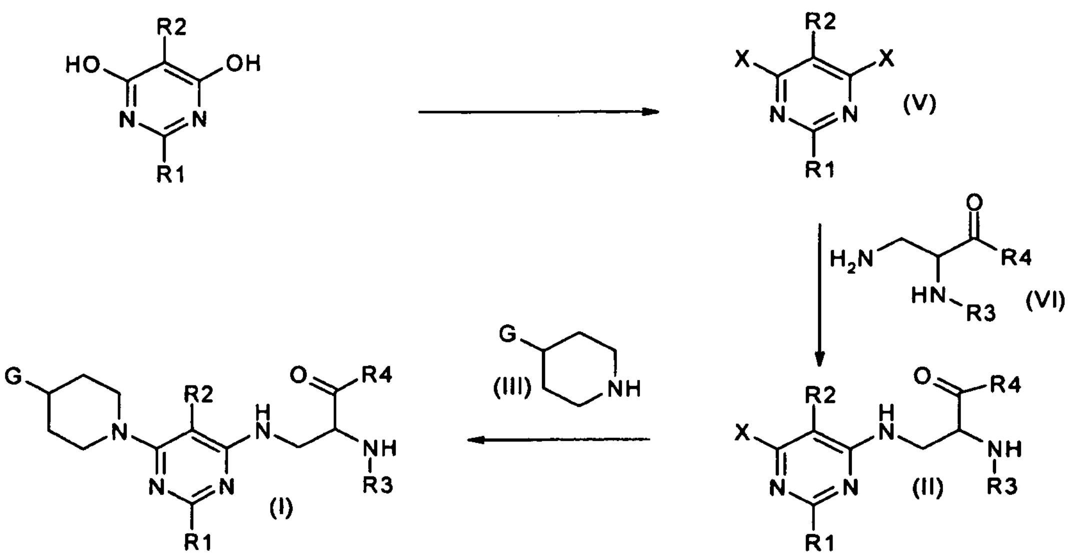

Les composés de formule(I) peuvent être préparés par un procédé de préparation dans lequel :

on fait réagir un composé de formule (II)

(II) 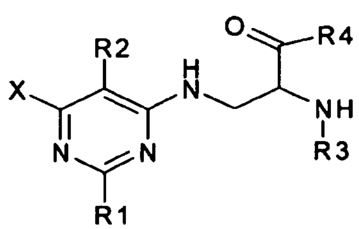

dans laquelle $R^1$, $R^2$, $R^3$ et $R^4$ sont tels que définis précédemment, et X représente un atome d'halogène.

a) avec un composé de formule (III)

(III) 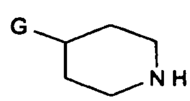

dans laquelle G est telle que définie précédemment en présence d'une base ou un réactif de couplage de métal de transition

b) puis le composé de formule (I) est soumis à l'hydrolyse et/ou à la salification.

Le mélange réactionnel est ensuite traité et si désiré le produit réactionnel est purifié selon les méthodes connues de l'homme du métier.

Les groupes protecteurs éventuellement présents dans les composés de formule (I) obtenus à partir des composés de formule (II) avec les composés de formule (III) sont ensuite éliminés par des procédés classiques; par exemple, les groupes tert-butyl ester sont convertis en acide carboxylique par traitement avec de l'acide trifluoroacétique, les groupes benzyles sont éliminés par hydrogénation ou encore les groupes fluorénylméthoxycarbonyle sont éliminés

en présence d'amine secondaire et d'autres réactions sont mises en oeuvre par des procédés standards, par exemple par des réactions d'acylation.

Les réactions d'hydrolyse afin d'obtenir un dérivé d'acide ($COR^4 = CO_2H$), ou d'estérification afin d'obtenir un ester ou une prodrogue (particulièrement $COR^4$ = alkyloxycarbonyle à partir de l'acide correspondant) s'effectuent selon les méthodes usuelles connues de l'homme du métier.

Notamment l'hydrolyse s'effectue en milieu acide, en présence d'acide trifluoracétique par exemple, dans un solvant organique halogéné comme le dichlorométhane par exemple.

Si nécessaire, la conversion en des sels physiologiquement acceptables s'effectue par des procédés connus de l'homme du métier.

Les composés de départs de formule (II) peuvent être préparés selon des procédés décrits dans la littérature ou encore sont accessibles par analogie. La préparation des composés de formule (II) est illustrée dans le schéma décrit plus haut, étant entendu que la présente invention n'est pas restreinte à ces synthèses ou ces produits de départ. Il n'y a pas de difficulté majeure pour l'homme du métier de prévoir des modifications des synthèses décrites dans notre demande pour la préparation d'autres composés de formule (II) selon l'invention.

Un autre procédé de préparation des composés de formule (II) est présenté dans lequel on fait réagir un composé de formule (V) :

(V)

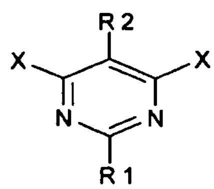

dans laquelle $R^1$ et $R^2$ sont tels que définis précédemment, et X représente un halogène, de préférence chlore avec un composé de formule (VI):

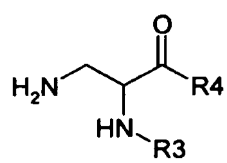

(VI)

dans laquelle $R^3$ et $R^4$ sont tels que définis précédemment, en présence d'une base forte.

En général, on utilise une base forte encombrée telle que la diisopropyléthylamine dans des conditions réactionnelles connues de l'homme du métier dans la mise en oeuvre de substitution nucléophile. De préférence on opère en présence de diméthylformamide et à la température de reflux. Par ailleurs le groupement COR4 représentera de préférence un groupement ester encombré tel que le groupement tertbutyloxycarbonyle.

L'invention a pour objet le procédé dans lequel les produits de formule (I) peuvent être préparés selon le schéma suivant :

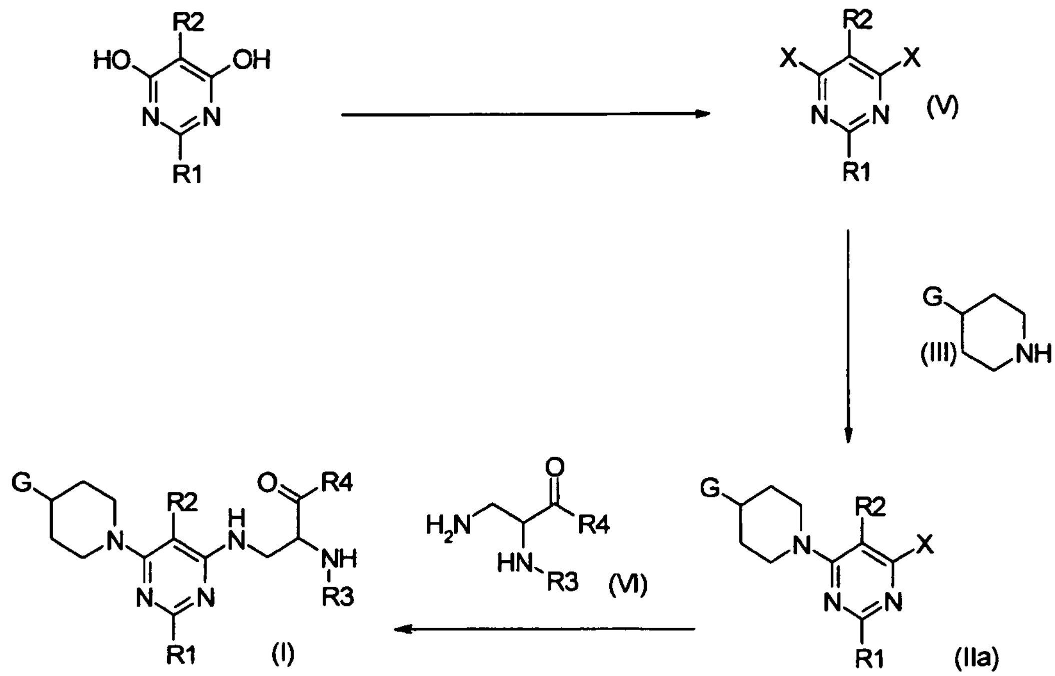

(V) (III) (VI) (I) (IIa)

Selon l'invention, le procédé de préparation des produits de formule (I) consiste dans

a) la réaction d'un produit de formule (IIa)

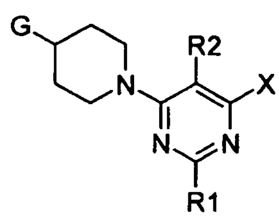

(IIa)

dans laquelle $R^1$, $R^2$, G et X sont tels que définis précédemment, avec un produit de formule (VI)

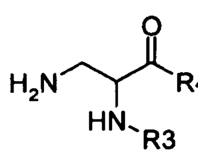

(VI)

dans laquelle $R^3$ et $R^4$ sont définis comme précédemment, soit en présence d'une base forte, soit par catalyse au palladium, pour obtenir un produit de formule (I)

b) puis le produit de formule (I) est éventuellement soumis à l'hydrolyse et/ou à la salification.

La réaction de la pyrimidine de formule (IIa) avec l'amine de formule générale (VI) s'effectue dans des conditions analogues aux conditions décrites précédemment pour la réaction d'une pyrimidine de formule (V) avec l'amine de formule générale (VI). Notamment, il est possible d'opérer dans la diisopropyléthylamine dans un solvant organique tel qu'un amide (diméthylacétamide, diméthylformamide par exemple), à une température comprise entre 90°C et la température de reflux du mélange réactionnel. Il est également possible d'opérer par catalyse au palladium (par exemple tris(dibenzylidèneacétone) dipalladium) en présence de fluorure de césium, à la température de reflux du mélange réactionnel. Il est entendu que les fonctions pouvant interférer avec la réaction sont protégées. La protection et la libération de ces fonctions s'effectue selon les méthodes habituelles qui n'altèrent pas le reste de la molécule.

Les dérivés de pyrimidine de formule (IIa) peuvent être préparés par action d'un produit de formule (III)

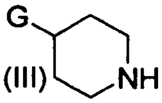

(III)

dans laquelle G est défini comme précédemment, sur un dérivé dihalogéné de la pyrimidine de formule (V)

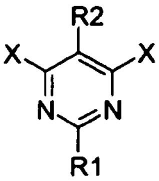

(V)

dans laquelle $R^1$, $R^2$ et X sont définis comme précédemment.

La réaction s'effectue avantageusement en présence d'une base forte encombrée, à la température de reflux du mélange réactionnel. On opère dans les conditions décrites ci-après dans les exemples et notamment en présence d'une amine encombrée comme la diisopropyléthylamine, dans un amide comme le diméthylacétamide par exemple. Il est entendu que les fonctions pouvant interférer avec la réaction sont protégées. La protection et la libération de ces fonctions s'effectue selon les méthodes habituelles qui n'altèrent pas le reste de la molécule.

Les composés de formule (I) sont des composés ayant une activité pharmacologique et peuvent ainsi être utilisés à titre de médicaments notamment dans le traitement ou la prévention des maladies de l'os, des maladies tumorales ainsi que des désordres cardiovasculaires.

La présente invention a donc pour objet les composés de formule (I) et/ou leurs sels physiologiquement acceptables à titre de médicament.

Les composés de formule (I) ainsi que leurs sels physiologiquement acceptables et leurs prodrugs peuvent être administrés aux animaux, de préférence aux mammifères et en particulier aux êtres humains comme médicaments à titre thérapeutique ou prophylactique.

Ils peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables ainsi que des supports et/ou additifs courants et pharmaceutiquement inertes.

Les compositions pharmaceutiques selon l'invention permettent une administration entérale ou parentérale qui renferment à titre de composé actif une dose efficace d'au moins un composé de formule (I) et/ou ses sels physiologiquement acceptables ainsi qu'un ou plusieurs supports pharmaceutiquement inertes, et/ou un ou plusieurs additifs usuels.

L'invention a donc pour objet les compositions pharmaceutiques renfermant un composé de formule (I) tel que défini précédemment ainsi qu'un ou plusieurs excipients.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire, par voie parentérale, par exemple sous forme de solutions injectables ou d'infusions, de microcapsules ou d'implants, par voie percutanée, par exemple sous la forme de pommade, de solutions, de pigments ou de colorants, par voie transdermique sous forme de patchs ou par d'autres voies telles que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I) et/ou leurs sels physiologiquement acceptables.

Pour la production de pilule, de comprimés, de comprimés enrobés et de capsule en gélatine dure, il est possible d'utiliser par exemple, du lactose, de l'amidon de maïs ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés

pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxylique et d'acide lactique. Les préparations pharmaceutiques contiennent normalement de 0,5 % à 90 % en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.

Elles peuvent également contenir deux ou plusieurs composés de formule (I) et/ou leurs sels physiologiquement acceptables. En outre, en plus d'au moins un ou plusieurs composés de formule (I) et/ou leurs sels physiologiquement acceptables, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

Les composés de formule (I) sont tout particulièrement des antagonistes des récepteurs de la Vitronectine et sont capables ainsi par exemple d'inhiber l'adhésion des ostéoclastes sur la surface de l'os et ainsi la résorption osseuse par les ostéoclastes.

L'action des composés de formule (I) peut être démontrée par exemple dans un test dans lequel l'inhibition de la liaison de la vitronectine aux cellules qui contiennent le récepteur de la vitronectine est déterminée. Des précisions sur ce test sont données plus bas. Comme antagonistes du récepteur de la vitronectine, les composés de formule (I) et leurs sels physiologiquement acceptables conviennent en général pour le traitement ou la prévention de maladies liées aux interactions entre les récepteurs de la vitronectine et leurs ligands, dans les processus d'interaction cellulecellule ou cellule-matrice ou qui peuvent être influencés par l'inhibition des interactions de ce type, pour soulager ou guérir lorsqu'une inhibition des interactions de ce type est désirée. Comme on l'a expliqué au début, une telle interaction joue un rôle important dans la résorption osseuse, dans l'angiogénèse ou dans la prolifération des cellules du muscle vasculaire lisse.

Les maladies de l'os dont le traitement ou la prévention nécessitent l'emploi des composés de formule (I), sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, et la maladie de Paget. En outre les composés de formule (I) peuvent être utilisés pour soulager, empêcher ou traiter les désordres de l'os qui sont causés par les traitements, par les glucocorticoïdes, les thérapies liées à la prise de stéroïdes ou de corticostéroïdes ou par les déficiences d'hormones sexuelles mâles ou femelles.

Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes. A côté de cette utilisation comme inhibiteur de la résorption osseuse médiée via les ostéoclastes, les composés de formule (I) et leurs sels physiologiquement acceptables sont utilisés comme inhibiteurs de la croissance tumorale ou des métastases cancéreuses, dans le traitement des désordres inflammatoires, pour le traitement ou la prévention des désordres cardiovasculaires, telles que l'artériosclérose ou la resténose, ou le traitement ou la prévention de néphropathie ou rétinopathie tel que par exemple la rétinopathie diabétique.

Les composés selon l'invention peuvent posséder également une activité vis-à-vis d'autres intégrines qui interagissent avec leur ligand via la séquence tripeptidique RGD ($\alpha_v\beta_1$, $\alpha_v\beta_5$, $\alpha_{IIb}\beta_3$), leur conférant des propriétés pharmacologiques utilisables pour traiter les pathologies associées à ces récepteurs.

Cette activité vis-à-vis des intégrines rend ainsi les composés de formule (I) utilisables dans la prévention ou le traitement de nombreuses maladies telles que celles mentionnées plus haut ou dans la revue de Dermot Cox DN§P 8(4) mai 1995, 197-205 dont le contenu est intégré dans la présente demande.

La présente invention a donc plus particulièrement pour objet un composé de formule (I) et/ou ses sels physiologiquement acceptables tel que défini plus haut à titre de médicament ayant une activité antagoniste du récepteur de la vitronectine.

La présente invention a donc plus particulièrement pour objet un composé de formule (I) et/ou ses sels physiologiquement acceptables et/ou ses prodrugs tel que défini plus haut à titre de médicament ayant une activité inhibitrice de la résorption osseuse ou pour le traitement ou la prévention de l'ostéoporose.

La présente invention a donc plus particulièrement pour objet un composé de formule (I) et/ou ses sels physiologiquement acceptables tel que défini plus haut à titre de médicament ayant une activité inhibitrice de la croissance tumorale ou des métastases cancéreuses.

La présente invention a donc plus particulièrement pour objet un composé de formule (I) et/ou ses sels physiologiquement acceptables tel que défini plus haut à titre de médicament ayant une activité anti-inflammatoire ou pour le traitement ou la prévention des désordres cardiovasculaires, la resténose, l'artériosclérose, les néphropathies ou rétinopathies.

La présente invention a également pour objet l'utilisation des composés de formule (I) et/ou leurs sels physiologiquement acceptables tels que définis plus haut pour la préparation de médicaments destinés à la prévention ou au traitement de l'ostéoporose.

La présente invention a également pour objet l'utilisation des composés de formule (I) et/ou leurs sels physiologiquement acceptables tels que définis plus haut pour la préparation de médicaments destinés à inhiber la croissance tumorale ou les métastases cancéreuses.

La présente invention a également pour objet l'utilisation des composés de formule (I) et/ou leurs sels physiologiquement acceptables tels que définis plus haut pour la préparation de médicaments destinés à la prévention ou au traitement des désordres cardiovasculaires, de la resténose, de l'artériosclérose, des néphropathies ou des rétinopathies.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100 mg/kg et de préférence de 0,1 à 50 mg/kg, en particulier de 0,1 à 5 mg/kg. Par exemple pour un adulte de 75 kg on pourra envisager une dose quotidienne variant de 0,3 à 0,5 mg/kg.

Dans le cas d'une administration par voie intraveineuse, la dose quotidienne varie approximativement de 0,01 à 100 mg/kg et de préférence de 0,05 à 10 mg/kg.

La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante. Mis à part l'utilisation des composés de formule (I) comme médicaments, on peut également envisager leur utilisation comme véhicule ou support de composés actifs afin de transporter ces composés actifs de manière spécifique vers un site d'action (Drug targeting, voir Targeted Drug Delivery, R. C. Juliano, Handbook of Experimental Pharmacology, Vol 100, Ed. Born, G. V. R. et al, Springer Verlag). Les composés actifs qui peuvent être transportés sont en particulier ceux utilisés pour le traitement ou la prévention des maladies citées plus haut.

Les composés de formule (I) et leurs sels peuvent également être employés comme agent de diagnostique, par exemple pour des méthodes in vitro ou comme auxiliaire dans des études biochimiques dans lesquelles on désire bloquer le récepteur de la vitronectine ou influencer les interactions cellules-cellules ou cellules-matrices. Ils peuvent en outre être utilisés comme intermédiaire pour la préparation d'autres composés, en particulier d'autres agents actifs, qui sont accessibles à partir des composés de formule (I), par exemple par modification ou introduction de radicaux ou de groupes fonctionnels.

## Exemples

Les produits ont été identifiés par spectre de masse (MS), infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

## Abréviations/noms chimiques éventuellement employés :

AcOEt : acétate d'éthyle; EDCI : chlorhydrate de 1-(3-diméthylaminopropyl)-3-éthyl-carbodiimide; DMF : diméthylformamide; DIPEA : Diisopropyléthylamine; MeOH : méthanol; TEA : triéthylamine; TFA : acide trifluoroacétique; THF : tétrahydrofurane; MCPBA : acide métachloroperoxybenzoique; DBU : 1,8-diazabicyclo[5.4.0] undec-7-ene; APTS : acide paratoluènesulfonique; DPPA : diphénylphosphorylazide; DMSO : diméthylsulfoxyde; Pd/C Palladium sur charbon; Boc : terbutoxycarbonyl; CBz : benzyloxycarbonyl; DCC 1,3-dicyclohexylcarbodiimide; TMSBr : bromotriméthylsilane; TMSI : iodure de triméthylsilane.

IR : Infrarouge; RMN : Résonnance Magnétique Nucléaire; SM : Spectre de Masse; ESP : Electrospray mode positif; ep.: Épaulement; F : fort; s : singulet; d : doublet; t : triplet; q : quadruplet; quint : quintuplet; 1 : large; m : multiplet ou massif; J : constante de couplage; Rf : facteur de rétention (chromatographie).

Il est entendu que dans les exemples qui suivent les produits des exemples 1 à 5 sont de forme racémique,

les produits des exemples 6 à 9, 11, et 13 à 41 ainsi que leurs esters précurseurs sont de forme (S) sur le centre asymétrique de la 3-amino alanine et les exemples 10 et 12 et le cas échéant leurs esters précurseurs sont de forme (R) sur le centre asymétrique de la 3-amino alanine.

**Préparation 1**

Synthèse de la 4,6-dichloro-5-éthyl-pyrimidine (Composé de formule (V).

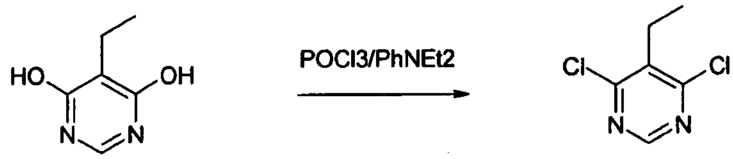


Un mélange de 5 g (35,7 mmoles) de 5-éthyl-4,6-dihydroxy-pyrimidine (commercialisée par Aldrich) dans 30 ml d'oxychlorure de phosphore est porté au reflux durant 1 heure. Après retour à température ambiante, on ajoute goutte à goutte un mélange de 4 ml de N,N-diéthylaniline dans 10 ml d'oxychlorure de phosphore et porte le tout au reflux pendant 4 heures. Après retour à température ambiante, le milieu réactionnel est versé sur un mélange de glace et d'eau. On extrait à l'acétate d'éthyle, lave les phases organiques avec de l'acide chlorhydrique 2N, sèche sur sulfate de magnésium et évapore à sec sous vide. On obtient 6 g (Rdt = 95 %) d'une huile marron de produit attendu utilisée telle quelle pour la suite.
CCM : Rf = 0,5 (silicagel, éluant : dichlorométhane-méthanol 90-10).

**Exemple de référence 1**

**3-[[5-éthyl-6-[4-(1,2,3,4-tétrahydro-1,8-naphthyridin-7-yl)-1-pipéridinyll-4-pyrimidinyljaminol-N-[(phénylméthoxy) carbonyl]alanine, bis(trifluoroacetate)**

**Etape a)**

Synthèse du 3-[(6-chloro-5-éthyl-4-pyrimidinyl)amino]-N-[(phénylméthoxy)carbonyl] alaninate de (1,1-diméthyléthyle).

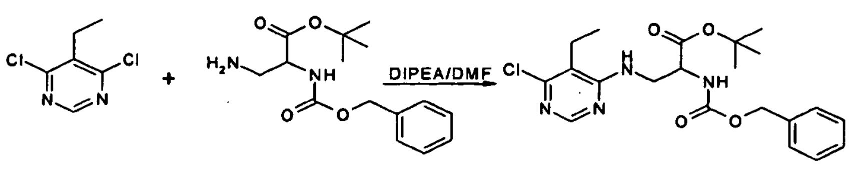


A un mélange de 3,8 g (21 mmoles) de 4,6-dichloro-5-éthyl-pyrimidine et de 4,4 g (15 mmoles) de 3-amino-N-[(phénylméthoxy)carbonyl]alaninate de (1,1-diméthyléthyle) (préparé selon J. Med. Chem.(2001), 44(8), 1158-1176) dans 50 ml de diméthylformamide, on additionne 10 ml de diisopropyléthylamine puis chauffe ce mélange à 120°C pendant 6 heures. On élimine alors la diméthylformamide sous vide et reprend le résidu par un mélange d'acétate d'éthyle, d'eau et une solution saturée de bicarbonate de sodium. La phase organique est décantée puis séchée sur sulfate de magnésium et le solvant éliminé par évaporation sous vide. Le résidu est chromatographié sur silicagel en éluant avec un gradient d'heptane 100 % jusqu'à heptane-acétate d'éthyle 50-50. On obtient 4,7 g (Rdt = 70 %) de produit attendu sous forme d'une huile.
**CCM :** Rf = 0,2 (silicagel, éluant : heptane-acétate d'éthyle 70-30)
**IR (CHC13)** : 3411 (NH); 1718 (C=O); 1571; 1498 cm-1 (Hétérocycle+Aromatique+Amide)
**1H-RMN (DMSO-d6) :** δ 1,02 (t, 3H, $CH_2$-$\underline{CH_3}$); 1,30 (s, 9H, tBu); 2,57 (q, 2H, $\underline{CH_2}$-$CH_3$); 3,74 (m, 2H, NH-$\underline{CH_2}$-CH-NH); 4,29 (ql, 1H, NH-$CH_2$-$\underline{CH}$-NH); 5,00 et 5,06 (syst. AB, 2H, O-$\underline{CH_2}$-Ph); 7,22 (tl, 1H, $\underline{NH}$-$CH_2$-CH-NH); 7,34 (m, 5H, Ph); 7,64 (dl, 1H, NH-$CH_2$-CH-$\underline{NH}$); 8,16 ppm (s, 1H, N=$\underline{CH}$-N)
**HPLC/SM :** (tr = 26 min) : 435 (MH+); 379 (MH-tBu+).

**Etape b)**

Synthèsedu3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

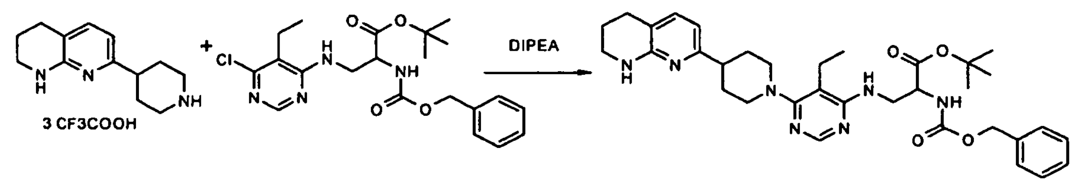


Un mélange de 1,68 g (3 mmoles) de 1,2,3,4-tétrahydro-7-(4-pipéridinyl)-1,8-naphthyridine, tris(trifluoroacetate) (préparé selon les brevets EP1065207 ou WO 0078317) et de 7,5 g (14,1 mmoles équivalents base) d'aminométhyl polystyrène (Polymer Labs 1,88 mmoles/g) dans 200 ml de solution de dichlorométhane-méthanol 50-50 est agité à température ambiante durant 1 heure. Le mélange est filtré, la résine lavée avec du méthanol et du dichlorométhane et le filtrat concentré à sec sous vide donnant 630 mg d'amine libre. On rajoute à ce résidu 651 mg (1,5 mmoles) de 3-[(6-chloro-5-ethyl-4-pyrimidinyl)amino]-N-[(phénylméthoxy) carbonyl]alaninate de (1,1-diméthyléthyle) et 2 ml de diisopropyléthylamine et porte au reflux durant 8 heures. Le milieu réactionnel est évaporé à sec sous vide et le résidu est repris par de l'acétate d'éthyle, de l'eau et une solution saturée de bicarbonate de sodium. La phase organique est séparée, séchée sur sulfate de magnésium et le solvant évaporé sous vide. Le résidu est chromatographié sur silicagel en éluant successivement avec du dichlorométhane pur, un mélange de dichlorométhane-méthanol 90-10 puis un mélange de dichlorométhane-méthanol-eau-acide acétique 90-10-1-1. On obtient 115 mg (Rdt = 12 %) de produit attendu sous forme d'huile. On récupère également 280 mg (43 %) de composé chloré de départ.
**CCM :** Rf = 0,55 (alumine, éluant : heptane-acétate d'éthyle 50-50).
**IR (CHC13)** : 3438 (NH 1717 (C=O); 1583; 1500 cm-1 (Hétérocycle+Aromatique+Amide).
**1H-RMM (DMSO-d6) :** δ 1,06 (t, 3H, $CH_2$-$\underline{CH_3}$); 1,31 (s, 9H, tBu): 1,77 (m, 2H, $CH_2$-$\underline{CH_2}$-$CH_2$-NH); 1,78 (m, 4H, $\underline{CH_2}$-CH-$\underline{CH_2}$) ; 2,43 (q, 2H, $\underline{CH_2}$-$CH_3$); 2, 51 (m, 1H, $CH_2$-$\underline{CH}$-$CH_2$); 2,61 (m, 2H, $\underline{CH_2}$-$CH_2$-$CH_2$-NH>; 2,82 et 3,44 (2m, 4H, $\underline{CH_2}$-$CH_2$-N-$CH_2$-$\underline{CH_2}$); 3,23 (m, 2H, $CH_2$-$CH_2$-$\underline{CH_2}$-NH); 3,72 (m, 2H, NH-$\underline{CH_2}$-CH-NH); 4,23 (m, 1H, NH-$CH_2$-$\underline{CH}$-NH); 5,03 (dl, 2H, $\underline{CH_2}$-Ph); 6,22 (m, 1H, $CH_2$-$CH_2$-$CH_2$-$\underline{NH}$); 6,30 et 7,05 (2m, 2H, H naphthyridine); 6,35 (m, 1H, $\underline{NH}$-$CH_2$-CH-NH); 7,34 (m, 5H, Ph); 7,60 (m, 1H, NH-$CH_2$-CH-$\underline{NH}$); 8,11 ppm (N=$\underline{CH}$-N).
**HPLC/SM :** (tr = 14 min) : 616 (MH+).

**Etape c)**

Synthèse de la 3-[[5-éthyl-6-[9-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

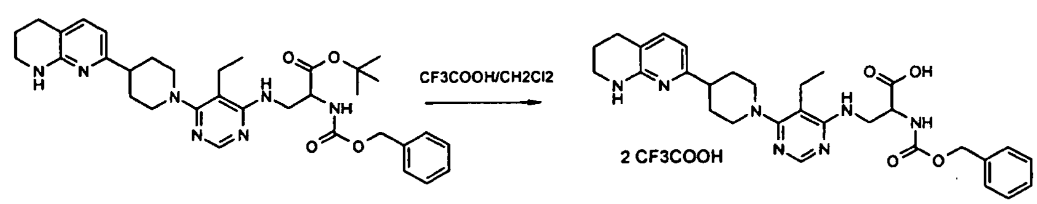


On agite 110 mg (0,18 mmoles) de 3-[[5-éthyl-6-[9-(1,2,3,4-tétrahydro-1,8-naphthyridin-7-yl)-1-pipéridinyl]-9-pyrimidinyl]amino]-N-[(phénylméthoxy)carbonyl]alaninate de (1,1-diméthyléthyle) dans 5 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le milieu réactionnel à sec sous vide. Le résidu est solubilisé dans le minimum de dichlorométhane avec un peu de méthanol puis versé sur de l'éther diisopropylique. Le précipité est filtré. On obtient 108 mg (Rdt = 76 %) de produit attendu sous forme d'un solide amorphe.
**CCM :** Rf = 0,33 (silicagel, éluant : dichlorométhane-méthanol-eau-acide acétique 90-10-1-1)

**IR ($CHCl_3$)** : 1677 (C=O); 1626; 1586; 1500 cm-1 (Hétérocycle+Aromatique+Amide).
**1H-RMN (DMSO-d6)** : δ 1,07 (t, 3H, CH2-CH3); 1,77 et 1,94 (2m, 4H, N-CH2-CH2-CH-); 1,84 (m, 2H, NH-CH2-CH2-CH2-); 2,45 (q, 2H, CH2-CH3); 2,75 (t, 2H, NH-CH2-CH2-CH2-); 2,85 (t, 1H, N-CH2-CH2-CH-); 2,98 et 3,53 (2m, 4H, N-CH2-CH2-CH-) ; 3,43 (m, 2H, N-CH2-CH2-CH2-) ; 3,61 et 3,85 (2m, 2H, NH-CH2-CH-NH); 4,32 (q, 1H, NH-CH2-CH-NH); 4,99 et 5,04 (syst AB, 2H, O-CH2-Ph); 6,67 (d, 1H, H naphthyridine); 7,22 (s1, 1H, NH-CH2-CH-NH); 7,35 (m, 5H, Ph); 7,60 (d, 1H, NH-CH2-CH-NH); 7,64 (d, 1H, H naphthyridine), 8,26 (s, 1H, N=CH-N); 8,29 ppm (s1, 1H, NH-CH2-CH2-CH2-).
**HPLC/SM :** (tr = 8,0 min) : 560 (MH+); 427 (MH-Naphthyridine+); 280 (M+2H++)

**Microanalyse :**

Théorie C=51,84 %; H=4,99 %; N=12,45 %;
Trouvé C=52,0 %; H=5,2 %; N=12,4 %;

**Exemple de référence 3**

**3-[[6-[4-(1,2,3,4-tetrahydro-1,8-naphthyridin-7-yl)-1-pipéridinyl]-5-méthyl-4-pyrimidinyl]amino]-N-[(phénylméthoxy) carbonyl]alanine**

**Stade a)**

Synthèse du 3-[(6-chloro-5-méthyl-4-pyrimidinyl)amino]-N-[(phénylméthoxy)carbonyl] alaninate de (1,1-diméthyléthyle).

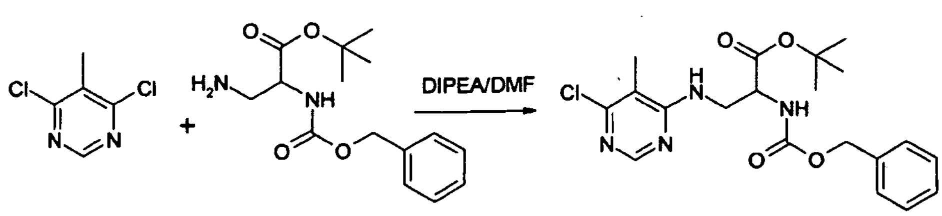


On chauffe à 120°C un mélange de 325 mg (2 mmoles) de 4,6-dichloro-5-méthyl-pyrimidine (commercialisée par SPECS), de 600 mg (2 mmoles) de 3-amino-N-[(phénylméthoxy)carbonyl] alaninate de (1,1-diméthyléthyle) (préparé selon J. Med. Chem. (2001), 44(8), 1158-1176) dans 3ml de diméthylformamide et 3 ml de diisopropyléthylamine pendant une nuit. On concentre le milieu réactionnel à sec sous vide et reprend le résidu par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous vide. Le résidu est chromatographié sur silicagel en éluant avec un gradient d'heptane (100 %) jusqu'à un mélange d'heptane-acétate d'éthyle (50-50). On obtient 450 mg (Rdt = 53 %) de produit attendu sous forme d'une huile.
**CCM :** Rf = 0,5 (silicagel, éluant : acétate d'éthyle-heptane(25-75)
**1H-RMN (CDCl3) :** δ 1, 48 (s, 9H, tBu) ; 2,08 (s, 3H, CH3) ; 3,80 et 3,97 (2m, 2H, NH-CH2-CH-NH); 4,50 (m, 1H, NH-CH2-CH-NH); 5,08 et 5,15 (syst AB, 2H, O-CH2-Ph); 5,86 (dl, 1H, NH-CH2-CH-NH); 6,12 (sl, 1H, NH-CH2-CH-NH); 7,35 (m, 5H, Ph); 8, 30 ppm (s, 1H, N=CH-N).
**SM (FAB) :** 421 (MH+); 365 (MH-tBu).

**Etape b)**

Synthèse du 3-[[6-[9-(1,2,3,9-tetrahydro-1,8-naphthyridin-7-yl)-1-pipéridinyl]-5-méthyl-4-pyrimidinyl]amino]-N-[(phénylméthoxy)carbonyl]alaninate de (1,1-diméthyléthyle).

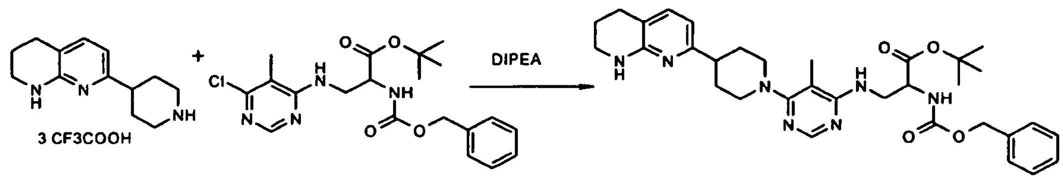


Un mélange de 370 mg (0,88 mmoles) de 3-[(6-chloro-5-méthyl-4-pyrimidinyl)amino]-N-[(phénylméthoxy)carbonyl] alaninate de (1,1-diméthyléthyle) et de 1,0 g (1,79 mmoles) de 1,2,3,4-tétrahydro-7-(4-pipéridinyl)-1,8-naphthyridine, tris (trifluoroacétate) (préparé selon les brevets EP1065207 ou WO 0078317) dans 1 ml de diisopropyléthylamine est chauffé à 120°C pendant 2 heures. On ajoute alors 10 ml de xylène et porte le milieu au reflux durant 4 heures. Le milieu réactionnel est repris par un mélange d'eau, d'acétate d'éthyle et d'une solution saturée de bicarbonate de sodium. La phase organique est décantée et la phase acqueuse est réextraite par de l'acétate d'éthyle. Les phases organiques regroupées sont séchées sur sulfate de magnésium et évaporées à sec sous vide et le résidu est chromatographié sur silicagel avec un gradient d'acétate d'éthyle 100 % jusqu'à un mélange d'acétate d'éthyle-méthanol-triéthylamine- dichlorométhane 50-10-10-50. On obtient 32 mg (Rdt = 6 %) de produit attendu et récupère 200 mg (54 %) de produit chloré de départ.

**CCM :** Rf = 0,6 (silicagel, éluant : acétate d'éthyle-dichlorométhane-méthanol 50-40-10)

**1H-RMN ($CDCl_3$) :** δ 1,49 (s, 9H, tBu) ; 1,97 (m, 2H, NH-CH2-CH2-CH2) ; 2,01 (s, 3H, CH3); 2,18 (m, 4H, N-CH2-CH2-CH); 2,79 (m, 2H, NH-CH2-CH2-CH2); 2,98 (m, 1H, N-CH2-CH2-CH); 3,39 et 3,89 (2m, 4H, N-CH2-CH2-CH); 3,52 (m, 2H, NH-CH2-CH2-CH2); 3,77 et 4,09 (2m, 2H, NH-CH2-CH-NH); 4,47 (m, 1H, NH-CH2-CH-NH); 5,13 (sl, 2H, O-CH2-Ph); 5,92 (s1, 1H, NH-CH2-CH-NH); 6,47 (dl, 1H, H naphthyridine); 7,37 (m, 5H, Ph); 7,38 (m, 1H, H naphthyridine); 8,41; 8,68 et 14,80 ppm (3s1, 3H, N=CH-N et mobiles).

**SM :** 602 (MH+); 546 (MH-tBu+).

**Etape c)**

Synthèse de la 3-[[6-[9-(1,2,3,4-tetrahydro-1,8-naphthyridin-7-yl)-1-pipéridinyl]-5-méthyl-4-pyrimidinyl]amino]-N-[(phénylméthoxy)carbonyl]alanine.

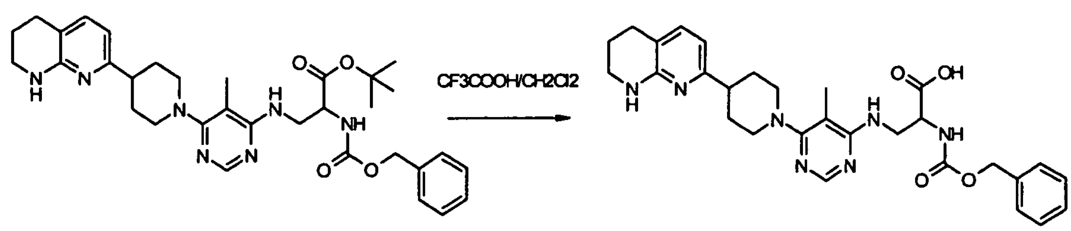


On agite à température ambiante un mélange de 38 mg (0,06 mmoles) de 3-[[6-[4-(1,2,3,4-tetrahydro-1,8-naphthyridin-7-yl)-1-pipéridinyl]-5-méthyl-4-pyrimidinyl]amino]-N-[(phényl méthoxy)carbonyl]alaninate de (1,1-diméthyléthyle) et de 2 ml d'acide trifluoroacétique dans 10 ml de dichlorométhane pendant 3 heures. On additionne alors 5 ml de toluène et évapore à sec le mélange. On obtient 26 mg (Rdt = 76 %) de produit attendu sous forme d'un solide amorphe.

**CCM :** Rf = 0,8 (silicagel, éluant : dichlorométhane-méthanol-eau-acide acétique 90-10-1-1).

**1H-RMN (DMSO-d6)** : δ 1,84 (m, 2H, NH-CH2-CH2-CH2); 1,92 (s, 3H, CH3); 1,75 et 1,91 (2m, 4H, N-CH2-CH2-CH); 2,74 (m, 2H, NH-CH2-CH2-CH2); 2,84 (m, 1H, N-CH2-CH2-CH); 2,92 et 3,67 (2m, 4H, N-CH2-CH2-CH); 3,44 (m, 2H, NH-CH2-CH2-CH2); 3,63 et 3,82 (2m, 2H, NH-CH2-CH-NH); 4,29 (m, 1H, NH-CH2-CH-NH); 5,02 (sl, 2H, O-CH2-Ph); 6,68 (d, 1H, H naphthyridine); 7,20 à 7,40 (m, 5H, Ph); 7,64 (dl, 1H, NH-CH2-CH-NH); 7,65 (d, 1H, H naphthyridine); 7,82 et 8,21 ppm (2s1, 3H, N=CH-N et mobiles).

**HPLC/SM :** (tr = 7min) 546 (MH+); 273 (M+2H++).

## Exemple de référence 5

**3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate d'éthyle, dichlorhydrate**

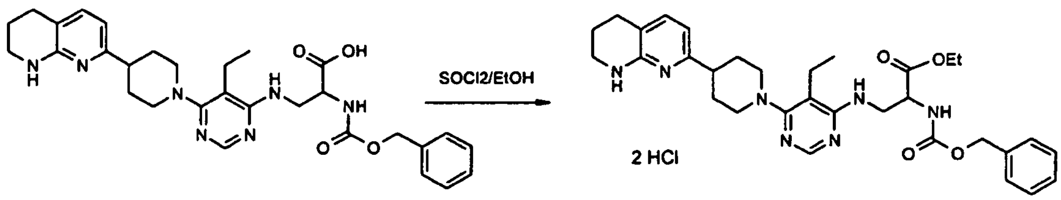


A 4 ml d'éthanol refroidi à -12°C sous atmosphère d'azote on ajoute 160 µl (2,19 mmoles) de chlorure de thionyle et agite le mélange pendant 30 minutes. On additionne alors une solution de 354 mg (0,45 mmoles) de 3-([5-éthyl-6-[4-(1,2,3,4-tétrahydro-1,8-naphthyridin-7-yl)-1-pipéridinyl]-4-pyrimidinyllaminol-N-[(phénylméthoxy)carbonyllalanine, bis(trifluoroacetate) dans 13 ml d'éthanol. Le mélange est agité 30 minutes à -12°C puis on laisse revenir à température ambiante et enfin chauffe à 40°C pendant 4 heures sous azote. On évapore le milieu réactionnel à sec sous vide et cristallise le résidu dans un mélange d'éther diisopropylique et de pentane et filtre le solide. On obtient 284 mg (Rdt = 95 %) de produit attendu sous forme d'un solide beige amorphe.
**CCM :** Rf = 0,4 (silicagel, éluant : dichlorométhane-méthanol-eau-acide acétique 90-10-1-1)
**IR ($CHCl_3$) :** 3400-3000(OH, NH); 1719(C=O); 1654; 1623; 1580; 1504 cm-1(C=C, C=N, aromatique)
**1H-RMN (DMSO-d6) :** δ 1,09 (t, 3H, CH2-CH3); 1,14 (t, 3H, O-CH2-CH3); 1,85 (m, 2H, NH-CH2-CH2-CH2); 1,85 et 2,00 (2m, 4H, N-CH2-CH2-CH); 2,48 (m, 2H, CH2-CH3); 2,75 (t, 2H, NH-CH2-CH2-CH2); 2,92 (m, 1H, N-CH2-CH2-CH); 3,03 et 3,57 (2m, 4H, N-CH2-CH2-CH); 3,44 (t, 2H, N-CH2-CH2-CH2); 3,76 et 3,87 (2m, 2H, NH-CH2-CH-NH); 4,07 (q, 2H, O-CH2-CH3); 4,39 (q, 1H, NH-CH2-CH-NH); 5,03 (s, 2H, O-CH2-Ph); 6,62 (d, 1H, H naphthyridine); 6,86 (sl, 1H, NH-CH2-CH-NH); 7,30 (m, 5H, Ph); 7,33 (sl, 1H, NH-CH2-CH-NH); 7,58 (d, 1H, H naphthyridine); 8,20 ppm (s, 1H, N=CH-N).
**HPLC/SM :** (tr = 6,2min) 588 (MH+); 454 (MH-COOCH2Ph+); 437 (MH-NHCOOCH2Ph+).

## Exemple de référence 6

Synthèse de la 4,6-dihydroxy-5-méthyl-pyrimidine.

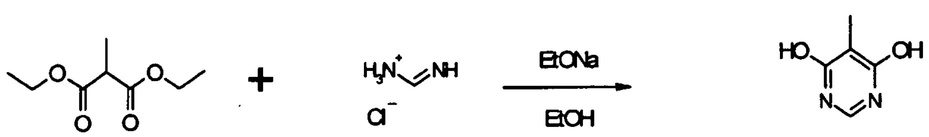


A une solution de 7,5 g (94 mmoles) de chlorhydrate de formamidine dans 250 ml d'éthanol refroidie à 0°C on ajoute 102 ml (282 mmoles) d'une solution d'éthylate de sodium à 21% dans l'éthanol et agite le mélange pendant 30 minutes; on additionne alors une solution de 13 ml (94 mmoles) de méthyl malonate de diéthyle dans 50 ml d'éthanol et agite une nuit à température ambiante. On évapore le mélange réactionnel à sec sous pression réduite (2 kPa) et reprend le résidu par de l'acétate d'éthyle, de l'eau et de l'acide acétique pour amener le pH à 6. Le précipité est filtré puis lavé successivement par de l'eau, de l'isopropanol, de l'éther diéthylique et enfin du pentane. On obtient 7 g (Rdt = 60%) de produit attendu sous forme d'un solide beige.
**CCM** : Rf = 0,20 (silicagel, éluant : acétate d'éthyle-dichlorométhane-méthanol 50-40-10)
**1H-RMN (DMSO d6) :** δ ppm 1,73(s, 3H, C-$CH_3$); 7,88 (s,N=CH-N).
**SM :** 127 (MH+); 125 (M-H-).

Synthèse de la 4,6-dibromo-5-méthyl-pyrimidine.

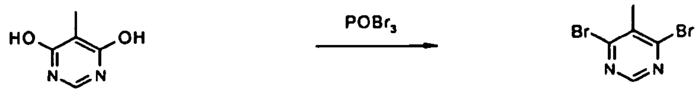


Un mélange de 6 g (47,6 mmoles) de 5-méthyl-4,6-dihydroxy-pyrimidine dans 18 g d'oxybromure de phosphore est porté à 200°C durant 3 heures. Après retour à température ambiante, le mélange réactionnel est repris par un mélange d'eau glacée de bicarbonate de sodium et extrait à l'acétate d'éthyle, lave les phases organiques avec de l'eau, sèche sur sulfate de magnésium et évapore à sec sous pression réduite (2 kPa). On obtient 9 g (Rdt = 75 %) d'un solide beige.
**CCM :** Rf = 0,27 (silicagel, éluant : dichlorométhane-pentane 50-50) **1H-RMM (CDCl3) :** δ ppm 2,58(s, 3H, C-$\underline{CH_3}$); 8,51 (s, N=$\underline{CH}$-N) .
**SM :** 253-255 (MH+).

Synthèse du de 6-[4-(1,2,3,4-tetrahydro-1,8-naphthyridin-7-yl)-1-pipéridinyl]-5-méthyl-4-bromo-pyrimidine :

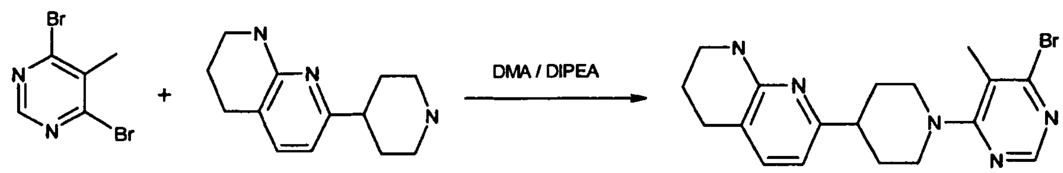


Dans un monocol contenant 8 g (36,8 mmoles) de 4-(1,2,3,4-tetrahydro-1,8-naphthyridin-7-yl)-1-pipéridine libérée de son sel, on ajoute 70 ml diméthylacétamide, 7,56 g (30 mmoles) de 4,6-dibromo-5-méthyl-pyrimidine mis en solution dans 40 ml de diméthylacétamide et 14 ml de diisopropyléthylamine. Ce mélange est chauffé à 140°C pendant 4 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous pression réduite (2 kPa). Le solide obtenu est lavé plusieurs fois à l'éther diisopropylique puis au pentane, on obtient 7.7 g du produit attendu sous la forme d'une poudre marron clair. Le filtrat est concentré à sec puis chromatographié sur silicagel en éluant avec un gradient d'acétate d'éthyle (100 %) puis d'acétate d'éthyle- méthanol (95-5). On obtient 533 mg de produit attendu sous la forme d'une poudre jaune. (Rdt global = 70 %)

Préparation de la naphtyridine sous forme d'amine libre :

22 g de naphtyridine est déplacée de son sel par 6 équivalents massiques de résine basique amberlyst A21 (résine de type R-$NMe_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 8 g (36,8 mmoles) de naphtyridine libre.
**CCM :** Rf = 0,33 (silicagel, éluant : acétate d'éthyle (100%)).
**1H-RMN (DMSO-d6) :** δ 1,7 à 1,85 (m, 6H, NH-<u>CH2</u>-CH2-CH2, N-CH2-<u>CH2</u>-CH-<u>CH2</u>); 2,23 (s, 3H, CH3-); 2,60 (m, 3H, CH2-<u>CH</u>-CH2, NH-CH2-CH2-<u>CH2</u>); 3,23 (m, 2H, NH-CH2-CH2-<u>CH2</u>): 2,97 et 3,92 (2m, 4H, CH2-<u>CH2</u>-N-<u>CH2</u>-CH2); 6,3 et 7,05 (2d, 2H, <u>CH</u>=<u>CH</u> naphthyridine); 8,28 (s, 1H, N=<u>CH</u>-N).
**SM (FAB) :** 388(M); 389(MH+)

Synthèse du 3-[[5-méthyl-6-[4-(1,2,3,9-tétrahydro-1,8-naphthyridin-7-yl)-l-pipéridinyll-4-pyrimidinyl]amino]-N-[(phénylméthoxy)carbonyl]alaninate de (1,1-diméthyléthyle).

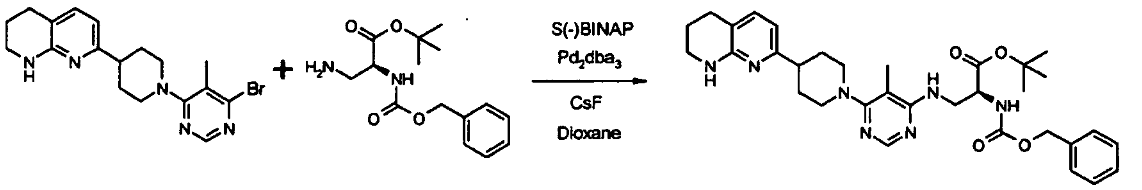


On chauffe au reflux un mélange de 620 mg (1,6 mmoles) de 4-bromo-5-méthyl-6-[9-(1,2,3,4-tétrahydro-1,8-naphthyridin-7-yl)-1-pipéridinyl]-pyrimidine, de 565 mg (1,92 mmoles) de 3-amino-N-[(phénylméthoxy)carbonyl]alaninate de (1,1-diméthyléthyle) (préparé selon J. Med. Chem.(2001), 44(8), 1158-1176), de 340 mg (2,25 mmoles) de fluorure de césium, de 73 mg (0,08 mmoles) de tris(dibenzylidèneacétone) dipalladium(0), de 100 mg (0,16 mmoles) de S(-)2,2'-bis(diphényl-phosphino)-1,1'-binaphtyl dans 50 ml de dioxane pendant 5 heures. On refroidit puis ajoute à nouveau 280 mg (0,96 mmoles) de 3-amino-N-[(phénylméthoxy)carbonyl]alaninate de (1,1-diméthyléthyle), 340 mg (2,25 mmoles) de fluorure de césium, 73 mg (0,08 mmoles) de tris(dibenzylidèneacétone) dipalladium(0),et 100 mg (0,16 mmoles) de S(-)2,2'-bis(diphényl-phosphino)-1,1'-binaphtyl et chauffe au reflux pendant 5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié une première fois sur silicagel en éluant avec un gradient d'acétate d'éthyle-chlorure de méthylène-triéthylamine-méthanol de 50-50-0-0 à 50-50-2-2. Le produit obtenu est chromatographié une deuxième fois sur alumine en éluant avec un mélange d'heptane-chlorure de méthylène 50-50 puis à l'acétate d'éthyle-éther diisopropylique 50-50. On obtient 360 mg (Rdt = 37%) de produit attendu sous forme de solide amorphe blanc.
**CCM :** Rf = 0,20 (silicagel, éluant : chlorure de méthylène-méthanol 95-5).
**IR (CHC13)** : 3435 (NH); 1717 (C=O); 1583,1555,1501 cm-1 (Hétérocycle+Aromatique+Amide).
**1H-RMN (CDCl3) :** δ 1,47 (s, 9H, tBu); 1,94 (s, 3H, C-$CH_3$); 1,75 à 2, 05 (m, 6H, $CH_2$-$CH_2$-$CH_2$-NH et $CH_2$-CH-$CH_2$); 2,60 à 2,77 (m, 3H, $CH_2$-CH-$CH_2$ et $CH_2$-$CH_2$-$CH_2$-NH); 2,93 et 3,90 (2m, 4H, $CH_2$-$CH_2$-N-$CH_2$-$CH_2$); 3,42 (m, 2H, $CH_2$-$CH_2$-$CH_2$-NH); 3,66 (m, 2H, NH-$CH_2$-CH-NH); 4,45 (m, 1H, NH-$CH_2$-CH-NH); 4,95, 5,25 et 6,20 (3m, 3H $CH_2$-$CH_2$-$CH_2$-NH NH-$CH_2$-CH-NH, NH-$CH_2$-CH-NH); 5,12 (s, 2H, $CH_2$-Ph); 7,35 (m, 5H, Ph); 8,29 ppm (s,N=CH-N).
**SM :** 602 (MH+); 546 (MH-tBu+); 412 (MH-COOCH2Ph+).
[$\alpha_{D]}$ (0,625 % EtOH) : -4,5°

Synthèse de la 3-[[5-méthyl-6-[4-(1,2,3,4-tétrahydro-1,8-naphthyridin-7-yl)-1-pipéridinyl]-9-pyrimidinyl]amino]-N-[(phénylméthoxy)carbonyl]alanine, bis(trifluoroacetate).

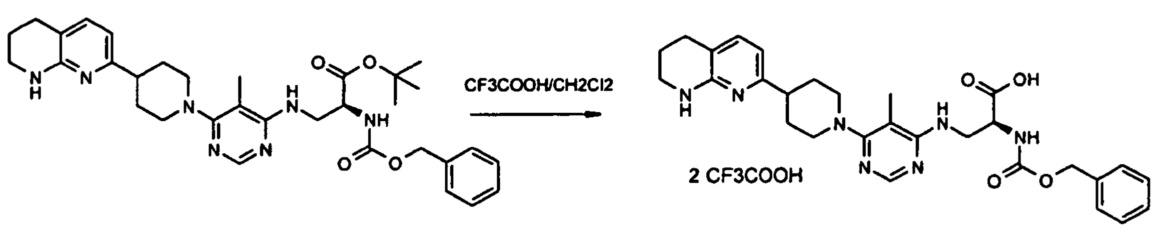


On agite 350 mg (0,58 mmoles) de 3-[[5-méthyl-6-[4-(1,2,3,4-tétrahydro-1,8-naphthyridin-7-yl)-1-pipéridinyl]-9-pyrimidinyl]amino]-N-[(phénylméthoxy)carbonyl]alaninate de (1,1-diméthyléthyle) dans 15 ml de dichlorométhane avec 3 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 330 mg (Rdt = 73% exprimé en ditrifluoroacétate) de produit attendu sous forme d'un solide blanc.
**CCM :** Rf = 0,44 (silicagel, éluant : dichlorométhane-méthanol-eau-acide acétique 90-10-1-1)
1H-RMN (CDC13) : δ 1, 96 (s, 3H, C-$CH_3$); 1,80 à 2,05 (m, 6H, $CH_2$-$CH_2$-$CH_2$-NH et $CH_2$-CH-$CH_2$); 2,76 (t, 2H, NH-CH2-CH2-CH2-); 2,96 (t, 1H, N-CH2-CH2-CH-); 3,21 et 3,83 (2m, 4H, N-CH2-CH2-CH-); 3,50 (m, 2H, N-CH2-CH2-CH2-); 3,83 et 4,05 (2m, 2H, NH-CH2-CH-NH); 4,54 (q, 1H, NH-CH2-CH-NH); 5,50 (s, 2H, O-CH2-Ph); 6,40 (d, 1H, H naphthyridine); 6,55 (dl, 1H, NH); 7,28 (m, 5H, Ph); 7,45 (ml, 1H, NH); 8,22 (d, 1H, H naphthyridine), 8,22 (s, 1H, N=CH-N); 9,62 ppm (s1, 1H).
**SM :** 546 (MH+); 412 (MH-COOCH2Ph+); 544- (M-H-); 436- (544-OCH2Ph-); 1089- (2M-H-)
[$\alpha_D$] (0,60% MeOH) : -14,0.

**Exemple de référence 7**

Préparation de la naphtyridine sous forme d'amine libre :

22 g de naphtyridine est déplacée de son sel par 6 équivalents massiques de résine basique amberlyst A21 (résine de type R-$NMe_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 8 g (36,8 mmoles) de naphtyridine libre.
**CCM :** Rf = 0,33 [silicagel, éluant : acétate d'éthyle (100%)]
**1H-RMN (DMSO-d6) :** δ 1,7 à 1,85 (m, 6H, NH-CH2-CH2-CH2, N-CH2-CH2-CH-CH2) ;2,23 (s, 3H, CH3-); 2,60 (m, 3H, CH2-CH-CH2, NH-CH2-CH2-CH2); 3,23 (m, 2H, NH-CH2-CH2-CH2); 2,97 et 3,92 (2m, 4H, CH2-CH2-N-CH2-CH2); 6,3 et 7,05 (2d, 2H, CH=CH naphthyridine); 8,28 (s, 1H, N=CH-N).
**SM (FAB) :** (tr = 0,50 min et 2,80 min): 388(M); 389(MH+)

Synthèse du N-(alpha)-Z-L,2-3-diaminopropionate d'éthyle.

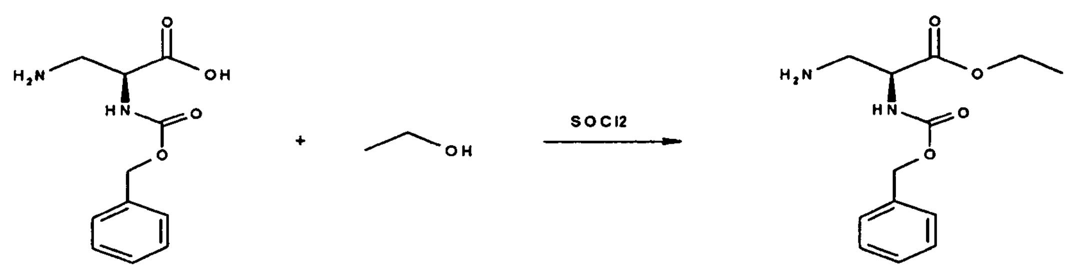


Dans un mono col contenant 26 ml d'éthanol absolu, on ajoute goutte à goutte et à 0°C, 12 ml (168 mmoles) de chlorure de thionyle. Ce mélange est agité pendant une vingtaine de minutes à TA puis on y ajoute par petites quantités 2 g (8,4 mmoles) d'acide N-(alpha)-Z-L,2-3-diamino-propionique, un trouble blanc apparaît.
Le mélange réactionnel est alors porté au reflux (78°C) pendant 2 heures (après quelques minutes de chauffage, la solution devient limpide).
Après refroidissement la solution est concentrée à sec, on obtient un liquide jaune auquel on ajoute de l'éther isopropylique. Le produit attendu prend en masse, on ôte alors le surnageant et le résidu est repris par un mélange d'eau, d'acétate d'éthyle et d'une solution de bicarbonate de sodium saturée. La phase organique est séparée et la phase aqueuse de nouveau extraite à l'acétate d'éthyle. Les phases organiques regroupées sont séchées sur sulfate de magnésium puis le solvant évaporé sous vide. On obtient 1,4 g d'une huile jaune pâle, il s'agit de l'ester sous la forme d'amine libre (Rendement = 63 %).
**CCM :** Rf = 0,41 [Silicagel, éluant : chlorure de méthylène-méthanol (85-15)et 2% d'eau-acide acétique(1-1).
**1H-RMN (CDC13):** δ 1,33 (t, 3H, CH3-CH2-O); 2,53 (m, 2H, NH2-CH2-CH) 3,15 (m, 2H, NH2-CH2-CH); 4,22 (q, 2H, CH3-CH2-O); 4,42 (m, 1H, NH2-CH2-CH-NH); 5,85 (m,1H, CH-NH-CO); 5,15 (s, 2H, O-CH2-Ph); 7,35 (m, 5H, Ph)
**SM (FAB) :** (tr = 0,51 min et 1,2 min) : 267(MH+); 533(2MH+)
[$\alpha$]$_D$ **(CHC13) =** +6,336

Synthèse du 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'éthyle.

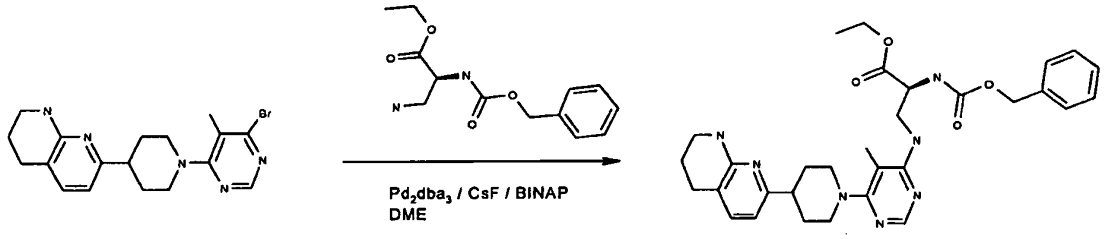


Un mélange de 1,9 g (4,9 mmoles) de 6-[4-(1,2,3,4-tetrahydro-1,8-naphthyridin-7-yl)-1-pipéridinyl]-5-méthyl-4-bromo-pyrimidine et de 1,62 g (6,08.mmoles) de 3-amino-N-[(phénylméthoxy)carbonyl] alaninate d'éthyle, en présence de 1,06 g (6,86 mmoles) de fluorure de cesium, de 310 mg (490 μmoles)de (2,2'-bis(diphénylphosphino)-1,1'-binaphthyl et de 230 mg (245 μmoles) de tris(dibenzylidèneacétone) dipalladium(o) dans 40 ml de 1,2-diméthoxyéthane est chauffé au reflux pendant 4 heures. Le mélange réactionnel est ensuite ramené à température pour l'ajout de 230 mg (245 μmoles) de tris(dibenzylidèneacétone)dipalladium(o), puis on porte de nouveau au reflux pendant 16 heures.

Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur silicagel par l'acétate d'éthyle 100%. On obtient 1,15 g de produit attendu sous la forme d'une huile jaune pâle (rendement = 40%)

**CCM :** Rf = 0.16 (silicagel éluant : acétate d'éthyle 100 %

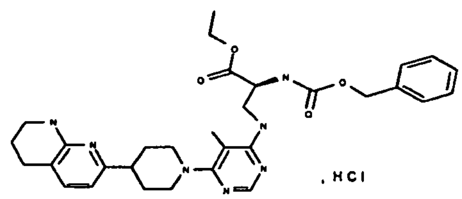

, HCl

M W =610.15
M F =C31H39N7O4,HCl

**1H-RMN (CDCl3) :** δ 1,25 (t, 3H, CH3-CH2-O); 1,80 à 2,05 (m, 9H, NH-CH2-CH2-CH2, N-CH2-CH2-CH-CH2, CH3-C=C-); 2,72 (m, 3H, CH2-CH-CH2, NH-CH2-CH2-CH2); 2,95 et 3,93 (2m, 4H, CH2-CH2-N-CH2-CH2); 3,45 (m, 2H, NH-CH2-CH2-CH2); 3,68 (d, 2H, NH-CH2-CH-NH) 4,23 (t, 2H, CH3-CH2-O), 4,45 (m, 1H, NH-CH2-CH-NH); 5,10 et 5,17 (syst AB, 2H, O-CH2-Ph); -6,41 et 7,18 (2d, 2H, CH=CH naphthyridine); 7,35 (m, 5H, Ph); 8,27 (s, 1H, N=CH-N).

**HPLC/SM :** (tr = 0,48 min et 2,9 min): 574 (MH+); 440 [MH - Z+]; 287 [MH - (tBu - Z+)]

**$[\alpha]_D$** (CHCl3) = + 0,788

Formation du Chlorhydrate:

1,15 g de 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'éthyle sont solubilisés dans un minimum de chlorure de méthylène. Cette solution est coulée sur de l'éther éthylique, la solution doit rester limpide. On ajoute ensuite goutte à goutte, sous agitation, 2,65 ml d'HCl 2N dilué dans un peu d'éther éthylique. Le chlorhydrate prend en masse, on ôte le surnageant, puis le résidu est cristallisé dans l'éther isopropylique. On obtient alors un solide qui est filtrée, rincée à l'éther puis au pentane. Après séchage, on obtient 890 mg d'une poudre jaune pâle. (Rendement = 73%)

## Exemple de réference 8

Préparation de la naphtyridine sous forme d'amine libre :

22 g de naphtyridine est déplacée de son sel par 6 équivalents massiques de résine basique amberlyst A21 (résine de type R-NMe$_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 8 g

(36,8 mmoles) de naphtyridine libre.
**CCM :** Rf = 0,33 [silicagel, éluant : acétate d'éthyle (100%)]
**1H-RMN (DMSO-d6) :** δ 1,7 à 1,85 (m, 6H, NH-CH2-CH2-CH2, N-CH2-CH2-CH-CH2) ;2,23 (s, 3H, CH3-); 2,60 (m, 3H, CH2-CH-CH2, NH-CH2-CH2-CH2); 3,23 (m, 2H, NH-CH2-CH2-CH2); 2,97 et 3,92 (2m, 4H, CH2-CH2-N-CH2-CH2); 6,3 et 7,05 (2d, 2H, CH=CH naphthyridine); 8,28 (s, 1H, N=CH-N).
**SM (FAB) :** (tr=0,50 min et 2,80 min): 388(M); 389(MH+)

Synthèse du N-(alpha)-Z-L,2-3-diaminopropionate d'isopropyle.

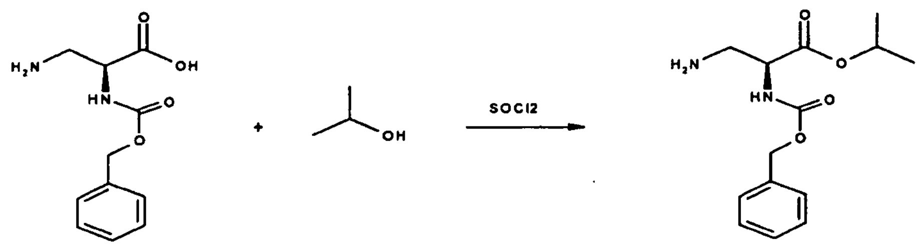


Dans un monocol contenant 125 ml d'isopropanol, on ajoute goutte à goutte et à 0°C, 46 ml (840 mmoles) de chlorure de thionyle. Ce mélange est agité pendant une vingtaine de minutes à TA puis on y ajoute par petites quantités 10 g (42 mmoles) d'acide N-(alpha)-Z-L,2-3-diamino-propionique, un trouble blanc apparaît.

Le mélange réactionnel est alors porté au reflux (78°C) pendant 2 heures (après quelques minutes de chauffage, la solution devient limpide).

Après refroidissement la solution est concentrée à sec, on obtient un liquide jaune auquel on ajoute de l'éther isopropylique. Le produit attendu prend en masse, on ôte alors le surnageant et le résidu est repris par un mélange d'eau, d'acétate d'éthyle et d'une solution de bicarbonate de sodium saturée. La phase organique est séparée et la phase aqueuse de nouveau extraite à l'acétate d'éthyle. Les phases organiques regroupées sont séchées sur sulfate de magnésium puis le solvant évaporé sous vide. On obtient 7,4 g d'une poudre blanche, il s'agit de l'ester sous la forme d'amine libre (Rendement = 42 %).
**CCM :** Rf = 0.5 [Silicagel, éluant : chlorure de méthylène-méthanol (85-15)et 2 % d'eau-acide acétique(1-1).
**1H-RMN (CDCl3) :** δ 1,25 (d, 6H, CH3-CH-CH3); 1,60 (m, 2H, NH2-CH2-CH) 3,10 (m, 2H, NH2-CH2-CH) ; 4,35 (m, 1H, NH2-CH2-CH-NH) 5,10 (m, 1H, CH3-CH-CH3); 5,15 (s, 2H, O-CH2-Ph) ;5,2 (m,1H, CH-NH-CO), 7,37 (m, 5H, Ph)
**SM (FAB) :** (tr = 0,52 min et 2,09 min) : 281(MH+); 239(MH-ipr+)
$[\alpha]_D$ **(CHCl3) = +15,02**

Synthèse du 3-[[6-[4-(1,2,3,4-tetrahydro-1,8-naphthyridin-7-yl)-1-pipéridinyl]-5-méthyl-4-pyrimidinyl]amino]-N-[(phénylméthoxy)carbonyl]alaninate d'isopropyle.

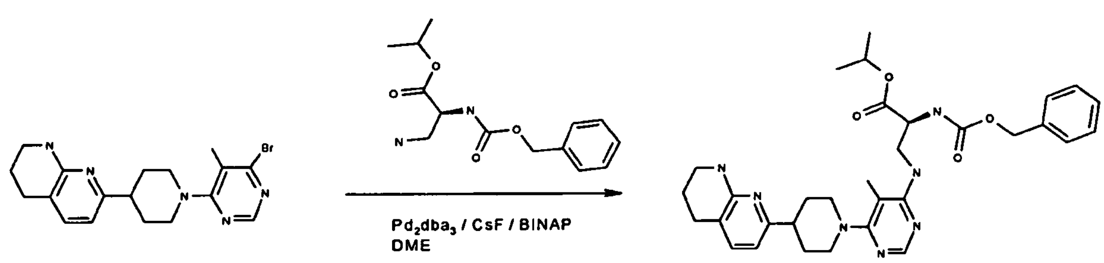


Un mélange de 1,5 g (3,86 mmoles) de 6-[4-(1,2,3,4-tetrahydro-1,8-naphthyridin-7-yl)-1-pipéridinyl]-5-méthyl-4-bromo-pyrimidine et de 1,30 g (4,63.mmoles) de 3-amino-N-[(phénylméthoxy)carbonyl] alaninate d'isopropyle, en présence de 825 mg (5,41 mmoles) de fluorure de cesium, de 241 mg (386 μmoles)de (2,2'-bis(diphénylphosphino)-1,1'-binaphthyl et de 180 mg (193 μmoles) de tris(dibenzylidèneacétone) dipalladium(o) dans 40 ml de 1,2-diméthoxyéthane est chauffé au reflux pendant 5 heures.et 30 minutes.Le mélange réactionnel est ensuite ramené à température pour l'ajout de 180 mg (245 µmoles) de tris(dibenzylidèneacétone) dipalladium(o), puis on porte de nouveau au reflux pendant 16 heures.

Le lendemain, on chauffe encore 9 heures après avoir ajouté 180 mg (193 µmoles) de tris(dibenzylidèneacétone) dipalladium(o).Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur silicagel par l'acétate d'éthyle 100%. On obtient 1,52 g de produit attendu sous la forme de cristaux jaune pâle (rendement = 50%)

**CCM :** Rf = 0.18 (silicagel éluant : acétate d'éthyle 100 %

**1H-RMN (CDCl3) :** δ 1,27 (d, 6H, CH3-CH-CH3); 1,83 à 2,05 (m, 9H, NH-CH2-CH2-CH2, N-CH2-CH2-CH-CH2, CH3-C=C-); 2,72 (m, 3H, CH2-CH-CH2, NH-CH2-CH2-CH2); 2,95 et 3,93 (2m, 4H, CH2-CH2-N-CH2-CH2); 3,45 (m, 2H, NH-CH2-CH2-CH2); 3,68 (d, 2H, NH-CH2-CH-NH), 4,50 (m, 1H, NH-CH2-CH-NH); 5,07 (m, 1H, CH3-CH-CH3); 5,12 (s, 2H, O-CH2-Ph); 6,42 et 7,18 (2d, 2H, CH=CH naphthyridine); 7,35 (m, 5H, Ph); 8,28 (s, 1H, N=CH-N).

**HPLC/SM :** (tr = 0,48 min et 3,03 min): 588 (MH+); 454 [MH - Z+]; 412 [MH - (ipr - Z)+]

$[\alpha]_D$ (CHCl3) = + 0,7655

Formation du Chlorhydrate :

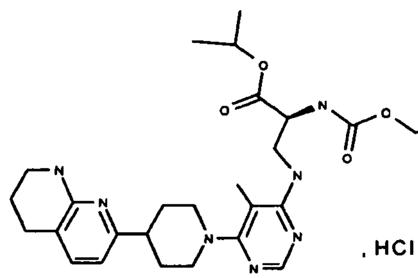

M W =624.19
M F =C32H41N7O4,HCl

3.8gde3-[[6-[4-(1,2,3,9-tetrahydro-1,8-naphthyridin-7-yl)-1-pipéridinyl]-5-méthyl-4-pyrimidinyl]amino]-N-[(phénylméthoxy)carbonyl]alaninate d'isopropyle sont solubilisés dans 20 ml de chlorure de méthylène. Cette solution est coulée sur de l'éther éthylique, la solution doit rester limpide. On ajoute ensuite goutte à goutte, sous agitation, 3,23 ml d'HCl 2N dilué dans un peu d'éther éthylique. Le chlorhydrate prend en masse, on ôte le surnageant, puis le résidu est cristallisé dans l'éther isopropylique. On obtient alors un solide qui est filtrée, rincée à l'éther puis au pentane. Après séchage, on obtient 3.44 g d'une poudre blanche. (Rendement = 85%)

**<u>Exemple de référence 9</u>**

Synthèse de la 4,6-dibromo-5-éthyl-pyrimidine

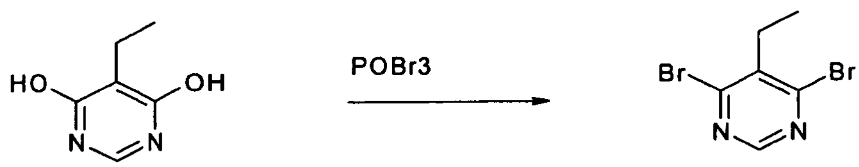


Un mélange de 2,8 g (20 mmoles) de 5-éthyl-4,6-dihydroxy-pyrimidine (commercialisée par Aldrich) et de 8,85 g (31 mmoles) d'oxybromure de phosphore est porté à 160°c durant 1 heure. Après retour à température ambiante, le mélange réactionnel est versé sur un mélange de glace, de solution saturée de bicarbonate de sodium et d'acétate

d'éthyle. On décante, lave la phase organique avec une solution saturée de bicarbonate de sodium, sèche sur sulfate de magnésium et le solvant est éliminé par évaporation sous pression réduite (2 kPa). Le résidu est chromatographié sur silicagel en éluant avec un gradient d'heptane 100 % jusqu'à heptane-acétate d'éthyle 50-50. On obtient 3,92 g (Rdt = 74 %) d'un solide marron clair de produit attendu utilisé telle quelle pour la suite.
**CCM :** Rf = 0,5 (silicagel, éluant : heptane-acétate d'éthyle 90-10)
**IR ($CHCl_3$) :** 1538; 1503 (C=C, C=N)
**1H-RMN (DMSO-d6) :** δ 1,16 (t, 3H, $CH_2$-$CH_3$); 2,86 (q, 2H, $CH_2$-$CH_3$); 8,63 ppm (s, 1H, N=CH-N)
**SM :** 266 (MH+); 249 (MH-CH3+); 184 (MH-HBr+).

Synthèse 4-bromo-5-éthyl-6-[4-(1,2,3,4-tétrahydro-1,8-naphthyridin-7-yl)-1-pipéridinyl]-pyrimidine

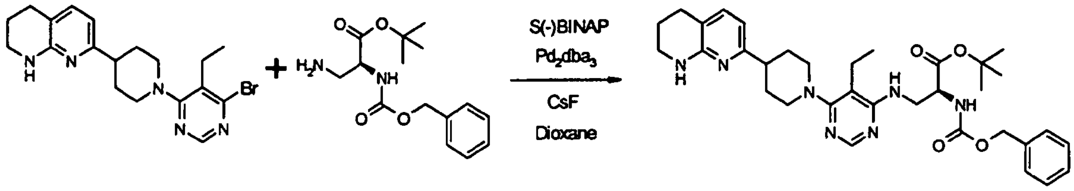


Un mélange de 3,67 g (6,45 mmoles) de 1,2,3,4-tétrahydro-7-(4-pipéridinyl)-1,8-naphthyridine, tris(trifluoroacetate) (préparé selon les brevets EP1065207 ou WO 0078317) et de 25 g de résine Amberlyst A-21 (origine Fluka 06424 préalablement lavée avec la solution dichlorométhane-acétate d'éthyle-méthanol 1-1-1) dans 150 ml de solution de dichlorométhane-acétate d'éthyle-méthanol 1-1-1 est agité à température ambiante durant 1 heure. Le mélange est filtré, la résine lavée avec la solution ternaire. Le filtrat obtenu est agité à température ambiante durant 1 heure en présence de 25 g de résine Amberlyst A-21 traitée comme précédemment; cette opération est renouvelée une troisième fois. Le filtrat ainsi obtenu est concentré à sec sous pression réduite (2 kPa) donnant 1,4 g d'amine libre. On rajoute à ce résidu 2,06 g (7,74 mmoles) de 4,6-dibromo-5-éthyl-pyrimidine, 200 ml de diméthylacétamide et 5 ml de diisopropyléthylamine et porte à 100°C durant 3,5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane 100 % jusqu'à heptane-acétate d'éthyle 50-50. On obtient 1,5 g (Rdt = 58 %) de produit attendu sous forme d'un solide beige.
CCM : Rf = 0,5 (alumine, éluant : heptane-acétate d'éthyle 50-50)
**IR (CHCl3)** 3440 (NH); 1596, 1586, 1547, 1508, 1480 cm-1 (Hétérocycle+Aromatique)
**1H-RMN (DMSO-d6) :** δ 1,23 (t, 3H, $CH_2$-$CH_3$); 1,75 (m, 6H, $CH_2$-$CH_2$-$CH_2$-NH, $CH_2$-CH-$CH_2$ ); 2,67 (m, 5H, $CH_2$-$CH_3$, $CH_2$-CH-$CH_2$, $CH_2$-$CH_2$-$CH_2$-NH); 3,02 (t, 2H, $CH_2$-$CH_2$-N); 3,23 (m, 2H, $CH_2$-$CH_2$-$CH_2$-NH); 3,90 (d, 2H, $CH_2$-$CH_2$-N) 6,20 (s, 1H, $CH_2$-$CH_2$-$CH_2$-NH) ; 6,30 et 7,04 (2d, 2H, H naphthyridine); 8,27 ppm (s,N=CH-N).
**SM :** 403 (MH+).

Synthèsedu3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

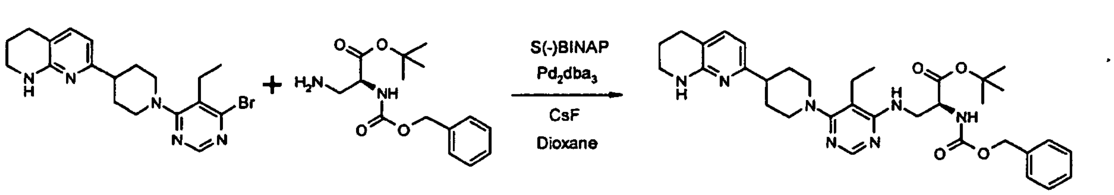


On chauffe au reflux un mélange de 317 mg (0,79 mmoles) de 4-bromo-5-éthyl-6-[4-(1,2,3,4-tétrahydro-1,8-naphthyridin-7-yl)-1-pipéridinyl]-pyrimidine, de 279 mg (0,95 mmoles) de 3-amino-N-[(phénylméthoxy)carbonyl]alaninate de (1,1-diméthyléthyle) (préparé selon J. Med. Chem.(2001), 44(8), 1158-1176), de 168 mg (1,11 mmoles) de fluorure de césium, de 36 mg (0,04 mmoles) de tris(dibenzylidèneacétone)dipalladium(0), de 49 mg (0,08 mmoles) de

S(-)2,2'-bis(diphényl-phosphino)-1,1'-binaphtyl dans 11 ml de dioxane pendant 21 heures. On refroidit puis ajoute à nouveau 140 g (0,48 moles) de 3-amino-N-[(phénylméthoxy)carbonyl]alaninate de (1,1-diméthyléthyle), 168 mg (1,11 mmoles) de fluorure de césium, 36 mg (0,04 mmoles) de tris(dibenzylidèneacétone)dipalladium(0),et 49 mg (0,08 mmoles) de S(-)2,2'-bis(diphényl-phosphino)-1,1'-binaphtyl dans 2 ml de dioxane et chauffe au reflux pendant 3,5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane-acétate d'éthyle-methanol 50-50-0, 25-75-0, 0-100-0, et enfin 0-100-3. On obtient 400 mg (Rdt = 83%) de produit attendu sous forme d'huile.

**CCM :** Rf = 0,55 (silicagel, éluant : acétate d'éthyle-méthanol-triéthylamine 95-2-2).

**IR (CHCl3)** : 3435 (NH); 1717 (C=O); 1583,1555,1501 cm-1 (Hétérocycle+Aromatique+Amide).

**1H-RMN (DMSO-d6) :** δ 1,06 (t, 3H, $CH_2$-$\underline{CH_3}$); 1,31 (s, 9H, tBu); 1,75 (m, 2H, $CH_2$-$\underline{CH_2}$-$CH_2$-NH); 1,77 (m, 4H, $\underline{CH_2}$-CH-$\underline{CH_2}$); 2,46 (m, 2H, $\underline{CH_2}$-$CH_3$); 2,53 (m, 1H, $CH_2$-$\underline{CH}$-$CH_2$); 2,62 (t, 2H, $\underline{CH_2}$-$CH_2$-$CH_2$-NH); 2,83 et 3,44 (2m, 4H, $CH_2$-$\underline{CH_2}$-N-$\underline{CH_2}$-$CH_2$); 3,24 (m, 2H, $CH_2$-$CH_2$-$\underline{CH_2}$-NH); 3,72 (m, 2H, NH-$\underline{CH_2}$-CH-NH); 4,23 (q, 1H, NH-$CH_2$-$\underline{CH}$-NH); 5,02 (s, 2H, $\underline{CH_2}$-Ph); 6,24 (s, 1H, $CH_2$-$CH_2$-$CH_2$-$\underline{NH}$); 6,30 et 7,06 (2d, 2H, H naphthyridine); 6,36 (m, 1H, $\underline{NH}$-$CH_2$-CH-NH); 7,34 (m, 5H, Ph); 7,62 (d, 1H, NH-$CH_2$-CH-$\underline{NH}$) ; 8,11 ppm (s,N=$\underline{CH}$-N).

**SM :** 616 (MH+); 560 (MH-tBu+); 426 (MH-COOCH2Ph+).

[$\alpha_D$] **(1 % CHCl3) :** +2.4°

[$\alpha_D$] **(1,05** % **EtOH) :** -6,1°

Synthèse de la 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

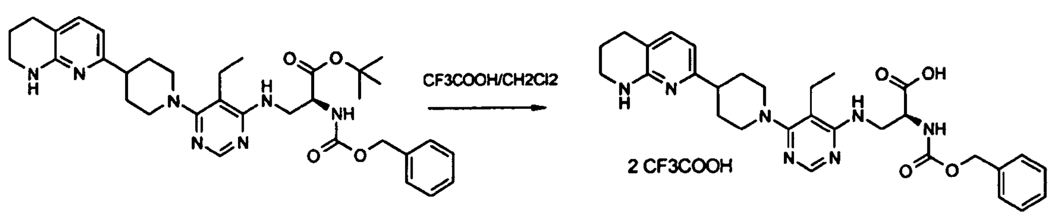


On agite 475 mg (0,77 mmoles) de 3-[[5-éthyl-6-[4-(1,2,3,9-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 15 ml de dichlorométhane avec 3,5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 368 mg (Rdt = 61 % exprimé en ditrifluoroacétate) de produit attendu sous forme d'un solide amorphe.

**CCM:** Rf = 0,33 (silicagel, éluant : dichlorométhane-méthanol-eau-acide acétique 90-10-1-1)

**IR ($CHCl_3$)** : 1677 (C=O); 1625; 1587; 1492 cm-1 (Système conjugué+Aromatique).

**1H-RMN** (DMSO-d6) : δ 1,04 (t, 3H, CH2-<u>CH3</u>); 1,77 et 1,90 (2m, 4H, N-CH2-<u>CH2</u>-CH-); 1,81 (m, 2H, NH-CH2-<u>CH2</u>-CH2-); 2,45 (q, 2H, <u>CH2</u>-CH3); 2,73 (t, 2H, NH-CH2-CH2-<u>CH2</u>-); 2,78 (t, 1H, N-CH2-CH2-<u>CH</u>-); 2,87 et 3,48 (2m, 4H, N-<u>CH2</u>-CH2-CH-); 3,23 (m, 2H, N-<u>CH2</u>-CH2-CH2-); 3,58 et 3,83 (2m, 2H, NH-<u>CH2</u>-CH-NH); 4,28 (q, 1H, NH-CH2-<u>CH</u>-NH); 5,01 (syst AB, 2H, O-<u>CH2</u>-Ph); 6,65 (d, 1H, H naphthyridine); 6,55 (sl, 1H, <u>NH</u>-CH2-CH-NH); 7,33 (m, 5H, Ph); 7,68 (d, 1H, NH-CH2-CH-<u>NH</u>); 7,58 (d, 1H, H naphthyridine); 8,15 (s, 1H, N=<u>CH</u>-N); 6,24 ppm (sl, 1H, <u>NH</u>-CH2-CH2-CH2-).

**SM :** 560 (MH+); 426 (MH-COOCH2Ph+); 558- (M-H-); 450- (558-OCH2Ph-); 1117- (2M-H-)

[$\alpha_D$] **(1** % **CHCl3)** :+2,4 °

<u>**Exemple** de référence 10</u>

N-[(phénylméthoxy)carbonyl]alaninate de (1,1 diméthyléthyle)

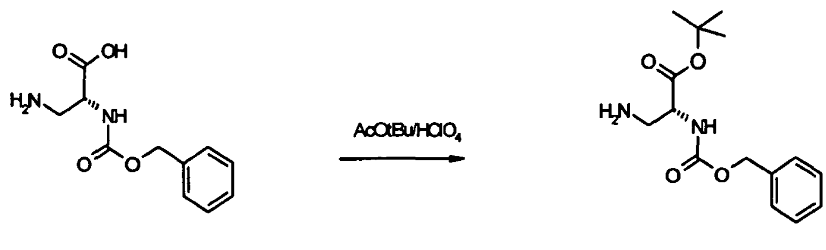


A 480 mg (2 mmoles) d'acide N-[(phénylméthoxy)carbonyl] alanique dans 15 ml d'acétate de terbutyle on ajoute goutte à goutte 1 ml d'acide perchlorique. Le mélange biphasique est agité 24 heures à température ambiante. Le mélange est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane-acétate d'éthyle-méthanol 100-0-0, 0-100-0, et enfin 0-95-05. On obtient 50 mg (Rdt = 10 %) de produit attendu sous forme d'huile.
**CCM :** Rf = 0,20 (silicagel, éluant : dichlorométhane-méthanol-eau-acide acétique 90-10-1-1)
[$\alpha_D$] **(1,25 % CHCl3) :** -9,0°

Synthèsedu3-[[5-éthyl-6-[9-(1,2,3,4-tétrahydro-1,8-naphthyridin-7-yl)-1-pipéridinyl]-4-pyrimidinyl]amino]-N-[(phénylméthoxy)carbonyl]alaninate d'(1,1 diméthyléthyle).

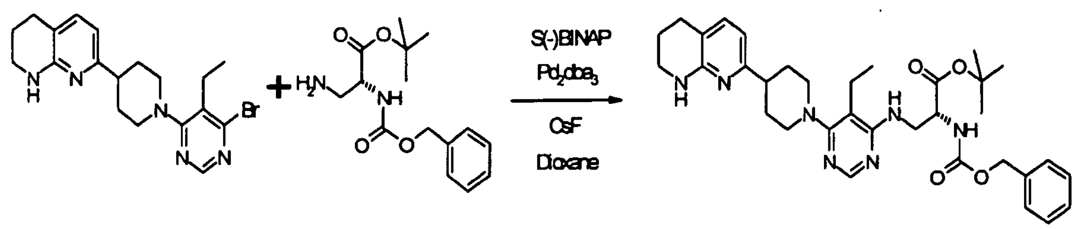


On chauffe au reflux un mélange de 200 mg (0,5 mmole) de 4-bromo-5-éthyl-6-[9-(1,2,3,4-tétrahydro-1,8-naphthyridin-7-yl)-1-pipéridinyl]-pyrimidine, de 50 mg (0,17 mmole) de (D) 3-amino-N-[(phénylméthoxy)carbonyl]alaninate d'(1,1 diméthyléthyle),de 40 mg (0,26 mmole) de fluorure de césium, de 15 mg (0,016 mmole) de tris(dibenzylidèneacétone)dipalladium(0), de 20 mg (0,032 mmole) de 2,2'-bis(diphényl-phosphino)-1,1'-binaphtyl dans 20 ml de dioxane pendant 5 heures. On refroidit puis ajoute à nouveau 15 mg (0,016 mmole) de tris(dibenzylidèneacétone)dipalladium (0) et chauffe au reflux pendant 15 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié une première fois sur alumine en éluant avec un gradient d'acétate d'éthyle-ether diisopropylique-chlorure de méthylène 50-50-50, 10-50-50, et enfin 10-20-70 puis sur silicagel en éluant avec de l'acétate d'éthyle. On obtient 40 mg (Rdt = 38 %) de produit attendu sous forme d'huile.
**CCM :** Rf = 0,25 (silicagel, éluant : acétate d'éthyle).
**SM :** 616 (MH+); 560 (MH-tBu+).
[$\alpha_D$] **(1,0 % CHCl3) :** -2,4

Synthèse de la 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

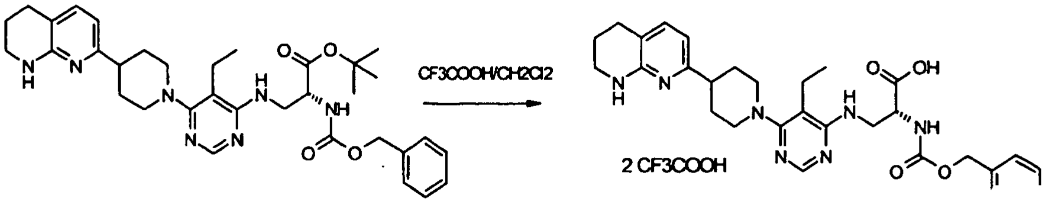


On agite 40 mg (0,77 mmoles) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyllalaninate de (1,1-diméthyléthyle) dans 10 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est chromatographié sur silicagel en éluant avec un mélange CH2Cl2-MeOH-H2O-AcOH 90-10-1-1. On obtient 25 mg (Rdt = 48 % exprimé en ditrifluoroacétate) de produit attendu sous forme d'un solide amorphe.
**CCM :** Rf = 0,10 (silicagel, éluant : dichlorométhane-méthanol-eau-acide acétique 90-10-1-1)
SM : 560 (MH+); 426 (MH-COOCH2Ph+); 558- (M-H-); 450- (558-OCH2Ph-); 1117- (2M-H-)
[$\alpha_D$] **(1,75 % CHCl3) :** -6,0

**Exemple de référence 11**

3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate d'éthyle, dichlorhydrate

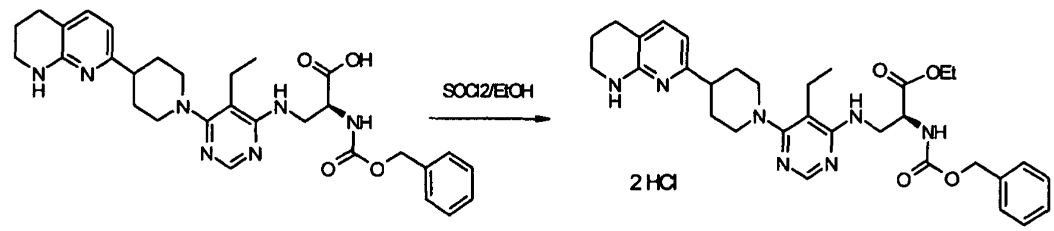


A 5 ml d'éthanol refroidi à -12°C sous atmosphère d'azote on ajoute 160 µl (2,19 mmoles) de chlorure de thionyle et agite le mélange pendant 30 minutes. On additionne alors une solution de 354 mg (0,45 mmoles) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate) dans 13 ml d'éthanol. Le mélange est agité 30 minutes à -12°C puis on laisse revenir à température ambiante et enfin chauffe à 40°C pendant 4 heures sous azote. On évapore le mélange réactionnel à sec sous pression réduite (2 kPa) et cristallise le résidu dans un mélange d'éther diisopropylique et de pentane et filtre le solide. On obtient 284 mg (Rdt = 95 %) de produit attendu sous forme d'un solide beige amorphe.
**CCM :** Rf = 0,4 (silicagel, éluant : dichlorométhane-méthanol-eau-acide acétique 90-10-1-1)
**IR ($CHCl_3$) :** 3400-3000(OH, NH); 1719(C=O): 1654; 1623; 1580; 1504 cm-1(C=C, C=N, aromatique)
**1H-RMN (DMSO-d6) :** δ 1, 09 (t, 3H, CH2-CH3) ; 1, 14 (t, 3H, O-CH2-CH3) ; 1, 85 (m, 2H, NH-CH2-CH2-CH2); 1,85 et 2,00 (2m, 4H, N-CH2-CH2-CH); 2,48 (m, 2H, CH2-CH3); 2,75 (t, 2H, NH-CH2-CH2-CH2); 2,92 (m, 1H, N-CH2-CH2-CH); 3,03 et 3,57 (2m, 4H, N-CH2-CH2-CH); 3,44 (t, 2H, N-CH2-CH2-CH2); 3,76 et 3,87 (2m, 2H, NH-CH2-CH-NH); 4,07 (q, 2H, O-CH2-CH3); 4,39 (q, 1H, NH-CH2-CH-NH); 5,03 (s, 2H, O-CH2-Ph); 6,62 (d, 1H, H naphthyridine); 6,86 (sl, 1H, NH-CH2-CH-NH); 7,30 (m, 5H, Ph); 7,33 (sl, 1H, NH-CH2-CH-NH); 7,58 (d, 1H, H naphthyridine); 8,20 ppm (s, 1H, N=CH-N).
**SM :** 588 (MH+); 454 (MH-COOCH2Ph+); 426 (454-C2H4+).
**[αD] (1 % CHCl3) :** +4

**Exemple** de référence **12**

N-[(phénylméthoxy)carbonyl]alaninate d'éthyle

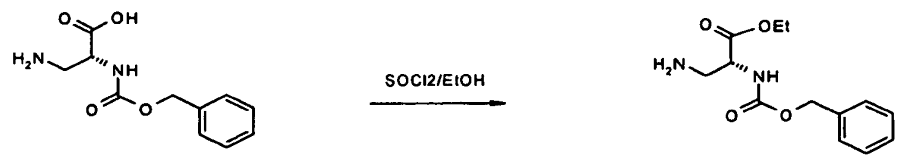


A 12 ml d'éthanol refroidi à 0°C sous atmosphère d'azote on ajoute 6 ml (63 mmoles) de chlorure de thionyle et agite le mélange pendant 20 minutes à température ambiante. On additionne alors une solution de 950 mg (4 mmoles) d'acide N-[(phénylméthoxy)carbonyl]alanique dans 5 ml d'éthanol. Le mélange est agité au reflux pendant 1 heure sous azote. On évapore le mélange réactionnel à sec sous pression réduite (2 kPa) et cristallise le résidu dans un mélange d'éther diisopropylique et de pentane et filtre le solide. Le solide obtenu est repris par de l'acétate d'éthyle, de l'eau et une solution saturée de bicarbonate de sodium. On obtient 535 mg (Rdt = 50 %) de produit attendu sous forme d'une huile incolore.
**CCM :** Rf = 0,4 (silicagel, éluant : dichlorométhane-méthanol-eau-acide acétique 90-10-1-1)
**HPLC/SM :** (tr = 0,5 et 1,4 min) 267 (MH+).
**[αD] (1,0 % CHCl3) :** -11,2

Synthèsedu3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate d'éthyle.

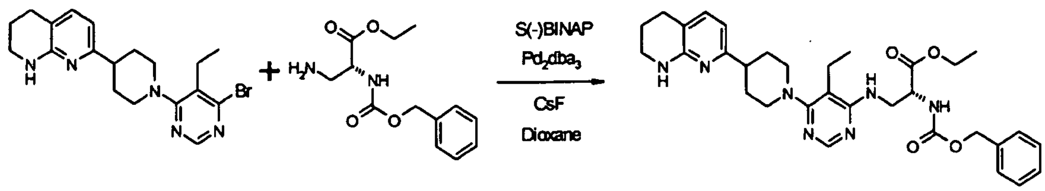


On chauffe au reflux un mélange de 320 mg (0,8 mmole) de 4-bromo-5-éthyl-6-[4-(1,2,3,4-tétrahydro-1,8-naphthyridin-7-yl)-1-pipéridinyl]-pyrimidine, de 215 mg (1,04 mmole) de (D) 3-amino-N-[(phénylméthoxy)carbonyl]alaninate d'éthyle, de 190 mg (1,25 mmoles) de fluorure de césium, de 37 mg (0,04 mmole) de tris(dibenzylidèneacétone) dipalladium(0), de 50 mg (0,08 mmole) de S(-)2,2'-bis(diphényl-phosphino)-1,1'-binaphtyl dans 15 ml de dioxane pendant 5 heures. On refroidit puis ajoute à nouveau 37 mg (0,04 mmole) de tris(dibenzylidèneacétone)dipalladium(0) et chauffe au reflux pendant 7 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient d'acétate d'éthyle-ether diisopropylique-chlorure de méthylène 50-50-50, 10-50-50, et enfin 10-20-70. On obtient 260 mg (Rdt = 55%) de produit attendu sous forme d'huile.
**CCM :** Rf = 0,25 (silicagel, éluant : acétate d'éthyle).
**1H-RMN (CDCl3) :** δ 1,16 (t, 3H, CH2-CH3); 1,27 (t, 3H, O-CH2-CH3); 1,85 à 2,00 (m, 6H, N-CH2-CH2-CH et NH-CH2-CH2-CH2); 2,46 (m, 2H, CH2-CH3); 2,75 (t, 3H, NH-CH2-CH2-CH2 et N-CH2-CH2-CH); 3,89 (t, 2H, N-CH2-CH2-CH2); 3,47 et 3,61 (2m, 4H, N-CH2-CH2-CH); 3,00 et 3,98 (2m, 2H, NH-CH2-CH-NH); 4,22 (m, 2H, O-CH2-CH3); 4,54 (m, 1H, NH-CH2-CH-NH); 5,13 (s, 2H, O-CH2-Ph); 6,43 (d, 1H, H naphthyridine); 7,22 (d, 1H, H naphthyridine); 7,35 (m, 5H, Ph); 8,27 ppm (s, 1H, N=CH-N).
**SM :** 588 (MH+); 454 (MH-COOCH2Ph+).
**[αD] (1,3 % CHCl3) :** -6,0

**Exemple** de référence **13**

Synthèse du de 6-[4-(1,2,3,4-tetrahydro-1,8-naphthyridin-7-yl)-1-pipéridinyl]-5-éthyl-4-chloro-pyrimidine :

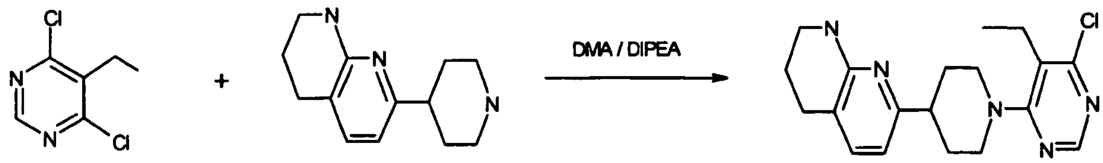


Dans un monocol contenant 12,5 g (57,5 mmoles) de 4-(1,2,3,4-tetrahydro-1,8-naphthyridin-7-yl)-1-pipéridine libérée de son sel, on ajoute 80 ml diméthylacétamide, 11,8 g (30 mmoles) de 4,6-dichloro-5-éthyl-pyrimidine mis en solution dans 40 ml de diméthylacétamide et 24 ml de diisopropyléthylamine. Ce mélange est chauffé à 140°C pendant 5 heures puis concentré à sec sous vide. Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous vide pour donner 15,5 g d'une huile orangée. Cette huile est solubilisée dans un minimum de chlorure de méthylène et précipitée dans l'éther isopropylique, on obtient 9 g du produit attendu sous la forme d'une poudre beige. Le filtrat est concentré à sec puis chromatographié sur silicagel en éluant avec un gradient de cyclohexane (100 %), d'acétate d'éthyle-cyclohexane (50-50) puis d'acétate d'éthyle-cyclohexane (70-30).

On obtient 3 g de produit attendu sous la forme d'une poudre jaune pâle. (Rdt global = 58 %)

Préparation de la naphtyridine sous forme d'amine libre :

28,5 g de naphtyridine est déplacée de son sel par 6 équivalents massiques de résine basique amberlyst A21 (résine de type R-$NMe_2$) dans un mélange $CH_2Cl_2$ / MeOH /AcOEt 1/1/1 sous agitation pendant 30 minutes. On lave préalablement la résine et on la laisse gonfler 20 minutes dans ce mélange de solvant.

Cette opération doit être répétée 3 fois pour que le déplacement du sel soit total.

Après filtration de la résine et évaporation des solvants, on obtient 12,5 g (57,5 mmoles) de naphtyridine libre.

**CCM :** Rf = 0,32 [silicagel, éluant : acétate d'éthyle (100 %)]

**1H-RMN () :** δ 1,32 (t, 3H, CH3-CH2); 1, 81 à 2,05 (m, 6H, NH-CH2-CH2-CH2, N-CH2-CH2-CH-CH2); 2,70 (m, 5H, CH2-CH-CH2, NH-CH2-CH2-CH2, CH3-CH2); 3,43 (m, 2H, NH-CH2-CH2-CH2); 3.05 et 3,97 (2m, 4H, CH2-CH2-N-CH2-CH2); 6,4 et 7,12 (2d, 2H, CH=CH naphthyridine); 8,37 (s, 1H, N=CH-N).

**HPLC / SM :** (tr = 0,51 min et 2,93 min): 358 (MH+)

Synthèsedu3-[[6-[4-(1,2,3,4-tétrahydro-1,8-naphthyridin-7-yl)-1-pipéridinyl]-5-éthyl-4-pyrimidinyl]amino]-N-[(phénylméthoxy)carbonyl]alaninate d'isopropyle.

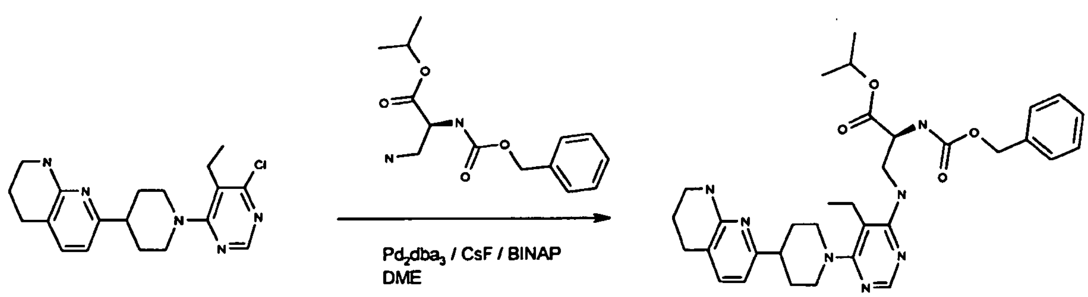


Un mélange de 1 g (2,79 mmoles) de 6-[4-(1,2,3,4-tetrahydro-1,8-naphthyridin-7-yl)-1-pipéridinyl]-5-éthyl-4-chloro-pyrimidine et de 940 mg (3,35.mmoles) de 3-amino-N-[(phénylméthoxy)carbonyl] alaninate d'isopropyle, en présence de 594 mg (3,91 mmoles) de fluorure de cesium, de 174 mg (279 µmoles) de (2,2'-bis(diphénylphosphino)-1,1'-binaphthyl et de 130 mg (140 µmoles) de tris(dibenzylidèneacétone) dipalladium(o) dans 35 ml de 1,2-diméthoxyéthane

est chauffé au reflux pendant 5 heures. Le mélange réactionnel est ensuite ramené à température pour l'ajout de 180 mg (140 μmoles) de tris(dibenzylidèneacétone)dipalladium(o), puis on porte de nouveau au reflux pendant 16 heures.

Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur silicagel par l'acétate d'éthyle 100 %. On obtient 1,07 g de produit attendu sous la forme de cristaux jaune pâle (rendement = 64 %)

**CCM :** Rf = 0.20 (silicagel éluant : acétate d'éthyle 100 %

**1H-RMN (CDCl3) :** δ 1,18 (t, 3H, CH3-CH2); 1.25 (d, 6H, CH3-CH-CH3); 1,83 à 2,05 (m, 6H, NH-CH2-CH2-CH2, N-CH2-CH2-CH-CH2); 2,45 (q, 2H, CH3-CH2); 2,72 (m, 3H, CH2-CH-CH2, NH-CH2-CH2-CH2); 2,97 et 3,93 (2m, 4H, CH2-CH2-N-CH2-CH2); 3,45 (m, 2H, NH-CH2-CH2-CH2); 3,60 (d, 2H, NH-CH2-CH-NH), 4,50 (m, 1H, NH-CH2-CH-NH); 4,97 à 5,20 (m, 3H, CH3-CH-CH3, O-CH2-Ph); 6,43 et 7,18 (2d, 2H, CH=CH naphthyridine); 7,35 (m, 5H, Ph); 8,28 (s, 1H, N=CH-N).

**HPLC/SM :** (tr = 0,50 min et 3,14 min) : 602 (MH+) ; 468 [MH - Z+] ; 426 [MH - (ipr - Z) +]

**[α]$_D$ (CHCl3)** = + 2,175

Formation du Chlorhydrate :

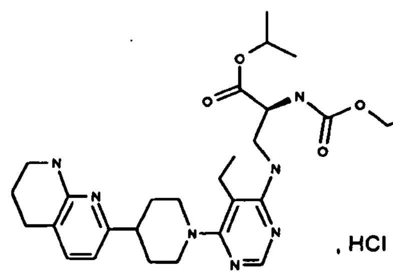

M W =638.22
M F =C33H43N7O4,HCl

3,2 g (5.3 mmoles) de 3-[[6-[4-(1,2,3,4-tetrahydro-1,8-naphthyridin-7-yl)-1-pipéridinyl]-5-éthyl-4-pyrimidinyl] amino]-N-[(phénylméthoxy)carbonyl]alaninate d'isopropyle sont solubilisés dans un minimum de chlorure de méthylène. Cette solution est coulée sur de l'éther éthylique, la solution doit rester limpide. On ajoute ensuite goutte à goutte, sous agitation, 2,65 ml d'HCl 2N dans l'éther éthylique. Le chlorhydrate prend en masse, on ôte le surnageant, puis le résidu est recristallisé dans l'éther isopropylique. On obtient alors un solide qui est filtrée, rincée à l'éther puis au pentane. Après séchage, on obtient 3,5 g d'une poudre blanc-cassé. (Rendement = quantitatif)

**Exemple** de référence **14**

Synthèse du 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-4-methylpentanoate de terbutyle.

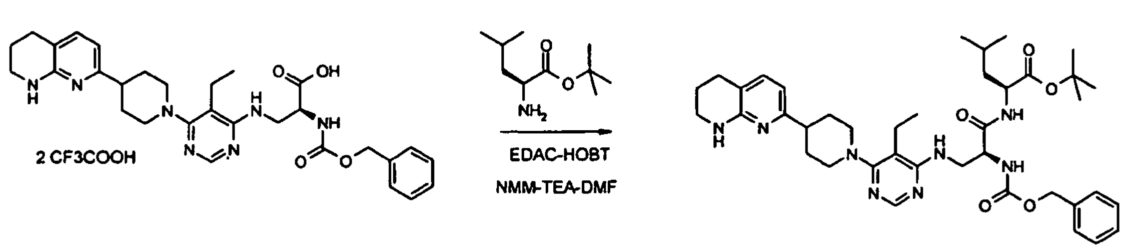

On agite 80 mg (0,15 mmole) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, 45 mg (0,20 mmole) de chlorhydrate de L-leucinate de terbutyle, 22 mg (0,16 mmole) de 1-hydoxy benzotriazole, 30 mg (0,16 mmole) de chlorhydrate de 1-[3-(diméthylamino) propyl]-3-ethyl carbodiimide, 0,050 ml (0,45 mmole) de N-methhylmorpholine et de 0,070 ml (0,50 mmole) de triéthylamine dans 5 ml de diméthylformamide pendant 24 heures à température ambiante. Le mélange réactionnel est évaporé à sec sous pression réduite (2 kPa) et le résidu est repris par de l'acétate d'éthyle et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'acétate d'éthyle-chlorure de méthylène-méthanol de 50-50-0 à 50-45-5. On obtient 60 mg (Rdt = 55 %) de produit attendu sous forme d'un solide blanc.
**CCM :** Rf = 0,66 (silicagel, éluant : dichlorométhane-méthanol-eau-acide acétique 85-15-2-2)
**1H-RMN (CDCl3) :** δ 0,91 (t, 3H, CH2-CH3) ; 1,16 (d, 6H, CHCH2CH(CH3)2); 1,28 (tl, 2H, CHCH2CH(CH3)2); 1,41 à 1,57 (m, 1H, CHCH2CH(CH3)2); 1,48 (s, 9H, tBu); 1,80 à 2,05 (m, 6H, $CH_2$-$CH_2$-$CH_2$-NH, $CH_2$-CH-$CH_2$); 2, 49 (m, 2H, $CH_2$-$CH_3$); 2,67 (tt, 1H, $CH_2$-CH-$CH_2$); 2,73 (t, 2H, $CH_2$-$CH_2$-$CH_2$-NH); 2,98 et 3,66 (ql et m, 4H, $CH_2$-$CH_2$-N-$CH_2$-$CH_2$); 3,43 (m, 2H, $CH_2$-$CH_2$-$CH_2$-NH); 3,70 et 4,16 (2m, 2H, NH-$CH_2$-CH-NH); 4,32 (m, 1H, NH-$CH_2$-CH-NH); 4,49 (m, 1H, CONH-CHCH2CH(CH3)2)-CO); 5,13 (m, 2H, $CH_2$-Ph); 5,42, 6,83 et 7,92 (3H, Hmobiles); 6,43 et 7,15 (2d, 2H, H naphthyridine); 7,37 (m, 5H, Ph); 8,2 ppm (s, 1H, N=CH-N).
**SM :** 729 (MH+).

Synthèse de l'acide 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-4-méthylpentanoique.

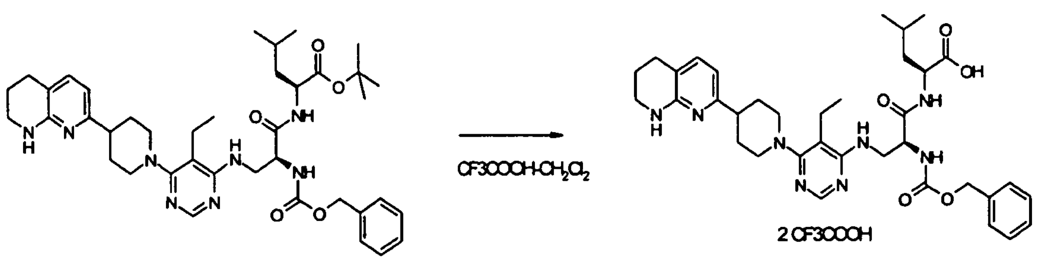


On agite 60 mg (0,083 mmoles) de 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-4-methylpentanoate de terbutyle dans 5 ml de dichlorométhane avec 0,5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 55 mg (Rdt = 74 % exprimé en ditrifluoroacétate) de produit attendu sous forme d'un solide blanc.
**CCH :** Rf = 0,52 (silicagel, éluant : dichlorométhane-méthanol-eau-acide acétique 85-15-2-2)
**SM :** 673 (MH+).

## Exemple de référence 15

Synthèse de la 4,6-dihydroxy-5-propyl-pyrimidine.

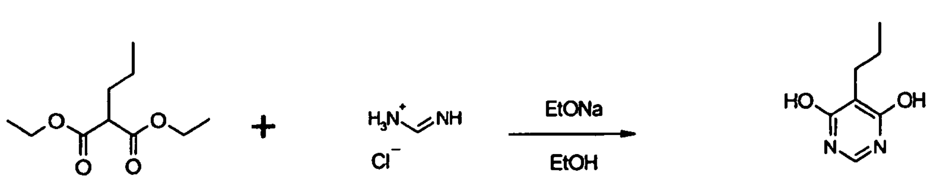

A une solution de 7,5 g (94 mmoles) de chlorhydrate de formamidine dans 200 ml d'éthanol refroidie à 0°C on ajoute 102 ml (282 mmoles) d'une solution d'éthylate de sodium à 21 % dans l'éthanol et agite le mélange pendant 30 minutes; on additionne alors une solution de 19,5 ml (94 mmoles) de propyl malonate de diéthyle dans 50 ml d'éthanol et agite une nuit à température ambiante. On évapore le mélange réactionnel à sec sous pression réduite (2 kPa). Le résidu est repris par 100 ml d'une solution saturée en chlorure de sodium et extrait par 800 et 200 ml de n-butanol. les phases organiques sont séchées sur sulfate de sodium, filtrées et évaporées à sec sous pression réduite (2 kPa). On obtient 7,3 g (Rdt = 50 %) de produit attendu sous forme d'une solide beige.
**1H-RMN (DMSO d6) :** δ 0,85(t, 3H, $CH_2$-$CH_2$-$\underline{CH_3}$) ; 1, 39 (sext, 2H, $CH_2$-$\underline{CH_2}$-$CH_3$); 2,23(t, 2H, $\underline{CH_2}$-$CH_2$-$CH_3$); 7,85 ppm (s, N=CH-N).
**SM :** 155 (MH+); 159 (M-H-).

Synthèse de la 4,6-dichloro-5-propyl-pyrimidine

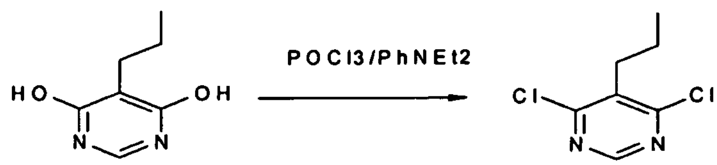


Un mélange de 3 g (19,5 mmoles) de 5-propyl-4,6-dihydroxy-pyrimidine dans 20 ml d'oxychlorure de phosphore est porté au reflux durant 1 heure. Après retour à température ambiante, on ajoute goutte à goutte un mélange de 3 ml de N,N-diéthylaniline dans 10 ml d'oxychlorure de phosphore et porte le tout au reflux pendant 4 heures. Après retour à température ambiante, le mélange réactionnel est versé sur un mélange de glace et d'eau puis on ajoute doucement du bicarbonate de sodium jusqu'à pH basique. On extrait à l'acétate d'éthyle, sèche sur sulfate de magnésium et évapore à sec sous pression réduite (2 kPa). Le résidu est chromatographié sur alumine en éluant avec un gradient d'heptane-chlorure de méthylène 100-0 à 80-20. On obtient 2 g (Rdt = 54 %) de produit attendu sous forme d'un solide bleuté.
**CCM :** Rf = 0,35 (silicagel, éluant : heptane-acétate d'éthyle 90-10).

Synthèse de la 6-[4-(1,2,3,4-tetrahydro-1,8-naphthyridin-7-yl)-1-pipéridinyl]-5-propyl-4-chloro-pyrimidine :

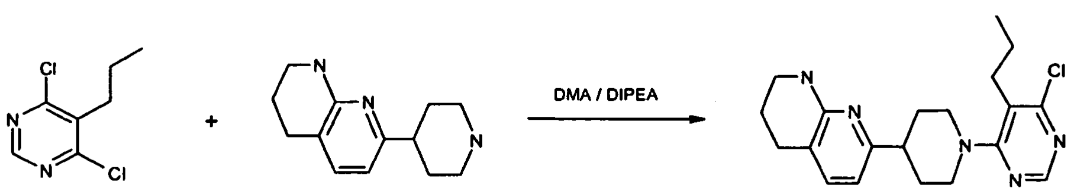


Dans un monocol contenant 1,6 g (7,4 mmoles) de 4-(1,2,3,4-tetrahydro-1,8-naphthyridin-7-yl)-1-pipéridine libérée de son sel, on ajoute 50 ml diméthylacétamide, 2 g (10,5 mmoles) de 4,6-dichloro-5-propyl-pyrimidine et 4 ml de diisopropyléthylamine. Ce mélange est chauffé à 140°C pendant 5 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase aqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'heptane-chlorure de méthylène- acétate d'éthyle de 50-50-0 à 0-0-100. On obtient 875mg de produit attendu sous la forme d'un solide amorphe. (Rdt = 50 %)
**CCM :** Rf = 0,25 [silicagel, éluant : acétate d'éthyle (100 %)]
**1H-RMN (CDCl3) :** δ 1,02(t, 3H, C-$CH_2$-$CH_2$-$\underline{CH_3}$); 1, 72 (sext, 2H, C-$CH_2$-$\underline{CH_2}$-$CH_3$); 1,81 à 2,07 (m, 6H, NH-CH2-<u>CH2</u>-CH2, N-CH2-<u>CH2</u>-CH-<u>CH2</u>); 2,64(t, 2H, C-$\underline{CH_2}$-$CH_2$-$CH_3$ ); 2,73 (m, 3H, CH2-<u>CH</u>-CH2, NH-CH2-CH2-<u>CH2</u>); 3,46 (t, 2H, NH-<u>CH2</u>-CH2-CH2); 3,06 et 3,96 (m et d, 4H, CH2-<u>CH2</u>-N-<u>CH2</u>-CH2); 5,00 (m, 2H, <u>NH</u>-CH2-CH2-CH2); 6,40 et 7,14 (2d, 2H, <u>CH</u>=<u>CH</u> naphthyridine); 8,38 (s, 1H, N=<u>CH</u>-N).
**SM :** 372,374 (MH+).

Synthèse du 3-[[5-propyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

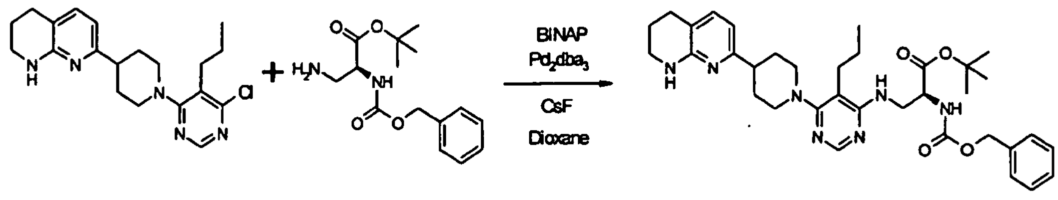


On chauffe au reflux un mélange de 600 mg (1,62 mmoles) de 4-chloro-5-propyl-6-[4-(1,2,3,4-tétrahydro-1,8-naphthyridin-7-yl)-1-pipéridinyl]-pyrimidine, de 590mg (2 mmoles) de 3-amino-N-[(phénylméthoxy)carbonyl]alaninate de (1,1-diméthyléthyle) (préparé selon J. Med. Chem. (2001), 44(8), 1158-1176), de 380 mg (2,51 mmoles) de fluorure de césium, de 76 mg (0,083 mmole) de tris(dibenzylidèneacétone)dipalladium(0), de 102 mg (0,163 mmole) de 2,2'-bis (diphényl-phosphino)-1,1'-binaphtyl dans 50 ml de dioxane pendant 20 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient de chlorure de méthylène-ether diisopropylique- acétate d'éthyle-methanol de 50-50-0-0 à 0-50-50-0 puis 0-0-100-0 et enfin 0-0-90-10. On obtient 620 mg (Rdt = 61 %) de produit attendu sous forme de solide amorphe jaune.
**CCM :** Rf = 0,12 (silicagel, éluant : acétate d'éthyle).
**1H-RMN (CDCl3) :** δ 0,90(t, 2H, C-$CH_2$-$CH_2$-$CH_3$); 1,01(t, 3H, C-$CH_2$-$CH_2$-$CH_3$); 1,48 (s, 9H, tBu); 1,81 à 2,07 (m, 6H, NH-CH2-CH2-CH2, N-CH2-CH2-CH-CH2) ; 2, 39 (t, 2H, C-$CH_2$-$CH_2$-$CH_3$); 2,65 (tl, 1H, CH2-CH-CH2); 2,73 (t, 2H, NH-CH2-CH2-CH2); 2,97 et 3,55 (tl et dl, 4H, CH2-CH2-N-CH2-CH2); 3,44 (m, 2H, NH-CH2-CH2-CH2); 3,85 et 3,93 (2m, 2H, NH-$CH_2$-CH-NH); 4,46 (m, 1H, NH-$CH_2$-CH-NH); 5,14 (s, 2H, $CH_2$-Ph); 6,16 (d, 1H $CH_2$-$CH_2$-$CH_2$-NH); 6,44 (d, 1H, H naphthyridine); 7,15 (d, 1H, H naphthyridine), 7,37 (m, 5H, CH2Ph); 8,29 (s, 1H, N=CH-N);
**SM :** 630 (MH+); 574 (MH-tBu+); 440 (MH-COOCH2Ph+).

Synthèsedela3-[[5-propyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

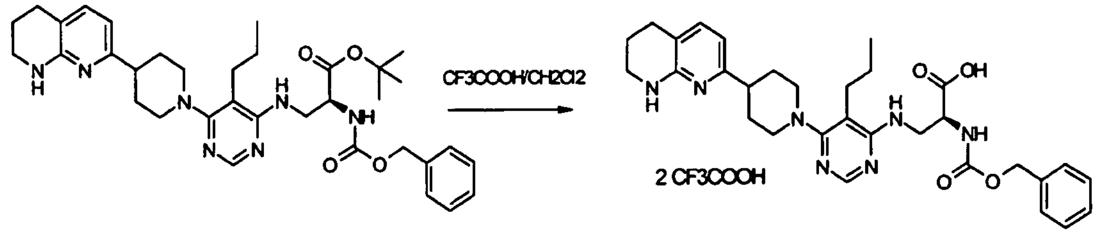


On agite 610 mg (0,97 mmole) de 3-[[5-propyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 50 ml de dichlorométhane avec 5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 400 mg (Rdt = 51 % exprimé en ditrifluoroacétate) de produit attendu sous forme d'un solide beige.
**CCM :** Rf = 0,40 (silicagel, éluant : dichlorométhane-méthanol-eau-acide acétique 85-15-2-2)
**1H-RMN (DMSO d6) :** δ 0, 93 (t, 3H, C-$CH_2$-$CH_2$-$CH_3$) ; 1, 48 (m, 2H, C-$CH_2$-$CH_2$-$CH_3$) ; 1,67 à 2,00(m, 6H, $CH_2$-$CH_2$-$CH_2$-NH et $CH_2$-CH-$CH_2$) ; 2, 40 (t, 2H, C-$CH_2$-$CH_2$-$CH_3$); 2,65 à 2,96 (m, 5H, NH-CH2-CH2-CH2-, N-CH2-CH2-CH-, N-CH2-CH2-CH-); 3,32 à 3,51 (m, 4H, N-CH2-CH2-CH- et NH-CH2-CH2-CH2-); 3,56 et 3,84 (2m, 2H, NH-CH2-CH-NH); 4,31 (q, 1H, NH-CH2-CH-NH); 5,02 (s, 2H, O-CH2-Ph); 6,68 (d, 1H, H naphthyridine); 7,33 (m, 5H, Ph); 7,61 (m, 1H, H naphthyridine); 8,20 (s, 1H, N=CH-N).
**SM :** 574 (MH+); 440 (MH-COOCH2Ph+); 572- (M-H-); 464- (572-OCH2Ph-); 1145- (2M-H-)
**[$\alpha_D$] (1,20 % CHCl3) :** -17, 7.

__Exemple de référence 16__

Synthèse de la 4,6-dihydroxy-5-isobutyl-pyrimidine.

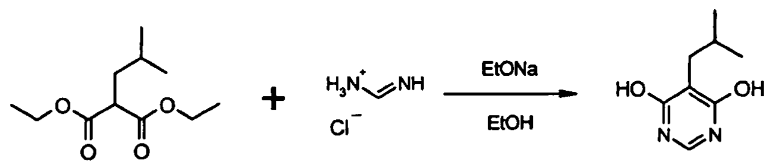


A une solution de 7,5 g (94 mmoles) de chlorhydrate de formamidine dans 200 ml d'éthanol refroidie à 0°C on ajoute 102 ml (282 mmoles) d'une solution d'éthylate de sodium à 21 % dans l'éthanol et agite le mélange pendant 30 minutes; on additionne alors une solution de 20,6 ml (94 mmoles) de propyl malonate de diéthyle dans 50 ml d'éthanol et agite une nuit à température ambiante. On évapore le mélange réactionnel à sec sous pression réduite (2 kPa). Le résidu est repris par 100 ml d'une solution saturée en chlorure de sodium et extrait par 800 et 200 ml de n-butanol. les phases organiques sont séchées sur sulfate de sodium, filtrées et évaporées à sec sous pression réduite (2 kPa). On obtient 13,7 g (Rdt = 86 %) de produit attendu sous forme d'une solide beige.
**CCM :** Rf = 0,53 (silicagel, éluant : dichlorométhane-méthanol-eau-acide acétique 85-15-2-2)
**1H-RMN (DMSO d6) :** δ 0,81(d, 6H,$CH_2$-CH-($CH_3$)$_2$); 1,85 (m, 1H, -$CH_2$-CH-($CH_3$)$_2$); 2,13(d, 2H, -$CH_2$-CH-($CH_3$)$_2$); 7,81ppm (s,N=CH-N).
**SM :** 169 (MH+); 167 (M-H-).

Synthèse de la 4,6-dichloro-5-isobutyl-pyrimidine

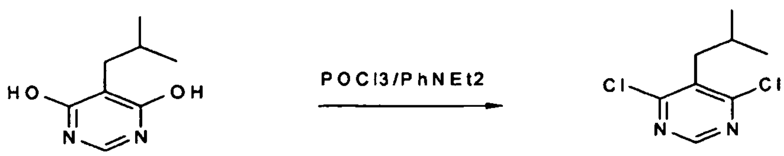


Un mélange de 3 g (17,9 mmoles) de 5-isobutyl-4,6-dihydroxy-pyrimidine dans 20 ml d'oxychlorure de phosphore est porté au reflux durant 1 heure. Après retour à température ambiante, on ajoute goutte à goutte un mélange de 3 ml de N,N-diéthylaniline dans 10 ml d'oxychlorure de phosphore et porte le tout au reflux pendant 4 heures. Après retour à température ambiante, le mélange réactionnel est versé sur un mélange de glace et d'eau puis on ajoute doucement du bicarbonate de sodium jusqu'à pH basique. On extrait à l'acétate d'éthyle, sèche sur sulfate de magnésium et évapore à sec sous pression réduite (2 kPa). Le résidu est chromatographié sur alumine en éluant avec un gradient d'heptane-chlorure de méthylène 100-0 à 80-20. On obtient 0,9 g (Rdt = 25 %) d'une huile marron.
**CCM :** Rf = 0,25 (silicagel, éluant : heptane-acétate d'éthyle 95-5).

Synthèse du de 6-[4-(1,2,3,4-tetrahydro-1,8-naphthyridin-7-yl)-1-pipéridinyl]-5-isobutyl-4-chloro-pyrimidine:

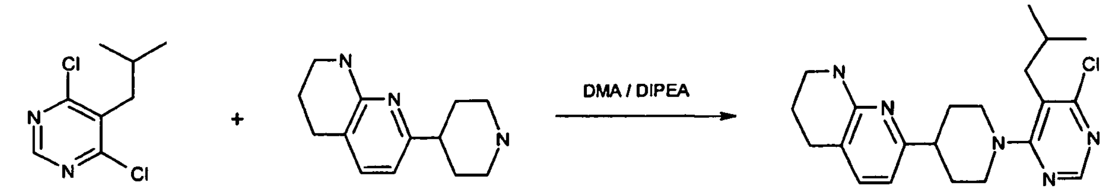


Dans un monocol contenant 0,95 g (4,4 mmoles) de 4-(1,2,3,4-tetrahydro-1,8-naphthyridin-7-yl)-1-pipéridine libérée de son sel, on ajoute 25 ml diméthylacétamide, 0,9 g (4,4 mmoles) de 4,6-dichloro-5-isobutyl-pyrimidine et 2 ml de diisopropyléthylamine. Ce mélange est chauffé à 120°C pendant 5 heures puis concentré à sec sous pression réduite (2 kPa). Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'heptane-chlorure de méthylène- acétate d'éthyle de 50-50-0 à 0-0-100. On obtient 1 g de produit attendu sous la forme d'un solide beige. (Rdt = 60 %)
**CCM :** Rf = 0,25 [silicagel, éluant : acétate d'éthyle (100 %)]
**1H-RMN** (CDCl3) : δ 0,89 (d, 6H, $CH_2$-CH-$\underline{(CH_3)_2}$); 1, 77 à 2, 12 (m, 7H, NH-CH2-CH2-CH2, N-CH2-CH2-CH-CH2 et $CH_2$-CH-$(CH_3)_2$); 2,66 (d, 2H, -$CH_2$-CH-$(CH_3)_2$); 2,73 (t, 2H, NH-CH2-CH2-CH2); 2,61 à 2,77 (1H, masqué, CH2-CH-CH2); 3,44 (m, 2H, NH-CH2-CH2-CH2); 3,03 et 3,87 (t et d, 4H, CH2-CH2-N-CH2-CH2); 5,11 (m, 2H, NH-CH2-CH2-CH2); 6,41 et 7,15 (2d, 2H, CH=CH naphthyridine); 8,40 (s, 1H, N=CH-N).
**SM :** 386,388 (MH+).

Synthèsedu3-[[5-isobutyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

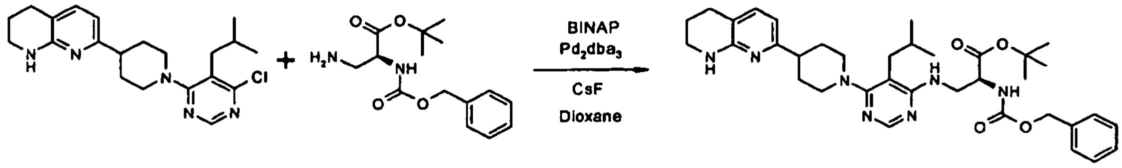


On chauffe au reflux un mélange de 450 mg (1,17 mmoles) de 4-chloro-5-isobutyl-6-[4-(1,2,3,4-tétrahydro-1,8-naphthyridin-7-yl)-1-pipéridinyl]-pyrimidine, de 413mg (1,4 mmoles) de 3-amino-N-[(phénylméthoxy)carbonyl]alaninate de (1,1-diméthyléthyle) (préparé selon J. Med. Chem.(2001), 44(8), 1158-1176), de 275 mg (1,81 mmoles) de fluorure de césium, de 55 mg (0,060 mmole) de tris(dibenzylidèneacétone)dipalladium(0), de 75 mg (0,12 mmole) de 2,2'-bis(diphényl-phosphino)-1,1'-binaphtyl dans 35 ml de dioxane pendant 20 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient de chlorure de méthylène-ether diisopropylique- acétate d'éthyle-methanol de 50-50-0-0 à 0-50-50-0 puis 0-0-100-0 et enfin 0-0-90-10. On obtient 630 mg (Rdt = 83 %) de produit attendu sous forme de solide amorphe jaune.
**CCM :** Rf = 0,12 (silicagel, éluant : acétate d'éthyle).
**1H-RMN (CDCl3) :** δ 0,91(d, 6H, $CH_2$-CH- $(CH_3)_2$); 1,49 (s, 9H, tBu); 1,77 à 2,07 (m, 7H, -$CH_2$-CH-$(CH_3)_2$), NH-CH2-CH2-CH2 et N-CH2-CH2-CH-CH2); 2,32(d, 2H, -$CH_2$-CH-$(CH_3)_2$); 2,65 (tl, 1H, CH2-CH-CH2); 2,73 (t, 2H, NH-CH2-CH2-CH2); 2,95 (tl, 2H CH2-CH2-N-CH2-CH2); 3,44 (m 4H, CH2-CH2-N-CH2-CH2 et NH-CH2-CH2-CH2); 3,85 et 3,92 (2m, 2H, NH-$CH_2$-CH-NH); 4,45 (m, 1H, NH-$CH_2$-CH-NH); 5,14 (s, 2H, $CH_2$-Ph); 6,12 (d, 1H $CH_2$-$CH_2$-$CH_2$-NH); 6,43 (d, 1H, H naphthyridine); 7,16 (d, 1H, H naphthyridine), 7,37 (m, 5H, CH2Ph); 8,31 (s, 1H, N=CH-N);
**SM :** 644 (MH+); 588 (MH-tBu+); 454 (MH-COOCH2Ph+).

Synthèse de la 3-[[5-isobutyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

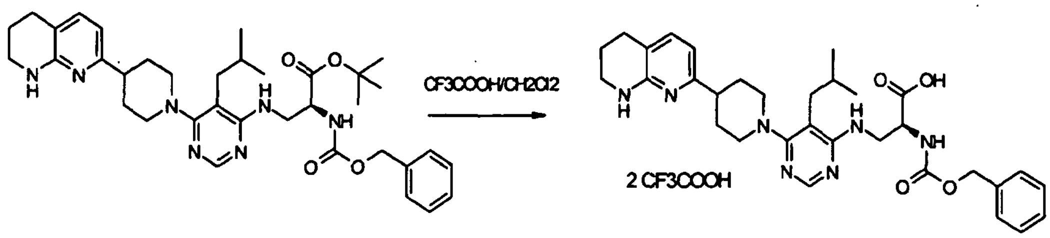


On agite 620 mg (0,96 mmole) de 3-[[5-isobutyl-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 50 ml de dichlorométhane avec 5 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 525 mg (Rdt = 67 % exprimé en ditrifluoroacétate) de produit attendu sous forme d'un solide beige.

**CCM :** Rf = 0,40 (silicagel, éluant : dichlorométhane-méthanol-eau-acide acétique 85-15-2-2)

**1H-RMN (DMSO d6) :** 0,83(d, 6H, $CH_2$-CH-$\underline{(CH_3)_2}$); 1,60 à 2, 05 (m, 7H, -$CH_2$-$\underline{CH}$-$(CH_3)_2$), NH-CH2-$\underline{CH2}$-CH2 et N-CH2-$\underline{CH2}$-CH-$\underline{CH2}$); 2,35(m, 2H, -$\underline{CH_2}$-CH-$(CH_3)_2$); 2,65 à 2,87 (m, 3H, CH2-$\underline{CH}$-CH2 et NH-CH2-CH2-$\underline{CH2}$); 2,91 (tl, 2H CH2-$\underline{CH2}$-N-CH2-CH2); 3,42 (m 4H, CH2-$\underline{CH2}$-N-CH2-CH2 et NH-CH2-CH2-CH2); 3,57 et 3,84 (2m, 2H, NH-$\underline{CH_2}$-CH-NH); 4,32 (m, 1H, NH-$CH_2$-$\underline{CH}$-NH); 5,0 (s, 2H, $\underline{CH_2}$-Ph); 6,68 (d, 1H, H naphthyridine); 7,37 (m, 5H, CH2Ph); 7,66 (d, 1H, H naphthyridine); 7,59 (d, 1H $CH_2$-$CH_2$-$CH_2$-$\underline{NH}$); 8,27 (s, 1H, N=$\underline{CH}$-N).

**SM :** 588 (MH+); 454 (MH-COOCH2Ph+); 586- (M-H-); 478- (586-OCH2Ph-); 1173- (2M-H-)

**[$\alpha_D$] (0,75 % CHCl3) :** +1,0.

**Exemple** de référence **17**

Synthèse de la 5-méthoxy-4,6-dihydroxy-pyrimidine :

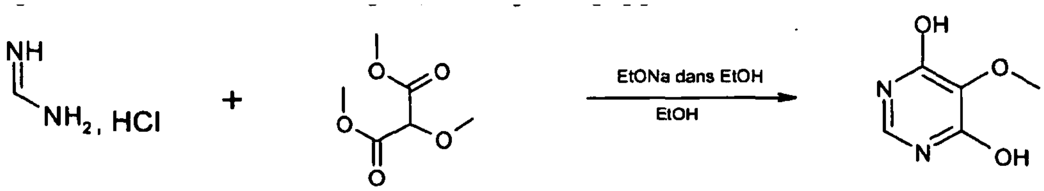


Dans un monocol contenant 30 ml d'éthanol absolu et 90 ml d'une solution d'éthylate de sodium de concentration à 1,7 mol/l dans l'éthanol, on ajoute à 0°C et par petites quantités 5 g (6,2 mmoles)de chlorhydrate de formamidine. On laisse agiter à température ambiante pendant une vingtaine de minutes, puis on ajoute goutte à goutte 8,5 ml de méthoxymalonate de diméthyle. On maintient l'agitation pendant 16 heures. Le mélange réactionnel est ensuite acidifié avec de l'acide acétique pur jusqu'à un pH entre 4 et 5, puis concentré à sec en présence de cyclohexane. Le brut obtenu est chromatographié sur silicagel après empâtage en éluant avec un mélange de chlorure de méthylène-méthanol (85-15)et 2 % d'eau-acide acétique(1-1). On obtient 9.5 g du produit attendu contena t une faible quantité de sels d'acétate de sodium.

**CCM :** Rf = 0 [Silicagel, éluant : chlorure de méthylène-méthanol (85-15)et 2 % d'eau-acide acétique(1-1).

**1H-RMN (MeOD) :** $\delta$ 3,67 (s, CH3-O); 7, 73 (s, 1H, N=$\underline{CH}$-N)

2,00 (s, 3H, CH3-COO-)

**HPLC / SM :** (tr = 0,5 min)

Synthèse de la 4,6-dichloro-5-méthoxy-pyrimidine :

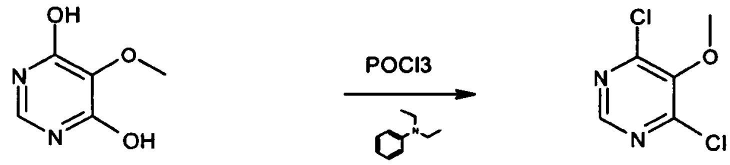


Un mélange de 5 g (35.2 mmoles) de 5-méthoxy-4,6-dihydroxy-pyrimidine et 40 ml d'oxychlorure de phosphore, est porté au reflux pendant une heure. Après retour à température ambiante, on ajoute goutte à goutte un mélange de 4.8 ml de N,N-diéthylaniline et 18 ml d'oxychlorure de phosphore.
L'ensemble est de nouveau porté au reflux pendant 4 heures 30 minutes. Après refroidissement, le mélange réactionnel est versé lentement sur un mélange de glace et d'eau puis neutralisé par une solution saturée de bicarbonate de sodium. Cette phase aqueuse est extraite à l'acétate d'éthyle. Les phases organiques regroupées sont séchées sur du sulfate de magnésium et évaporées à sec sous vide.
Le résidu obtenu est purifié sur silice en éluant avec un mélange de cyclohexane et d'acétate (95-5).
On obtient 2.5 g (Rdt = 40 %) de produit attendu sous forme d'une poudre blanche.
**CCM :** Rf = 0,48 [Silicagel, éluant : cyclohexane-acétate d'éthyle(80-20)]
**1H-RMN (MeOD) :** δ 4,00 (s, CH3-O) 8,55 (s, 1H, N=CH-N)
**HPLC/SM :** tr = 1,3 min

Synthèse de la 6-[4-(1,2,3,4-tetrahydro-1,8-naphthyridin-7-yl)-1-pipéridinyl]-5-méthoxy-4-chloro-pyrimidine :

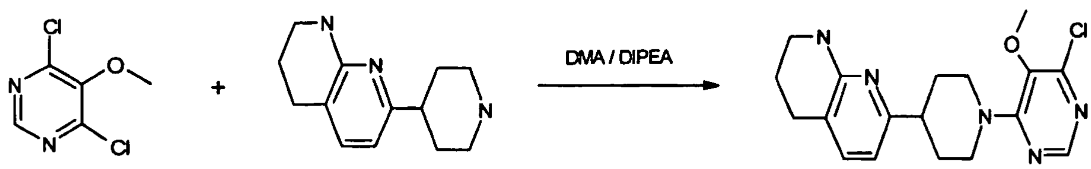


Dans un monocol contenant 800 mg (3,68 mmoles) de 4-(1,2,3,4-tetrahydro-1,8-naphthyridin-7-yl)-1-pipéridine libérée de son sel, on ajoute 2.2 g (12,3 mmoles) de 4,6-dichloro-5-méthoxy-pyrimidine mis en solution dans 25 ml de diméthylacétamide et 3640 µl de diisopropyléthylamine. Ce mélange est chauffé à 130°C pendant 2 heures puis concentré à sec sous vide. Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous vide. Le résidu est chromatographié sur alumine en éluant avec un mélange de cyclohexane et d'acétate (80-20). On obtient 900 mg (Rdt = 68 %) de produit attendu sous forme d'une poudre jaune.

Préparation de la naphtyridine sous forme d'amine libre :

2.4 g de naphtyridine est déplacée de son sel par 6 équivalents massiques de résine basique amberlyst A21 (résine de type R-$NMe_2$ ) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répétée 3 fois pour que le déplacement du sel soit total. Après filtration de la résine et évaporation des solvants, on obtient 800 mg (3.68 mmoles) de naphtyridine libre. (Rdt = 88 %).
**CCM :** Rf = 0,4 [alumine, éluant : acétate d'éthyle cyclohexane (30-70)]
**1H-RMN (MeOD) :** δ 1,75 à 1,95 (m, 6H, NH-CH2-CH2-CH2, N-CH2-CH2-CH-CH2); 2,70 (t, 1H, CH2-CH-CH2); 2,8 (m, 2H, NH-CH2-CH2-CH2); 3,15 et 3,75 (2m, 4H, CH2-CH2-N-CH2-CH2); 3,75 (s, CH3-O); 6,4 et 7,15 (2d, 2H, CH=CH naphthyridine); 8,1 (s, 1H, N=CH-N).
**HPLC / SM :** (tr = 0,53 min et 2,56 min): 359(M); 360(MH+); 361 (M+2H++).

Synthèsedu3-[[6-[4-(1,2,3,4-tetrahydro-1,8-naphthyridin-7-yl)-1-pipéridinyl]-5-méthoxy-4-pyrimidinyl]amino]-N-[(phénylméthoxy)carbonyl]alaninate de (1,1-diméthyléthyle).

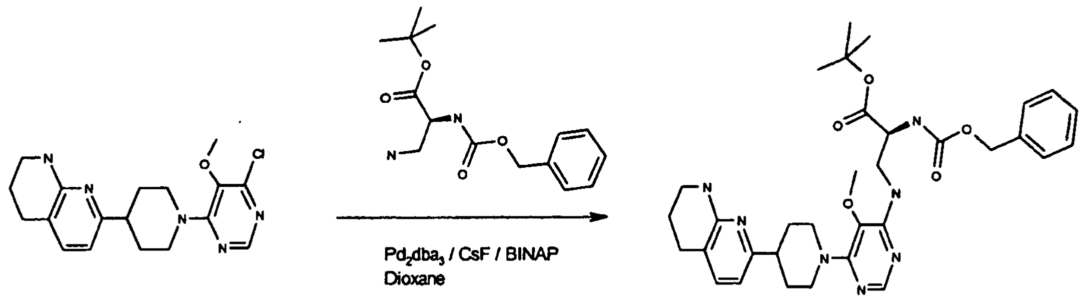


Un mélange de 300 mg (0,83 mmoles) de 6-[4-(1,2,3,4-tetrahydro-1,8-naphthyridin-7-yl)-1-pipéridinyl]-5-méthoxy-4-chloro-pyrimidine et de 295 mg (1 mmole) de 3-amino-N-[(phénylméthoxy)carbonyl] alaninate de (1,1-diméthyléthyle) (préparé selon J. Med. Chem. (2001), 44(8), 1158-1176), ( en présence de 177 mg (1,17 mmoles) de fluorure de cesium, de 52 mg (83 μmoles) de (2,2'-bis(diphénylphosphino)-1,1'-binaphthyl et de 40 mg (42 μmoles) de tris-dibenzylidèneacétone)dipalladium(o), dans 10 ml de dioxane est chauffé au reflux pendant 3 heures 30 minutes. Le mélange réactionnel est ensuite ramené à température. On ajoute alors 0,5 mmoles de 3-amino-N-[(phénylméthoxy) carbonyl] alaninate de (1,1-diméthyléthyle) et 1.17 mmoles de fluorure de cesium, 83 μmoles de (2,2'-bis(diphénylphosphino)-1,1'-binaphthyl et 42 μmoles de tris-dibenzylidèneacétone)dipalladium(o), puis le mélange réactionnel est porté au reflux pendant encore 8 heures.

Après refroidissement la solution est concentrée à sec puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous vide. Le résidu est chromatographié sur alumine avec un gradient d'éther isopropylique et d'acétate d'éthyle (70-30, 60-40 , 50-50) en terminant par l'acétate d'éthyle 100 %. Les fractions contenant le produit attendu sont rassemblées pour une seconde purification sur silicagel avec un gradient d'acétate d'éthyle 100 %. On obtient le produit attendu sous la forme d'une huile jaune pâle qui est repris dans un mélange éther isopropylique et pentane pour donner 220 mg (Rendement = 43 %) d'une poudre blanche.

**CCM :** Rf = 0.3 (alumine éluant : éther isopropylique-acétate d'éthyle 60-40)

**1H-RMN (MeOD) :** δ 1,46 (s, 9H, tBu); 1,80 (m, 2H, NH-CH2-<u>CH2</u>-CH2); 1,90 (m, 4H, N-CH2-<u>CH2</u>-CH-<u>CH2</u>); 2,65 (m, 1H, CH2-<u>CH</u>-CH2); 2,69 (t, 2H, NH-CH2-CH2-<u>CH2</u>); 2,95 et 3,78 (2m, 4H, CH2-<u>CH2</u>-N-<u>CH2</u>-CH2); 3,38 (t, 2H, NH-<u>CH2</u>-CH2-CH2); 3,59 (s, CH3-O) 4,35 (m, 1H, NH-CH2-<u>CH</u>-NH); 4.45 (d, 2H, NH-<u>CH2</u>-CH-NH); 5,03 et 5,12 (syst AB, 2H, O-<u>CH2</u>-Ph); 6,38 et 7,15 (2d, 2H, <u>CH</u>=<u>CH</u> naphthyridine); 7,32 (m, 5H, Ph); 7,90 (s, 1H, N=<u>CH</u>-N).

**HPLC/SM :** (tr = 3,4 min) : 618 (MH+) ;562 (MH -tBu); 428 [MH -(tBu-Z+)]

Synthèse de l'acide correspondant :

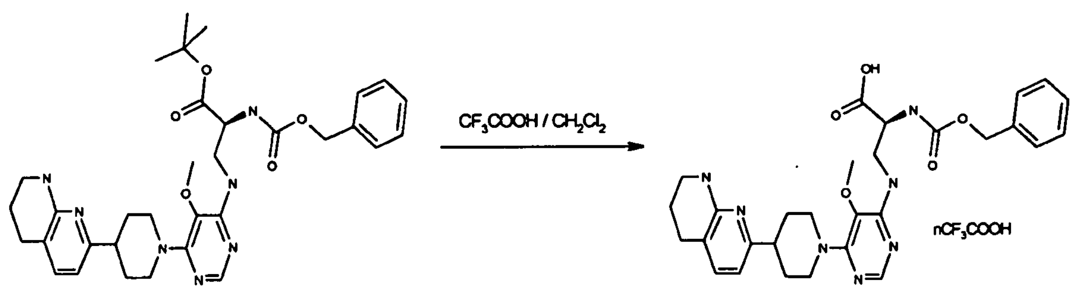


On agite à température ambiante un mélange de 200 mg (0,32 mmoles) de 3-[[5-methoxy-6-[4-(1,2,3,4-tetrahydro-1,8-naphthyridin-7-yl)-1-pipéridinyl]-5-méthyl-4-pyrimidinyl]amino]-N-[(phényl méthoxy)carbonyl]alaninate de (1,1-diméthyléthyle) et de 1.5 ml d'acide trifluoroacétique dans 15 ml de dichlorométhane pendant 8 heures. On additionne alors du toluène et évapore à sec le mélange. On obtient 350 mg d'une huile jaune, cette huile est repris dans un minimum de chlorure de méthylène et précipité dans l'éther isopropylique, on obtient ainsi le sel de trifluoroacétate impur. On renouvelle la précipitation dans l'éther jusqu'à purification (controlée en CCM). On obtient finalement 156 mg du produit attendu (Rdt = 71 %) sous la forme d'une poudre blanche

CCM : Rf = 0,4 (silicagel, éluant : dichlorométhane-méthanol-eau-acide acétique 90-10-1-1).
**1H-RMN (MeOD) :** δ 1,85 (m, 2H, NH-CH2-CH2-CH2); 2,00 (m, 4H, N-CH2-CH2-CH-CH2); 2,83 (t, 2H, NH-CH2-CH2-CH2); 3,90 (m, 1H, CH2-CH-CH2); 3,12 et 3.72 (2m, 4H, CH2-CH2-N-CH2-CH2); 3,52 (t, 2H, NH-CH2-CH2-CH2); 3.62 (s, 3H, CH3-O); 4,52 (m, 2H, NH-CH2-CH-NH); 4,60 (m, 1H, NH-CH2-CH-NH); 5.06 et 5, 10 (syst AB, 2H, O-CH2-Ph); -6,65 et 7,62 (2d, 2H, CH=CH naphthyridine); 7,43 (m, 5H, Ph); 8,00 (s, 1H, N=CH-N).
**HPLC/SM :** (tr = 0,5 min et 2,75 min) : 562 (MH+); 428 [MH -(tBu-Z+)]; 428

**Exemple de référence 18**

Synthèse de la 4,6-dibromo-5-fluoro-pyrimidine.

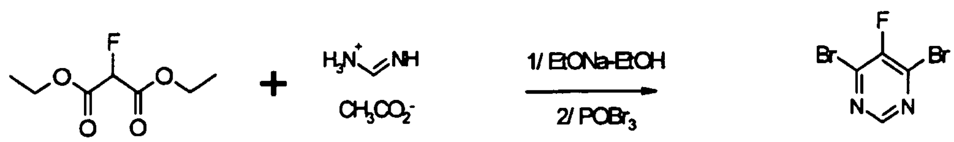


1/ A une solution de 5,83 g (56 mmoles) d'acétate de formamidine dans 300 ml d'éthanol refroidie à 0°C on ajoute 60,7 ml (168 mmoles) d'une solution d'éthylate de sodium à 21 % dans l'éthanol et agite le mélange pendant 30 minutes; on additionne alors une solution de 10 g (56 mmoles) de fluoro malonate de diéthyle dans 25 ml d'éthanol et agite une nuit à température ambiante. Le mélange est refroidi à 0°C puis on ajoute 17,85 ml d'acide chlorhydrique concentré pour amener le pH à 6. Le précipité est filtré puis lavé successivement par de l'eau, de l'isopropanol, de l'éther diéthylique et enfin du pentane. On obtient 14,2 g (théorie = 7,3 g) de produit attendu et de sels minéraux utilisé tel quel pour la suite.
2/ Un mélange de 14,2 g (56 mmoles) de 5-fluoro-4,6-dihydroxy-pyrimidine dans 21 g d'oxybromure de phosphore est porté à 200°C durant 3 heures. Après retour à température ambiante, le mélange réactionnel est repris par un mélange d'eau glacée de bicarbonate de sodium et extrait à l'acétate d'éthyle, lave les phases organiques avec de l'eau, sèche sur sulfate de magnésium et évapore à sec sous pression réduite (2 kPa). Le résidu est chromatographié sur silicagel en éluant avec un gradient de chlorure de méthylène-heptane de 0-100 à 100-0. On obtient 850 mg (Rdt = 06 %) de produit attendu sous forme de solide beige utilisé tel quel pour la suite.

**CCM :** Rf = 0,50 (silicagel, éluant : dichlorométhane-heptane 50-50)

Synthèse 4-bromo-5-fluoro-6-[4-(1,2,3,4-tétrahydro-1,8-naphthyridin-7-yl)-1-pipéridinyl]-pyrimidine

Un mélange de 840 mg (1,5 mmoles) de 1,2,3,4-tétrahydro-7-(4-pipéridinyl)-1,8-naphthyridine, tris(trifluoroacetate) (préparé selon les brevets EP1065207 ou WO 0078317) et de 5 g de résine Amberlyst A-21 (origine Fluka 06424 préalablement lavée avec la solution dichlorométhane-acétate d'éthyle-méthanol 1-1-1) dans 100 ml de solution de dichlorométhane-acétate d'éthyle-méthanol 1-1-1 est agité à température ambiante durant 1 heure. Le mélange est filtré, la résine lavée avec la solutin ternaire. Le filtrat obtenu est agité à température ambiante durant 1 heure en présence de 5 g de résine Amberlyst A-21 traitée comme précédemment. Le filtrat ainsi obtenu est concentré à sec sous pression réduite (2 kPa) donnant 320 mg d'amine libre. On rajoute à ce résidu 384 mg (1,5 mmoles) de 4,6-dibromo-5-fluoro-pyrimidine, 6 ml de diméthylacétamide et 1,5 ml de diisopropyléthylamine et porte à 130°C durant 1,5 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec heptane-acétate d'éthyle 50-50. On obtient 300 mg (Rdt = 52 %) de produit attendu sous forme d'un solide amorphe.
**CCM :** Rf = 0,15 (silicagel, éluant : heptane-acétate d'éthyle 50-50).
**IR (CHCl3) :** 3440 (NH); 1573, 1542, 1503 cm-1 (Hétérocycle)
**1H-RMN (CDCl3) :** δ 1, 82, 1, 92 et 2,02(dt, t et d 6H, $CH_2$-$CH_2$-$CH_2$-NH et $CH_2$-CH-$CH_2$); 2,72 (t, 2H, $CH_2$-$CH_2$-$CH_2$-NH); 2,80 (tl, 1H, $CH_2$-CH-$CH_2$); 3,13 et 4,68 (tl et d, 4H, $CH_2$-$CH_2$-N-$CH_2$-$CH_2$); 3,42 (m, 2H, $CH_2$-$CH_2$-$CH_2$-NH); 5,00 (m, 1H NH); 6,37 (d, 1H, naphthyridine); 7,14 (d, 1H, naphthyridine); 8,11 ppm (s,N=CH-N).
**SM :** 392 (MH+).

Synthèse du 3-[[5-fluoro-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle).

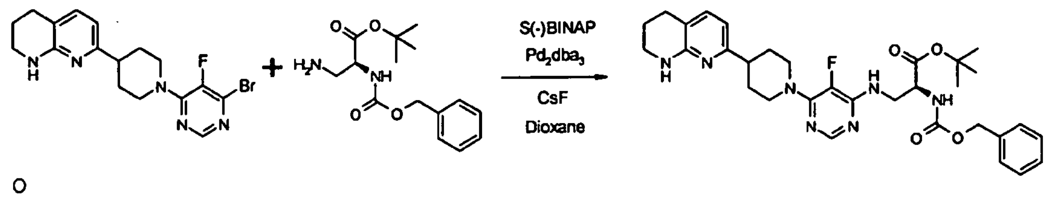


O

n chauffe au reflux un mélange de 280 mg (0,72 mmole) de 4-bromo-5-fluoro-6-[4-(1,2,3,4-tétrahydro-1,8-naphthyridin-7-yl)-1-pipéridinyl]-pyrimidine, de 253 mg (0,86 mmole) de 3-amino-N-[(phénylméthoxy)carbonyl]alaninate de (1,1-diméthyléthyle) (préparé selon J. Med. Chem.(2001), 44(8), 1158-1176), de 152 mg (1,00 mmole) de fluorure de césium, de 33 mg (0,036 mmole) de tris(dibenzylidèneacétone)dipalladium(0), de 45 mg (0,072 mmole) de 2,2'-bis (diphényl-phosphino)-1,1'-binaphtyl dans 50 ml de dioxane pendant 6 heures. On refroidit puis ajoute à nouveau 125 mg (0,43 mmole) de 3-amino-N-[(phénylméthoxy)carbonyl]alaninate de (1,1-diméthyléthyle), 152 mg (1,0 mmole) de fluorure de césium, 33 mg (0,036 mmole) de tris(dibenzylidèneacétone)dipalladium(0), et 45 mg (0,072 mmole) de 2,2'-bis(diphényl-phosphino)-1,1'-binaphtyl et chauffe au reflux pendant 4 heures. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'heptane-acétate d'éthyle-methanol de 100-0-0 à 0-100-0 et enfin 0-95-5. Le produit obtenu est chromatographié une deuxième fois sur alumine en éluant avec un mélange d'heptane-chlorure de méthylène 50-50 puis à l'acétate d'éthyle-ether diisopropylique 50-50. On obtient 300 mg (Rdt = 70 %) de produit attendu sous forme d'une huile jaune.
**CCM :** Rf = 0,35 (silicagel, éluant : acétate d'éthyle.
**IR (CHCl3) :** 3439 (NH); 1718 (C=O); 1609,1503 cm-1 (Hétérocycle+Aromatique+Amide).
**1H-RMN (CDCl3) :** $\delta$ 1,47 (s, 9H, tBu); 1,70 à 2,05 (m, 6H, $CH_2$-$CH_2$-$CH_2$-NH et $CH_2$-CH-$CH_2$); 2,73 (t, 2H, $CH_2$-$CH_2$-$CH_2$-NH); 2,80 (tl, 1H, $CH_2$-CH-$CH_2$); 3,01 et 3,90 (tl et m, 4H, $CH_2$-$CH_2$-N-$CH_2$-$CH_2$); 3,43 (m, 2H, $CH_2$-$CH_2$-$CH_2$-NH); ; 9,42 (m, 1H, NH-$CH_2$-CH-NH) ; 4,51 (dl, 2H, NH-$CH_2$-CH-NH); 5,11 (m, 1H NH); 5,14 (s, 2H, $CH_2$-Ph); 6,12 (m, 1H $CH_2$-$CH_2$-$CH_2$-NH); 6,37 (d, 1H, naphthyridine); 7,16 (d, 1H, naphthyridine); 7,35 (m, 5H, Ph); 7,98 ppm (s,N=CH-N).
**SM :** 606 (MH+); 550 (MH-tBu+); 416 (550-COOCH2Ph+), 604- [(M-H)]-, 650- [(604+HCOOH)]-, 496- [(604-OCH2Ph)]-.
**[$\alpha_D$] (1,0 % CHCl3) :** +4,4

Synthèsedela3-[[5-fluoro-6-[4-(1,2,3,4-tétrahydro-1,8-naphthyridin-7-yl)-1-pipéridinyl]-4-pyrimidinyl]amino]-N-[(phénylméthoxy)carbonyl]alanine, bis(trifluoroacetate).

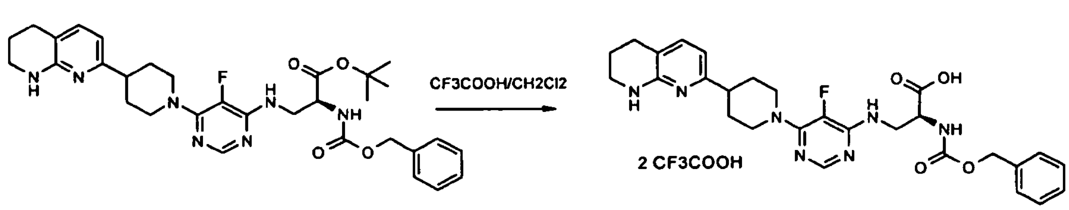


On agite 290 mg (0,48 mmole) de 3-[[5-fluoro-6-[4-(1,2,3,4-tétrahydro-1,8-naphthyridin-7-yl)-1-pipéridinyl]-4-pyrimidinyl]amino]-N-[(phénylméthoxy)carbonyl]alaninate de (1,1-diméthyléthyle) dans 40 ml de dichlorométhane avec 4 ml d'acide trifluoroacétique à température ambiante jusqu'à disparition du produit de départ en CCM (silicagel, éluant : CH2Cl2-MeOH-H2O-AcOH 90-10-1-1), On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 310 mg (Rdt=83% exprimé en ditrifluoroacétate) de produit

attendu sous forme d'un solide blanc.
**CCM :** Rf = 0,44 (silicagel, éluant : dichlorométhane-méthanol-eau-acide acétique 90-10-1-1)
**SM :** 550 (MH+); 416 (MH-COOCH2Ph+); 548- (M-H-); 440- (548-OCH2Ph-); 1097- (2M-H-)
**[$\alpha_D$] (0,30 % MeOH) :** -9,3.

**Exemple de référence 19**

Synthèse du 3-[5-éthyl-6-[4-(1,2,3,4-tétrahydro-1,8-naphthyridin-7-yl)-1-pipéridinyl]-4-pyrimidinyl]amino]alaninate de (1,1-diméthyléthyle).

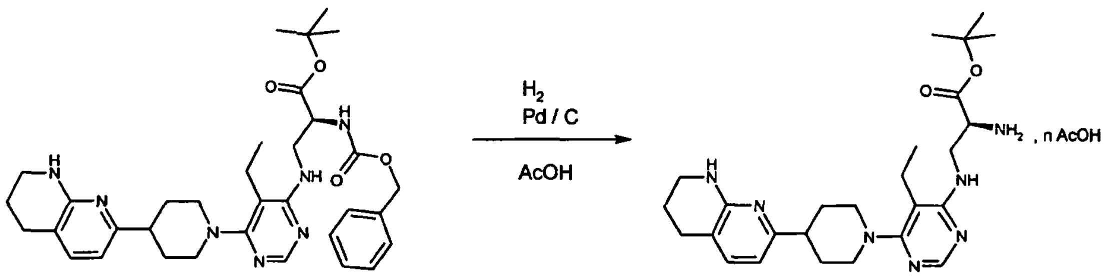


Dans un monocol contenant 3 g (4.87 mmoles) de 3-[[6-[4-(1,2,3,4-tetrahydro-1,8-naphthyridin-7-yl)-1-pipéridinyl]-5-éthyl-4-pyrimidinyl]amino]-N-[(phénylméthoxy) carbonyl]alanine, on charge 150 ml d'acide acétique 100 % et
300 mg de palladium activé sur charbon (5-10 %). Ce mélange est purgé sous pression réduite (2 kPa) puis on le laisse agiter à température ambiante et sous pression atmosphérique d'hydrogène pendant 22 heures.
Le milieu hétérogène obtenu est filtré sur clarcel. Le filtrat est concentré à sec puis repris par un mélange d'eau, d'acétate d'éthyle et d'une solution de bicarbonate de sodium saturée. La phase organique est extraite à l'acétate d'éthyle et séchée sur sulfate de magnésium puis le solvant est évaporé sous pression réduite (2 kPa). On obtient 1,95 g d'une huile jaune pâle.
CCM : Rf = 0,65 (silicagel, éluant : acétate d'éthyle-méthanol-triéthylamine 90-5-5)
**1H-RM (CDCl3) :** δ 1,09 (t, 3H, $CH_2$-$\underline{CH_3}$); 1,35 (s, 9H, tBu); 1,75 (m, 6H, $\underline{CH_2}$-CH-$\underline{CH_2}$ et $CH_2$-$\underline{CH_2}$-$CH_2$-NH); 2,44 (m, 2H, $\underline{CH_2}$-$CH_3$); 2,60 (t, 2H, $\underline{CH_2}$-$CH_2$-$CH_2$-NH); 2,81 (m, 2H, $CH_2$-$\underline{CH}$-$CH_2$ et NH-$CH_2$-$\underline{CH}$-NH); 3,23 (m, 2H, $CH_2$-$CH_2$-$\underline{CH_2}$-NH); 3, 41 (m, 4H, $CH_2$-$\underline{CH_2}$-N-$\underline{CH_2}$-$CH_2$); 3,42 et 3,57 (2m, 2H, NH-$\underline{CH_2}$-CH-NH); 6,25 (m, 2H, $CH_2$-$CH_2$-$CH_2$-$\underline{NH}$ et $\underline{NH}$-$CH_2$-CH-NH); 6,30 et 7,04 (2d, 2H, H naphthyridine); 8,08 ppm (s, 1H, N=$\underline{CH}$-N).
**IR (CHCl3) :** 3439 (NH); 1726 (C=O); 1580, 1502 cm-1 (heterocycle).
**SM :** $482^+$ $[MH]^+$; $426^+$ $[MH^+ - tBu]^+$; $339^+$ $[MH^+ - CH_2CH(NH_2)CO_2tBu]^+$; $241.7^+$ $[M+2H]^2$
**[$\alpha_D$] :** -3 (1 % CHCl3)

Synthèsedu3-[[5-éthyl-6-[4-(1,2,3,4-tétrahydro-1,8-naphthyridin-7-yl)-1-pipéridinyl]-4-pyrimidinyl]amino]-N-[(adamantylméthoxy)carbonyl]alaninate de (1,1-diméthyléthyle).

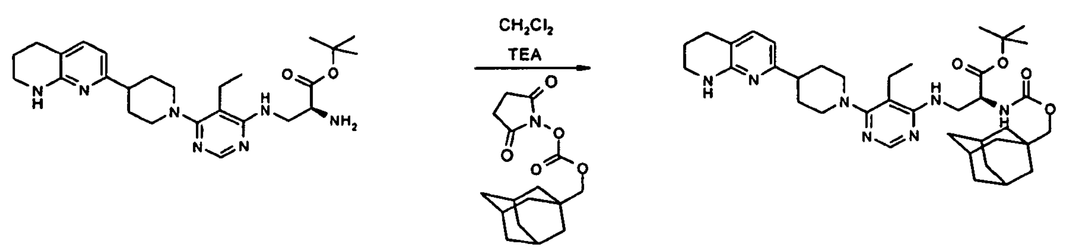


Un mélange de 239 mg (0,49 mmoles) de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle), 152 mg (0,49 mmoles) de N-adamantylméthoxycarbonyloxysuccinimide et de 0,104 ml (0,75 mmoles) de triéthylamine dans 50 ml de chlorure de méthylène est agité durant 5 heures à température ambiante. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient de chlorure de méthylène 100 % jusqu'à chlorure de méthylène-méthanol 90-10. On obtient 77 mg (Rdt = 17 %) de produit attendu sous forme d'un solide beige.

**CCM :** Rf = 0,45 (silicagel, éluant : chlorure de méthylène-méthanol-acide acétique-eau 90-10-1-1).

**IR (CHCl3) :** 3437 (NH); 1711 (C=O); 1583, 1556, 1501 cm-1 (Hétérocycle)

**1H-RMN (DMSO-d6) :** δ 1,08 (t, 3H, $CH_2$-$CH_3$); 1, 45 à 1,92 (m, 15H, adamantyl); 1,32 (s, 9H, tBu); 1,77 (m, 6H, $CH_2$-CH-$CH_2$ et $CH_2$-$CH_2$-$CH_2$-NH); 2,44 (q, 2H, $CH_2$-$CH_3$); 2,50 (m, 1H, $CH_2$-CH-$CH_2$); 2,61 (t, 2H, $CH_2$-$CH_2$-$CH_2$-NH); 2,82 et 3,44 (2m, 4H, $CH_2$-$CH_2$-N-$CH_2$-$CH_2$); 3,22 (m, 2H, $CH_2$-$CH_2$-$CH_2$-NH); 3,56 (s, 2H, O-$CH_2$-adam); 3,66 (m, 2H, NH-$CH_2$-CH-NH); 4,17 (q, 1H, NH-$CH_2$-CH-NH); 6,24 (s, 1H, $CH_2$-$CH_2$-$CH_2$-NH); 6,30 et 7,05 (2d, 2H, H naphthyridine); 6,37 (t, 1H, NH-$CH_2$-CH-NH); 7,44 (d, 1H, NH-$CH_2$-CH-NH); 8,10 ppm (s,N=CH-N).

**SM :** 674 (MH+); 618 (MH-tBu+); 426 (618-COOCH2adam+); 337,7 (M2H++); 718- (M-H-HCOOH); 672- (M-H).

**[α]$_D$ :** +2,5 (1% CHCl3).

Synthèsedu3-[[5-éthyl-6-[4-(1,2,3,4-tétrahydro-1,8-naphthyridin-7-yl)-1-pipéridinyl]-4-pyrimidinyl]amino]-N-[(adamantylméthoxy)carbonyl]alanine

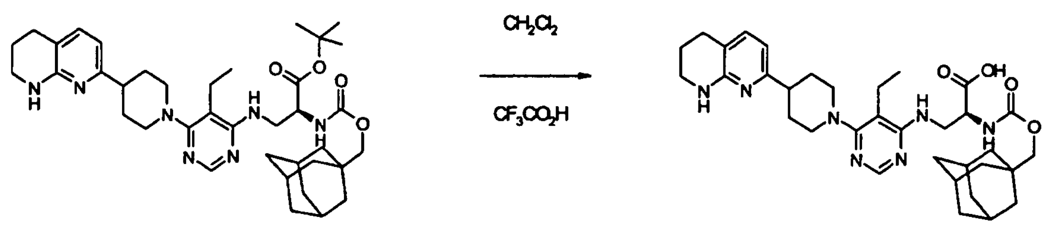


On agite 130 mg (0,19 mmoles) de 3-[[5-éthyl-6-[4-(1,2,3,4-tétrahydro-1,8-naphthyridin-7-yl)-1-pipéridinyl]-4-pyrimidinyl]amino]-N-[(adamantylméthoxy)carbonyl]alaninate de (1,1-diméthyléthyle) dans 20 ml de dichlorométhane avec 2 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 368 mg (Rdt = 61 % exprimé en ditrifluoroacétate) de produit attendu sous forme d'un solide amorphe.

**CCM :** Rf = 0,35 (silicagel, éluant : dichlorométhane-méthanol-eau-acide acétique 90-10-1-1)

**IR (CHCl3) :** 3401 (NH); 1668 (C=O); 1581, 1492 cm-1 (Hétérocycle)

**1H-RMN (DMSO-d6) :** δ 1,08 (t, 3H, $CH_2$-$CH_3$); 1, 45 à 1, 92 (m, 15H, adamantyl); 1,76 (m, 6H, $CH_2$-CH-$CH_2$, $CH_2$-$CH_2$-$CH_2$-NH); 2,43 (q, 2H, $CH_2$-$CH_3$); 2,50 (m, 1H, $CH_2$-CH-$CH_2$); 2,60 (t, 2H, $CH_2$-$CH_2$-$CH_2$-NH); 2,82 et 3,45 (2m, 4H, $CH_2$-$CH_2$-N-$CH_2$-$CH_2$); 3,23 (m, 2H, $CH_2$-$CH_2$-$CH_2$-NH); 3,49 et 3,58 (m, 2H, O-$CH_2$-adam); 3,56 et 3,76 (m, 2H, NH-$CH_2$-CH-NH); 4,18 (m, 1H, NH-$CH_2$-CH-NH); 6,26 (s, 1H, $CH_2$-$CH_2$-$CH_2$-NH); 6,30 et 7,05 (2d, 2H, H naphthyridine); 6,44 (m, 1H, NH-$CH_2$-CH-NH); 7,25 (d, 1H, NH-$CH_2$-CH-NH); 8,11 ppm (s,N=CH-N).

**SM :** 618 (MH+); 426 (MH-COOCH2Ph+); 1235 (2MH+); 309,8 (M2H++); 616-(M-H-)

**[α$_D$] :** (0,7 % CH3OH) -2,8

**Exemple de référence 20**

Synthèse du 3-[5-éthyl-6-[4-(1,2,3,4-tétrahydro-1,8-naphthyridin-7-yl)-1-pipéridinyl]-4-pyrimidinyl]amino]alaninate de (1,1-diméthyléthyle).

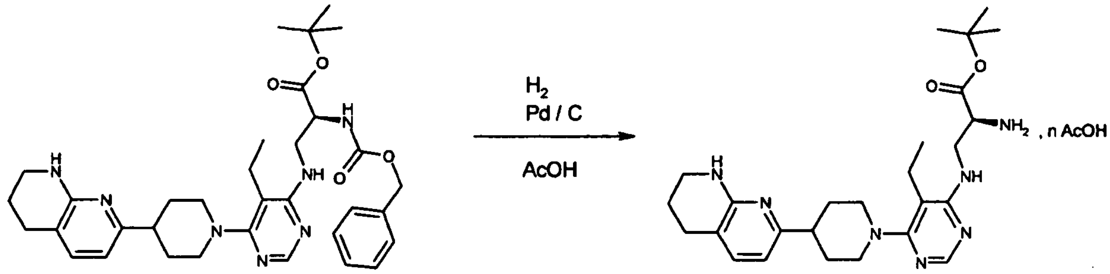


Dans un monocol contenant 3 g (4.87 mmoles)de 3-[[6-[4-(1,2,3,4-tetrahydro-1,8-naphthyridin-7-yl)-1-pipéridinyl]-5-éthyl-4-pyrimidinyl]amino]-N-[(phénylméthoxy)carbonyl]alanine, on charge 150 ml d'acide acétique 100 % et

300 mg de palladium activé sur charbon (5-10 %). Ce mélange est est purgé sous pression réduite (2 kPa) puis on le laisse agiter à TA sous pression atmosphérique d'hydrogène pendant 22 heures.

Le milieu hétérogène obtenu est filtré sur clarcel. Le filtrat est concentré à sec sous pression réduite (2 kPa) puis repris par un mélange d'eau, d'acétate d'éthyle et d'une solution de bicarbonate de sodium saturée. La phase organique est extraite à l'acétate d'éthyle et séchée sur sulfate de magnésium puis le solvant est évaporé sous pression réduite (2 kPa). On obtient 1,95 g d'une huile jaune pâle.
**CCM** rf = 0.65 (silicagel éluant AcOEt-MeOH-TEA = 90-5-5)
$\alpha_d$ = -3 (1% $CHCl_3$)
**1H-RMN (CDCl3) :** δ 1,09 (t, 3H, $CH_2$-$\underline{CH_3}$); 1,35 (s, 9H, tBu); 1,75 (m, 6H, $\underline{CH_2}$-CH-$\underline{CH_2}$ et $CH_2$-$\underline{CH_2}$-$CH_2$-NH); 2, 44 (m, 2H, $\underline{CH_2}$-$CH_3$); 2, 60 (t, 2H, $\underline{CH_2}$-$CH_2$-$CH_2$-NH) ; 2,81 (m, 2H, $CH_2$-$\underline{CH}$-$CH_2$ et NH-$CH_2$-$\underline{CH}$-NH); 3,23 (m, 2H, $CH_2$-$CH_2$-$\underline{CH_2}$-NH); 3,41 (m, 4H, $CH_2$-$\underline{CH_2}$-N-$\underline{CH_2}$-$CH_2$); 3,42 et 3,57 (2m, 2H, NH-$\underline{CH_2}$-CH-NH); 6,25 (m, 2H, $CH_2$-$CH_2$-$CH_2$-$\underline{NH}$ et $\underline{NH}$-$CH_2$-CH-NH); 6,30 et 7,04 (2d, 2H, H naphthyridine); 8,08 ppm (s, 1H, N=$\underline{CH}$-N).
**IR (CHCl3)** 3439 (NH); 1726 (C=O); 1580 - 1502 (heterocycle)
**HPLC** / **SM** 482$^+$ [MH]$^+$; 426$^+$ [MH$^+$ - tBu]$^+$; 339$^+$ [MH$^+$ - $CH_2CH(NH_2)CO_2tBu$ ]$^+$; 241.7$^+$ [M+2H]$^2$

**Exemples** de référence **21 à 31**

**Exemple** de référence **28**

**Etape a)**

Synthèse du 3-[[5-éthyl-6-[4-(1,2,3,4-tétrahydro-1,8-naphthyridin-7-yl)-1-pipéridinyl]-4-pyrimidinyl]amino]-N-(4-méthoxy-benzènesulfonyl)alaninate de (1,1-diméthyléthyle).

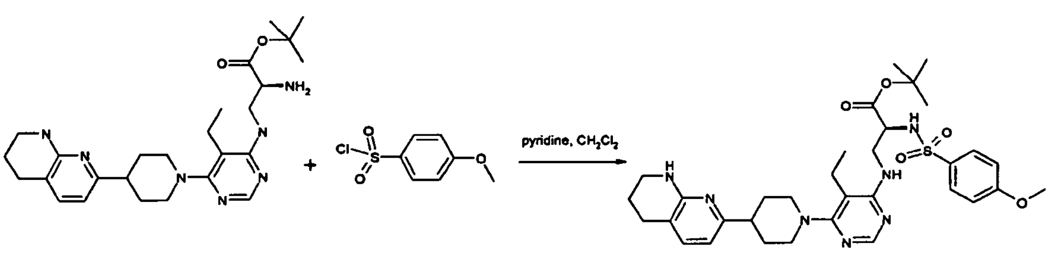


A un mélange de 150 mg (0,31 mmoles) de 3-[5-éthyl-6-[4-(1,2,3,4-tétrahydro-1,8-naphtiridin-7-yl)-1-pipéridinyl]-4-pyrimidinyl]amino]alaninate de (1,1-diméthyléthyle) en solution dans 6 ml de dichlorométhane et 650 µl de pyridine, on additionne 64,3 mg (0,31 mmoles)de chlorure de 4-méthoxy-benzènesulfonyle en solution dans 3 ml de dichlorométhane. Le mélange réactionnel est agité à température ambiante pendant 5 heures. Puis, le solvant est évaporé sous pression réduite (2 kPa) et le résidu est chromatographié sur silicagel avec l'éluant suivant : acétate d'éthyle-dichlorométhane/méthanol(95/5) 50-50 à acétate d'éthyle-méthanol 90-10. On obtient 55,8 mg (Rdt = 28 %) de produit attendu.
**CCM :** Rf = 0,63 (silicagel, éluant : dichlorométhane-méthanol-eau-acide acétique 85-15-2-2)
**1H-RMN ($CDCl_3$) :** δ 1,21 (t, 3H, CH2-$\underline{CH3}$); 1,30 (s, 9H, tBu); 1,92 et 2,02 (2m, 6H, N-CH2-$\underline{CH2}$-CH-$\underline{CH2}$-CH2, CH2-$\underline{CH2}$-CH2-NH); 2,48 (q, 2H, $\underline{CH2}$-CH3); 2,72 (m, 3H, N-CH2-CH2-$\underline{CH}$-CH2-CH2, $\underline{CH2}$-CH2-CH2-NH); 2,97 et 3,61

(2m, 4H, N-CH2-CH2-CH-CH2-CH2); 3,45 (m, 2H, CH2-CH2-CH2-NH); 3,74 et 3,86 et 3,95 (3m, 6H, NH-CH2-CH-NH, OCH3); 4,95 (t, 1H, NH mobile); 5,92 (dl, 1H, NH mobile); 6.43 et 7,20 (2d, 2H, CH=CH naphtiridine); 6,95 et 7,79 (2d, 4H, CH=CH benzène); 8,29 (s, 1H, N=CH-N).
**SM :** 652(MH+).

**Etape b)**

Synthèse du 3-[[5-éthyl-6-[4-(1,2,3,4-tétrahydro-1,8-naphthyridin-7-yl)-1-pipéridinyl]-4-pyrimidinyl]amino]-N-(4-méthoxy-benzènesulfonyl)alanine.

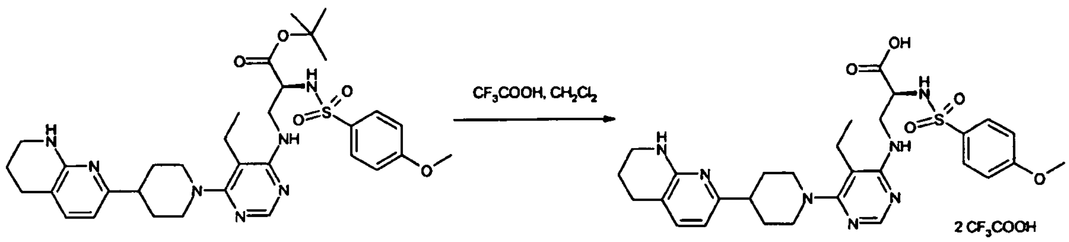


On agite 55,8 mg (0,086 mmoles) de 3-[[5-éthyl-6-[4-(1,2,3,4-tétrahydro-1,8-naphthyridin-7-yl)-1-pipéridinyl]-4-pyrimidinyl]amino]-N-(4-méthoxy-benzènesulfonyl)alaninate de (1,1-diméthyléthyle) dans 5 ml de dichlorométhane avec 0,5 ml d'acide trifluoroacétique à température ambiante jusqu'à disparition du produit de départ en CCM (silicagel, éluant : CH2C12-MeOH-H2O-AcOH 85-15-2-2). On rajoute du toluène et on évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 59,8 mg (Rdt = 85 % exprimé en ditrifluoroacétate) de produit attendu.
**CCM :** Rf = 0,33 (silicagel, éluant : dichlorométhane-méthanol-eau-acide acétique 85-15-2-2).
**1H-RMN (MeOD) :** δ 1,22 (t, 3H, CH2-CH3) ; 1,96 et 2,10 (2m, 6H, N-CH2-CH2-CH-CH2-CH2, CH2-CH2-CH2-NH); 2,55 (q, 2H, CH2-CH3); 2,85 (t, 2H, CH2-CH2-CH2-NH); 2,95 (m, 1H, N-CH2-CH2-CH-CH2-CH2); 3,20 et 3,67 et 3,92 (3m, 6H, N-CH2-CH2-CH-CH2-CH2, NH-CH2-CH-NH); 3,51 (m, 2H, CH2-CH2-CH2-NH); 4,24 (m, 1H, NH-CH2-CH-NH); 6.71 et 7,64 (2d, 2H, CH=CH naphtiridine); 6,99 et 7,74 (2d, 4H, CH=CH benzène); 8,27 (s, 1H, N=CH-N).
**SM :** 596(MH+).

Mode opératoire général de préparation des sulfonamides

**Etape a)**

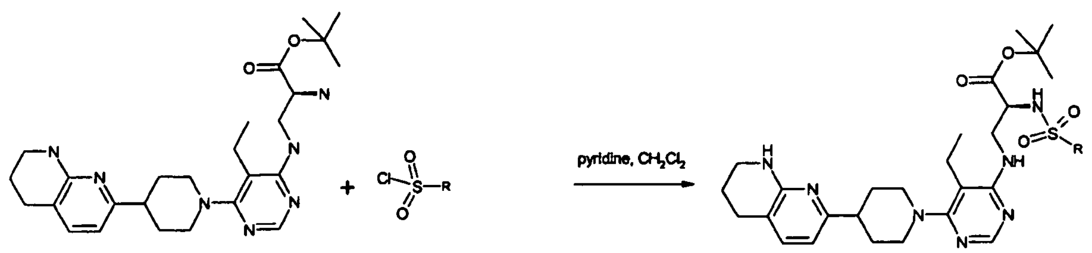


A un mélange de 150 mg (0,31 mmoles) de 3-[5-éthyl-6-[4-(1,2,3,4-tétrahydro-1,8-naphtiridin-7-yl)-1-pipéridinyl]-4-pyrimidinyl]amino]alaninate de (1,1-diméthyléthyle) en solution dans 6 ml de dichlorométhane et 650 µl de pyridine, on additionne 0,31 mmoles (masse $m_x$) de chlorure de sulfonyle en solution dans 3 ml de dichlorométhane. Le mélange réactionnel est agité à température ambiante pendant 5 heures. Puis, le solvant est évaporé sous pression réduite (2 kPa) et le résidu est chromatographié sur silicagel avec l'éluant suivant : acétate d'éthyle-dichlorométhane/méthanol(95/5) 50-50 à acétate d'éthyle-méthanol 90-10. On obtient une masse $m_y$ de produit attendu.

CCM : Rf (éluant : dichlorométhane-méthanol-eau-acide acétique 85-15-2-2).

Etape b)

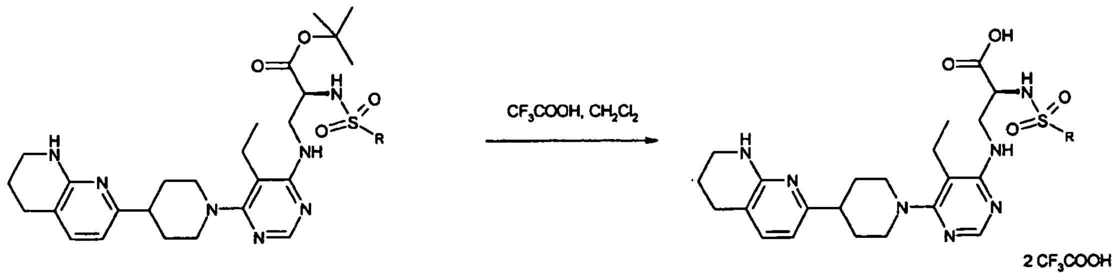


On agite une masse $m_y$ de 3-[[5-éthyl-6-[4-(1,2,3,4-tétrahydro-1,8-naphthyridin-7-yl)-1-pipéridinyl]-4-pyrimidinyl]amino]-N-(R-sulfonyl)alaninate de (1,1-diméthyléthyle) dans 5 ml de dichlorométhane avec 0,5 ml d'acide trifluoroacétique à température ambiante jusqu'à disparition du produit de départ en CCM (silicagel, éluant : CH2C12-MeOH-H2O-AcOH 85-15-2-2). On rajoute du toluène et on évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient une masse $m_z$ de produit attendu.

CCM : Rf (éluant : dichlorométhane-méthanol-eau-acide acétique 85-15-2-2).

| | Chlorure de sulfonyle | $m_x$ (mg) | Ester formé | $m_y$ (mg) | Rdt | SM (MH+) | Rf |
|---|---|---|---|---|---|---|---|
| Exemple de référence 21 | 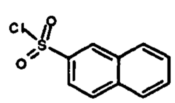 | 47.1 | 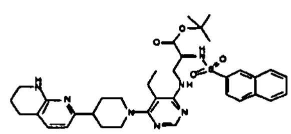 | 30 | 22 | 672 | 0.61 |
| Exemple de référence 22 | 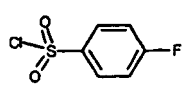 | 40.4 | 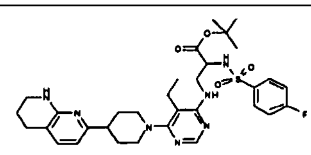 | 38.7 | 29 | 640 | 0.63 |
| Exemple de référence 23 | 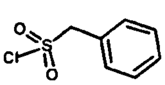 | 59.3 | 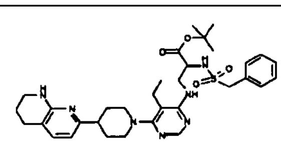 | 48 | 24 | 636 | 0.69 |
| Exemple de référence 24 | 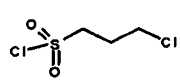 | 55.1 | 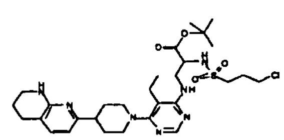 | 18.4 | 10 | 622-624 | 0.65 |
| Exemple de référence 25 | 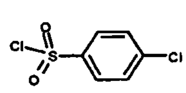 | 65.6 | 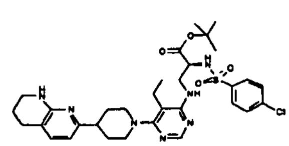 | 57.6 | 28 | 656-658 | 0.67 |

(suite)

| | Chlorure de sulfonyle | $m_x$ (mg) | Ester formé | $m_y$ (mg) | Rdt | SM (MH+) | Rf |
|---|---|---|---|---|---|---|---|
| Exemple de référence 26 | 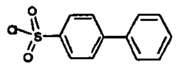 | 78.6 | 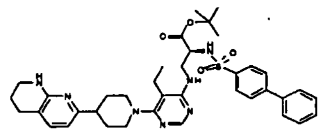 | 70 | 32 | 698 | 0.67 |
| Exemple de référence 27 | 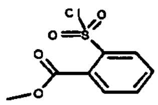 | 73.0 | 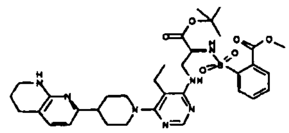 | 49.3 | 23 | 680 | 0.67 |
| Exemple de référence 29 | 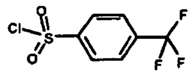 | 76.1 | 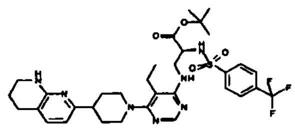 | 63.4 | 30 | 690 | 0.63 |
| Exemple de référence 30 | 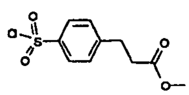 | 81.7 | 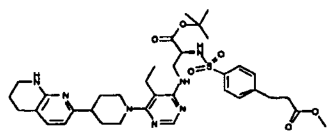 | 21.4 | 10 | 708 | 0.63 |
| Exemple de référence 31 | 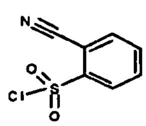 | 62.7 | 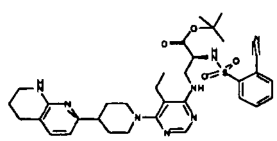 | 56.3 | 28 | 647 | 0.65 |
| $M_x$ : masse de chlorure de sulfonyle introduite.<br>$M_y$ : masse d'ester obtenue. | | | | | | | |

| | Acide obtenu | FW (base libre) | base libre + 2TFA | $m_z$ (mg) | SM (MH+) | Rf |
|---|---|---|---|---|---|---|
| Exemple de référence 21 | 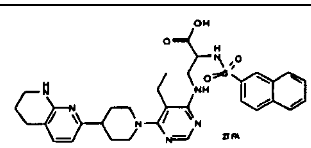 | 615.76 | 843.76 | 20.8 | 616 | 0.4 |
| Exemple de référence 22 | 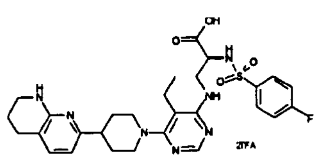 | 583.69 | 811.69 | 31.2 | 584 | 0.33 |
| Exemple de référence 23 | 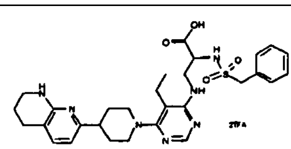 | 579.73 | 807.73 | 45.4 | 580 | 0.3 |
| Exemple de référence 24 | 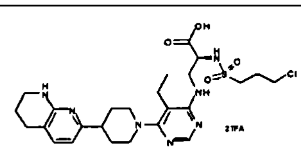 | 566.13 | 794.13 | 11.6 | 566-568 | 0.26 |

(suite)

| | Acide obtenu | FW (base libre) | base libre + 2TFA | $m_z$ (mg) | SM (MH+) | Rf |
|---|---|---|---|---|---|---|
| Exemple de référence 25 | 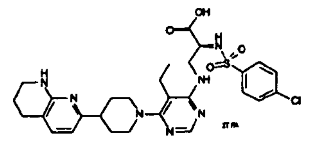 | 600.14 | 828.14 | 58.3 | 600-601 | 0.3 |
| Exemple de référence 26 | 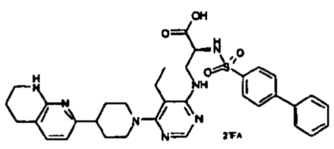 | 641.8 | 869.8 | 56.6 | 642 | 0.37 |
| Exemple de référence 27 | 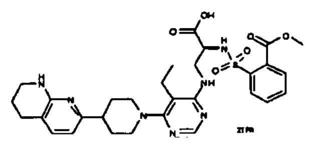 | 623.74 | 851.74 | 41.2 | 624 | 0.37 |
| Exemple de référence 29 | 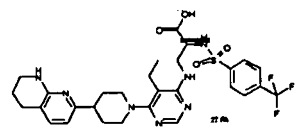 | 633.7 | 861.7 | 59.8 | 634 | 0.33 |
| Exemple de référence 30 | 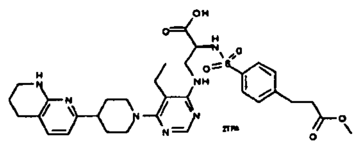 | 651.79 | 879.79 | 48.5 | 652 | 0.35 |
| Exemple de référence 31 | 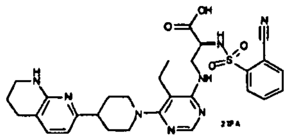 | 590.71 | 818.71 | 28 | 591 | 0.26 |
| $M_z$ : masse d'acide obtenue. | | | | | | |

**Exemple** de référence **32**

Synthèse du 3-[[5-éthyl-6-[4-(1,2,3,4-tétrahydro-1,8-naphthyridin-7-yl)-1-pipéridinyl]-4-pyrimidinyl]amino]-N-(1-naphtalènesulfonyl)alaninate de (1,1-diméthyléthyle).

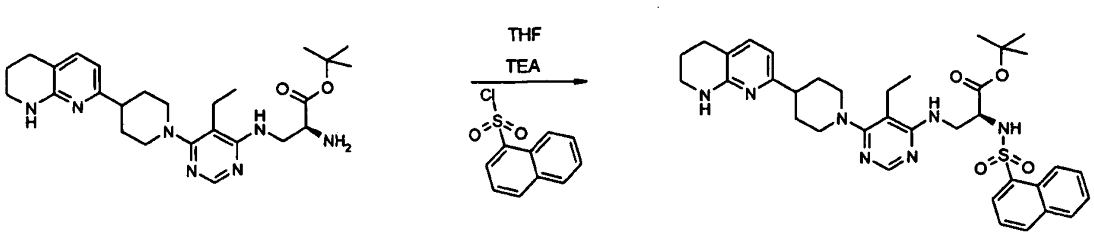


Un mélange de 239 mg (0,49 mmoles) de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle), 113 mg (0,5 mmoles) de chlorure de 1-naphtalènesulfonyle

et de 0,104 ml (0,75 mmoles) de triéthylamine dans 50 ml de tétrahydrofurane est agité durant 5 heures à température ambiante. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un gradient d'heptane-acétate d'éthyle 75-25 à 0-100. On obtient 220 mg (Rdt = 67 %) de produit attendu sous forme d'un solide blanc.

**CCM :** Rf = 0,10 (silicagel, éluant : chlorure de méthylène-méthanol-acide acétique-eau 90-10-1-1).

**IR (CHC13) :** 3440 (NH); 1728 (C=O); 1583, 1555, 1503 cm-1 (Hétérocycle+aromatique)

**1H-RMN (CDCl3) :** δ 1, 02 (t, 3H, $CH_2\underline{CH_3}$) ; 1,15 (s, 9H, tBu) ; 1, 81 à 2, 03 (m, 6H, $\underline{CH_2}$-CH-$\underline{CH_2}$ et_$CH_2$-$\underline{CH_2}$-$CH_2$-NH); 2, 23 et 2, 31 (2q, 2H, $\underline{CH_2}$-$CH_3$) ; 2,67 (t, 1H, $CH_2$-$\underline{CH}$-$CH_2$) ; 2,72 (t, 2H, $\underline{CH_2}$-$CH_2$-$CH_2$-NH); 2,93 et 3,56 (tl et dl, 4H, $CH_2$-$\underline{CH_2}$-N-$\underline{CH_2}$-$CH_2$) ; 3,43 (m, 2H, $CH_2$-$CH_2$-$\underline{CH_2}$-NH); 3,66 et 3,76 (2m, 2H, NH-$\underline{CH_2}$-CH-NH); 3,97 (m, 1H, NH-$CH_2$-$\underline{CH}$-NH); 4,73 (t, 1H, t, 1H, $\underline{NH}$-$CH_2$-CH-NH); 6,55 (s,1H, $CH_2$-$CH_2$-$CH_2$-$\underline{NH}$); 6,42 et 7,05(2d, 2H, H naphthyridine); 7,51 (t, 1H, H3 1-SO2naphtyl); 7,57 et 7,62 (2t, 2H, H6 et H7 1-SO2naphtyl); 7,90 (d, 1H, H2 1-SO2naphtyl); 8,02 (d, 1H, H4 1-SO2naphtyl) ; 8,22 (m, 2H, N=$\underline{CH}$-N et H5 1-SO2naphtyl); 8,61 ppm (m, 1H, H8 1-SO2naphtyl).

**SM :** 672 (MH+); 426 (616-SOOnapht+); 670- (M-H).

**$[\alpha]_D$ :** +3,3 (1% CHCl3).

Synthèse du 3-[[5-éthyl-6-[4-(1,2,3,4-tétrahydro-1,8-naphthyridin-7-yl)-1-pipéridinyl]-4-pyrimidinyl]amino]- N-(1-naphtalènesulfonyl)alanine

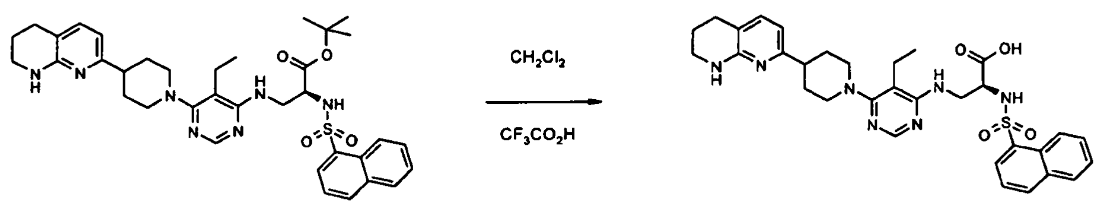


On agite 220 mg (0,33 mmoles) de 3-[[5-éthyl-6-[4-(1,2,3,4-tétrahydro-1,8-naphthyridin-7-yl)-1-pipéridinyl]-4-pyrimidinyl]amino]-N-(1-naphtalènesulfonyl)alaniriate de (1,1-diméthyléthyle) dans 20 ml de dichlorométhane avec 2 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 230 mg (Rdt = 82 % exprimé en ditrifluoroacétate) de produit attendu sous forme d'un solide blanc.

**CCM :** Rf = 0,10 (silicagel, éluant : dichlorométhane-méthanol-eau-acide acétique 90-10-1-1)

**IR (CHCl3) :** 3412-3257(OH, NH); 1668(C=O); 1628, 1582, 1505 cm-1(C=C, C=N, aromatique)

**1H-RMN (DMSO-d6) :** δ 0, 87 (t, 3H, $CH_2$-$\underline{CH_3}$) ; 1, 77 (m, 6H, $\underline{CH_2}$-cH-$\underline{CH_2}$, $CH_2$-$\underline{CH_2}$-$CH_2$-NH); 2,05 (q, 1H, $\underline{CH_2}$-$CH_3$); 2,16 (q, 1H, $\underline{CH_2}$-$CH_3$); 2,62 (t, 2H, $\underline{CH_2}$-$CH_2$-$CH_2$-NH); 2,73 et 3,50 (2m, 4H, $CH_2$-$\underline{CH_2}$-N-$\underline{CH_2}$-$CH_2$); 2,80 (m, 1H, $CH_2$-$\underline{CH}$-$CH_2$) ; 3,20 (m, 2H, $CH_2$-$CH_2$-$\underline{CH_2}$-NH); 3,50 (m, 2H, CH-$CH_2$-$\underline{CH_2}$-N) ; 3,66 et 3,76 (2m, 2H, NH-$\underline{CH_2}$-CH-NH); 3,97 (t, 1H, NH-$CH_2$-$\underline{CH}$-NH); 6,26 (s, 1H, $CH_2$-$CH_2$-$CH_2$-$\underline{NH}$); 6,31 et 7,06 (2d, 2H, H naphthyridine); 7,55 (t, 1H, H3 1-SO2naphtyl); 7,62 (m, 2H, H6 et H7 1-SO2naphtyl); 7,96 ppm (s,N=$\underline{CH}$-N); 8,02 (m, 1H, H5 1-SO2naphtyl); 8,08 (d, 1H, H2 1-SO2naphtyl) 8,14 (d, 1H, H4 1-SO2naphtyl); 8,58 (m, 1H, H8 1-SO2naphtyl).

**SM :** 616 (MH+); 426 (MH-SOOnapht+) ; 1231 (2MH+); 308,8 (M2H++); 614-(M-H-)

**$[\alpha_D]$ :** (0,4 % CH3OH) : +7,0

## Exemple de référence 33

Synthèse du 3-[[5-éthyl-6-[4-(1,2,3,4-tétrahydro-1,8-naphthyridin-7-yl)-1-pipéridinyl]-4-pyrimidinyl]amino]-N-(2-pyridinyl)alaninate de (1,1-diméthyléthyle).

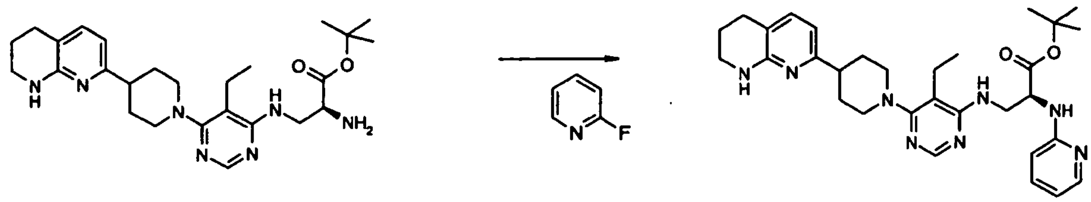

Un mélange de 240 mg (0,50 mmole) de 3-[5-éthyl-6-[4-(1,2,3,9-tétrahydro-1,8-naphthyridin-7-yl)-1-pipéridinyl]-4-pyrimidinyl]amino] alaninate de (1,1-diméthyléthyle) et de 3 ml de 2-fluoropyridine est agité durant 24 heures au reflux. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié sur alumine en éluant avec un mélange d'acétate d'éthyle-chlorure de méthylène-méthanol 80-16-4. On obtient 13 mg (Rdt = 05 %) de produit attendu sous forme d'huile.
**CCM :** Rf = 0,40 (silicagel, éluant : chlorure de méthylène-méthanol 90-10).
**SM :** 559 (MH+).

Synthèse du 3-[[5-éthyl-6-[4-(1,2,3,4-tétrahydro-1,8-naphthyridin-7-yl)-1-pipéridinyl]-4-pyrimidinyl]amino]- N-(2-pyridinyl)alanine

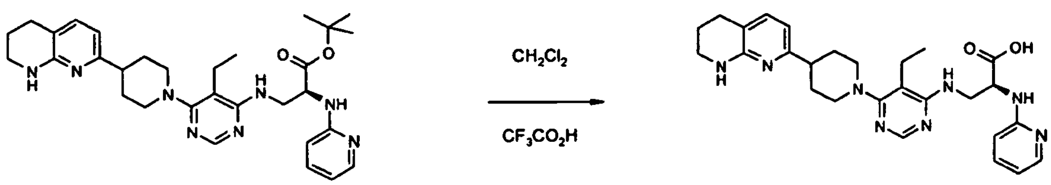


On agite 13 mg (0,023 mmole) de 3-[[5-éthyl-6-[4-(1,2,3,4-tétrahydro-1,8-naphthyridin-7-yl)-1-pipéridinyl]-4-pyrimidinyl]amino]-N-(2-pyridinyl)alaninate de (1,1-diméthyléthyle) dans 2 ml de dichlorométhane avec 0,2 ml d'acide trifluoroacétique à température ambiante jusqu'à disparition du produit de départ en CCM (silicagel, éluant : CH2C12-MeOH-H2O-AcOH 90-10-1-1). On rajoute du toluène et évapore le mélange réactionnel à sec sous pression réduite (2 kPa). Le résidu est solubilisé dans le minimum de dichlorométhane puis versé sur de l'éther diisopropylique. Le précipité est filtré. On obtient 13 mg (Rdt = 76 % exprimé en ditrifluoroacétate) de produit attendu sous forme d'un solide beige.
**CCM :** Rf = 0,35 (silicagel, éluant : dichlorométhane-méthanol-eau-acide acétique 85-15-2-2)
**SM :** 503 (MH+); 501- (M-H-)

## Exemple de référence 34

Synthèse du 3-[[5-éthyl-6-[4-(1,2,3,4-tétrahydro-1,8-naphthyridin-7-yl)-1-pipéridinyl]-4-pyrimidinyl]amino]-N-(2-benzothiazolyl)alaninate de (1,1-diméthyléthyle).

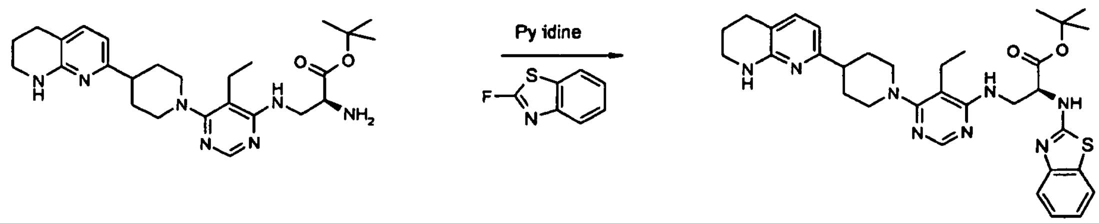

Un mélange de 200 mg (0,42 mmole) de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle) et de 90mg (0,6 mmole) de 2-fluorobenzothiazol dans 5 ml de pyridine est agité durant 2 heures à 100°C. Le mélange réactionnel est évaporé à sec sous pression réduite (2 kPa) et le résidu est repris par de l'acétate d'éthyle, de l'eau et une solution saturée de bicarbonate de sodium. La phase organique est séparée, séchée sur sulfate de magnésium et le solvant évaporé sous pression réduite (2 kPa). Le résidu est chromatographié une première fois sur silicagel en éluant avec un gradient de chlorure de méthylène-acétate d'éthyle-méthanol 100-0-0 à 0-100-0 puis 0-95-5; puis une deuxième fois sur phase inverse RP8 en éluant avec un gradient de méthanol-eau 80-20 à 100-0. On obtient 50 mg (Rdt=19 %) de produit attendu sous forme d'un solide rosé.
**CCM :** Rf= 0,20 (silicagel, éluant : acétate d'éthyle-méthanol 98-2).
**1H-RMN (CDCl3) :** δ 1,06 (t, 3H, $CH_2CH_3$); 1, 49 (s, 9H, tBu); 1,77 à 2,01(m, 6H, $CH_2$-CH-$CH_2$et $CH_2$-$CH_2$-$CH_2$-NH) ; 2, 41 (q, 2H, $CH_2$-$CH_3$); 2,61 (t, 1H, $CH_2$-CH-$CH_2$); 2,72 (t, 2H, $CH_2$-$CH_2$-$CH_2$-NH) ; 2,92 et 3, 51 (ql et tl, 4H, $CH_2$-$CH_2$-N-$CH_2$-$CH_2$); 3,42 (m, 2H, $CH_2$-$CH_2$-$CH_2$-NH) : 3, 95 et 4,09 (2m, 2H, NH-$CH_2$-CH-NH) ; 4,88 (m, 1H, NH-$CH_2$-CH-NH); 4,98 et 5,63 (m et .t, 2H, NH-$CH_2$-CH-NH et NH-$CH_2$-CH-NH) ; 6,67 (sl, 1H, $CH_2$-$CH_2$-$CH_2$-NH); 6,41 et 7, 13 (2d, 2H, H naphthyridine); 7,12, 7,31 et 7,57 (t masqué, t et tl 4H, H benzothiazol): 8,32ppm (s, 1H, N=CH-N) .
**SM :** 615 (MH+); 559 (MH-tBu+); 613- (M-H-).

Synthèse du 3-[[5-éthyl-6-[4-(1,2,3,4-tétrahydro-1,8-naphthyridin-7-yl)-1-pipéridinyl]-4-pyrimidinyl]amino]- N-(2-benzothiazolyl)alanine

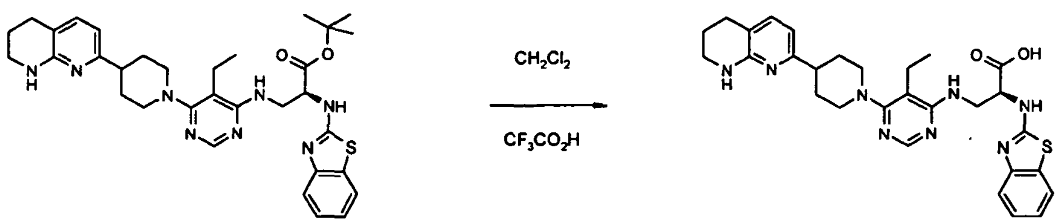


On agite 45 mg (0,073 mmole) de 3-[[5-éthyl-6-[4-(1,2,3,4-tétrahydro-1,8-naphthyridin-7-yl)-1-pipéridinyl]-4-pyrimidinyl]amino]-N-(2-benzothiazolyl)alaninate de (1,1-diméthyléthyle) dans 10 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 60 mg (Rdt = 97 % exprimé en ditrifluoroacétate) de produit attendu sous forme d'un solide blanc.
**CCM :** Rf = 0,45 (silicagel, éluant : dichlorométhane-méthanol-eau-acide acétique 85-15-2-2)
**1H-RMN (CDCl3) :** δ 1,15 (t, 3H, $CH_2$-$CH_3$) ; 1,75 à 2, 07 (m, 6H, $CH_2$-CH-$CH_2$, $CH_2$-$CH_2$-NH); 2,51 (q, 1H, $CH_2$-$CH_3$); 2,76 (tl, 2H, $CH_2$-$CH_2$-$CH_2$-NH); 2,96 (m, 1H, $CH_2$-CH-$CH_2$); 3,20 et 3,73 (2m, 4H, $CH_2$-$CH_2$-N-$CH_2$-$CH_2$) ; 3,51 (m, 2H, $CH_2$-$CH_2$-$CH_2$-NH) ; 4,23 (m, 2H, NH-$CH_2$-CH-NH) ; 4,67 (m, 1H, NH-$CH_2$-CH-NH); 6,38 et 7,35(2d, 2H, H naphthyridine); 7,20, 7,35, 7,47 et 7,54 (t, t masqué, dl et dl 4H, Hbenzothiazol); 7,73 et 10,05 (2m, 2H, Hmobiles); 8,36 ppm (s, 1H, N=CH-N).
**SM :** 559 (MH+); 557- (M-H-)

## Exemple 35

En opérant par analogie avec l'exemple 34, à partir de 3-[5-éthyl-6-[4-(1,2,3,4-tétrahydro-1,8-naphthyridin-7-yl)-1-pipéridinyl]-4-pyrimidinyl]amino] alaninate de (1,1-diméthyléthyle), on prépare la 3-[[5-éthyl-6-[4-(1,2,3,4-tétrahydro-1,8-naphthyridin-7-yl)-1-pipéridinyl]-4-pyrimidinyl]amino]-N-[2-(4-methoxybenzymidazolyl)]alanine sous forme d'un solide beige.
**CCM :** Rf = 0,40 (silicagel, éluant : dichlorométhane-méthanol-eau-acide acétique 85-15-2-2)
**1H-RMN (CDCl3) :** δ 1, 12 (m, 3H, $CH_2CH_3$) ; 1,65 à 2,07 (m, 6H, $CH_2$-CH-$CH_2$ et_$CH_2$-$CH_2$-$CH_2$-NH); 2,47 (m, 2H, $CH_2$-$CH_3$) ; 2,74 (m, 2H, $CH_2$-$CH_2$-$CH_2$-NH) ; 2,92 (m, 1H, $CH_2$-CH-$CH_2$) ; 3,17 et 3,73 (2m, 4H, $CH_2$-$CH_2$-N-$CH_2$-$CH_2$) ; 3,52 (m, 2H, $CH_2$-$CH_2$-$CH_2$-NH); 3,79 (s, 1H, OCH3); 4,18 (m, 2H, NH-$CH_2$-CH-NH); 4,81 (m, 1H, NH-$CH_2$-CH-NH); 6,35 et 7,36c (2d, 2H, H naphthyridine); 6,72 et 7,18 (2d, 2H, NC(C)CHCHC(CH)OCH3) ; 6,87 (s, 1H, NC(C)CHC(CH) OCH3) ; 6,37, 7,70 et 9,84 (3Hmobiles); 8,32 ppm (s, 1H, N=CH-N).

**SM :** 572 (MH+); 570- (M-H-)

**Exemple de référence 36**

Synthèse du 2-(2-benzyloxycarbonylamino-3-{5-methyl-6-[4-(5,6,7,8-tétrahydro-[1,8] naphtyridin-2-yl)-pipéridin-1-yl]-pyrimidin-4-ylamino}-propinylamino)-propionate de terbutyle.

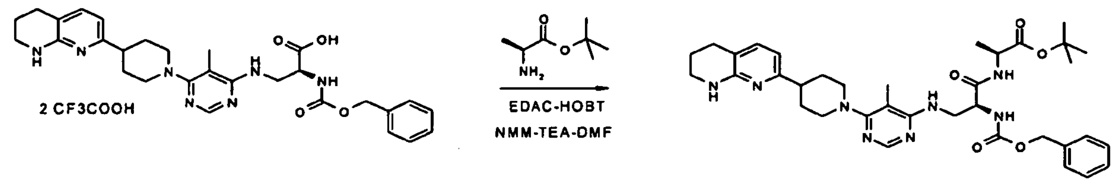


On agite 170 mg (0,30 mmole) de 3-[[5-méthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, 150 mg (1,0 mmole) de L-alaninate de terbutyle, 66 mg (0,48 mmole) de 1-hydoxy benzotriazole, 90 mg (0,48 mmole) de chlorhydrate de 1-[3-(diméthylamino) propyl]-3-ethyl carbodiimide, 0,015 ml (0,135 mmole) de N-methhylmorpholine et de 0,210 ml (1,50 mmoles) de triéthylamine dans 10 ml de diméthylformamide pendant 24 heures à température ambiante. Le mélange réactionnel est évaporé à sec sous pression réduite (2 kPa) et le résidu est repris par de l'acétate d'éthyle et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un mélange d'acétate d'éthyle-chlorure de méthylène-méthanol de 50-50-10. On obtient 120 mg (Rdt=60 %) de produit attendu sous forme d'un solide blanc.
**CCM :** Rf=0,40 (silicagel, éluant : dichlorométhane-méthanol-eau-acide acétique 90-10-1-1)
**SM :** 673 (MH+).

Synthèse de l'acide 2-(2-benzyloxycarbonylamino-3-{5-methyl-6-[4-(5,6,7,8-tétrahydro-[1,8] naphtyridin-2-yl)-pipéridin-1-yl]-pyrimidin-4-ylamino}-propinylamino)-propionique.

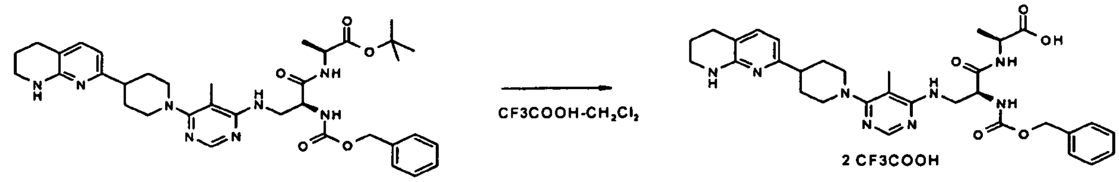


On agite 120 mg (0,179 mmoles) de 2-(2-benzyloxycarbonylamino-3-{5-methyl-6-[4-(5,6,7,8-tétrahydro-[1,8] naphtyridin-2-yl)-pipéridin-1-yl]-pyrimidin-4-ylamino}-propinylamino)-propionate de terbutyle dans 5 ml de dichlorométhane avec 1 ml d'acide trifluoroacétique à température ambiante jusqu'à disparition du produit de départ en CCM (silicagel, éluant : CH2Cl2-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 95 mg (Rdt = 63 % exprimé en ditrifluoroacétate) de produit attendu sous forme d'un solide blanc.
**CCM :** Rf = 0,45 (silicagel, éluant : dichlorométhane-méthanol-eau-acide acétique 85-15-2-2)
**SM :** 616 (MH+).

**Exemple de référence 37**

Synthèse du 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-propionate de terbutyle.

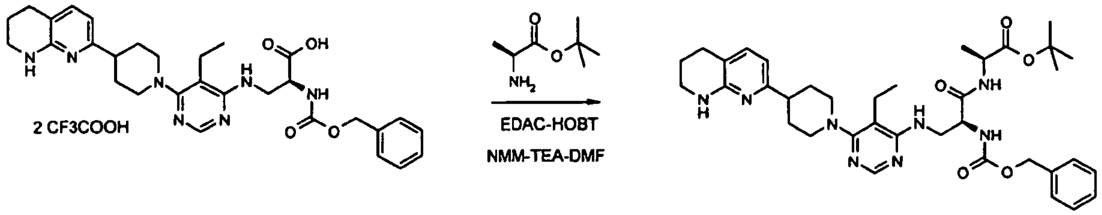


On agite 80 mg (0,15 mmole) de 3-[[5-éthyl-6-[4-(1,2,3,4-tétrahydro-1,8-naphthyridin-7-yl)-1-pipéridinyl]-4-pyrimidinyl]amino]-N-[(phénylméthoxy)carbonyl]alanine, 32 mg (0,20 mmole) de L-alaninate de terbutyle, 22 mg (0,16 mmole) de 1-hydoxy benzotriazole, 30 mg (0,16 mmole) de chlorhydrate de 1-[3-(diméthylamino) propyl]-3-ethyl carbodiimide, 0,050 ml (0,45 mmole) de N-methhylmorpholine et de 0,070 ml (0,50 mmole) de triéthylamine dans 5 ml de diméthylformamide pendant 24 heures à température ambiante. Le mélange réactionnel est évaporé à sec sous pression réduite (2 kPa) et le résidu est repris par de l'acétate d'éthyle et une solution saturée de bicarbonate de sodium. La phase organique est séparée, séchée sur sulfate de magnésium et le solvant évaporé sous pression réduite (2 kPa). Le résidu est chromatographié sur silicagel en éluant avec un gradient d'acétate d'éthyle-chlorure de méthylène-méthanol de 50-50-0 à 50-45-5. On obtient 55 mg (Rdt = 53 %) de produit attendu sous forme d'un solide blanc.
**CCM :** Rf = 0,30 (silicagel, éluant : dichlorométhane-méthanol-eau-acide acétique 90-10-1-1)
**1H-RMN (CDCl3) :** δ 0,91 (t, 3H, CH2-CH3); 1,38 (d, 3H, CONH-CH(CH3)-CO); 1,48 (s, 9H, tBu); 1, 79 à 2,07 (m, 6H, $CH_2$-$CH_2$-$CH_2$-NH, $CH_2$-CH-$CH_2$) ; 2,49 (m, 2H, $CH_2$-$CH_3$); 2,66 (m, 1H, $CH_2$-CH-$CH_2$) ; 2,73 (t, 2H, $CH_2$-$CH_2$-$CH_2$-NH); 2,97 et 3,63 (2m, 4H, $CH_2$-$CH_2$-N-$CH_2$-$CH_2$); 3,42 (m, 2H, $CH_2$-$CH_2$-$CH_2$-NH); 3,73 et 4,14 (2m, 2H, NH-$CH_2$-CH-NH) ; 4, 31 (m, 1H, NH-$CH_2$-CH-NH); 4,44 (q, 1H, CONH-CH(CH3)-CO); 5,14 (m, 2H, $CH_2$-Ph); 5,35, 6,92 et 7,89 (3H, Hmobiles); 6,42 et 7,14 (2d, 2H, H naphthyridine); 7,36 (m, 5H, Ph); 8,29 ppm (s, 1H, N=CH-N) .
**SM :** 687 (MH+).

Synthèse de l'acide 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-propionique.

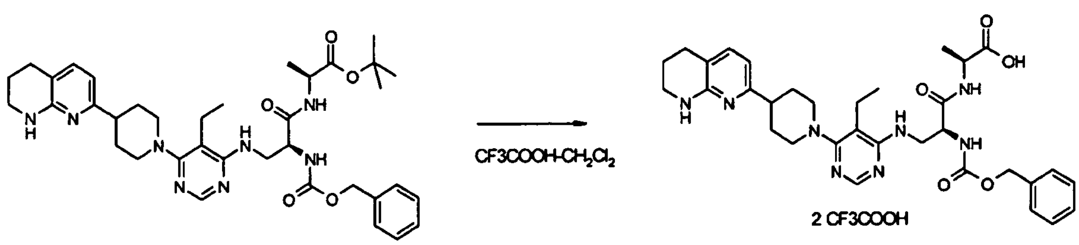


On agite 50 mg (0,073 mmoles) de 2-(2-benzyloxycarbonylamino-3-{5-ethyl-6-[4-(5,6,7,8-tétrahydro-[1,8] naphtyridin-2-yl)-pipéridin-1-yl]-pyrimidin-4-ylamino}-propinylamino)-propionate de terbutyle dans 5 ml de dichlorométhane avec 0,5 ml d'acide trifluoroacétique à température ambiante jusqu'à disparition du produit de départ en CCM (silicagel, éluant : CH2C12-MeOH-H2O-AcOH 90-10-1-1). On rajoute du toluène et évapore le mélange réactionnel à sec sous pression réduite (2 kPa). Le résidu est solubilisé dans le minimum de dichlorométhane avec un peu de méthanol puis versé sur de l'éther diisopropylique. Le précipité est filtré. On obtient 53 mg (Rdt = 85 % exprimé en ditrifluoroacétate) de produit attendu sous forme d'un solide blanc.
**CCM :** Rf = 0,12 (silicagel, éluant : dichlorométhane-méthanol-eau-acide acétique 90-10-1-1)
**1H-RMN (CDCl3) :** δ 1,16 (t, 3H, CH2-CH3); 1,41 (d, 3H, CONH-CH(CH3)-CO); 1,80 à 2,10 (m, 6H, $CH_2$-$CH_2$-$CH_2$-NH, $CH_2$-CH-$CH_2$); 2,48 (m, 2H, $CH_2$-$CH_3$); 2,79 (m, 2H, $CH_2$-$CH_2$-$CH_2$-NH); 3,00 (tl, 1H, $CH_2$-CH-$CH_2$); 3,28 et 3,84 (2m, 4H, $CH_2$-$CH_2$-N-$CH_2$-$CH_2$); 3,52 (m, 2H, $CH_2$-$CH_2$-$CH_2$-NH); 3,78 et 4,13 (2m, 2H, NH-$CH_2$-CH-NH); 4,46 (m, 1H, NH-$CH_2$-CH-NH); 4,59 (m, 1H, CONH-CH(CH3)-CO): 5,05 (m, 2H, $CH_2$-Ph); 6,41 et 7,38 (2d, 2H, H naphthyridine); 6,68, 7,20, 7,95 et 9,68 (4H, Hmobiles); 7,31 (m, 5H, Ph); 8,28 ppm (s, 1H, N=CH-N).
**SM :** 631 (MH+).

**Exemple 41**

Synthèse de la 2,5-diméthyl-4,6-dihydroxy-pyrimidine :

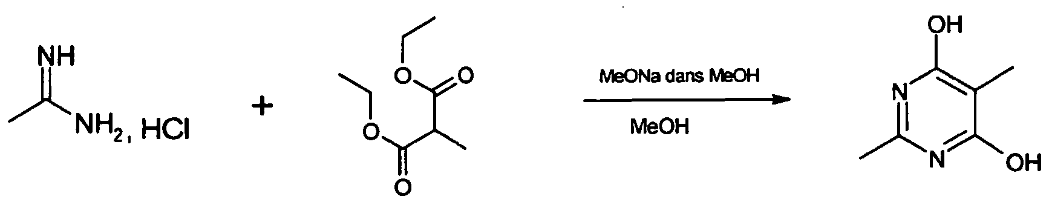


Un monocol contenant 40 ml de méthanol, placé sous atmosphère d'azote, est refroidit à 0°C par un bain de glace, on additionne 9,72 g de méthylate de sodium (soit une solution de concentration c=3 $mol.1^{-1}$) au mélange réactionnel puis on ajoute à 0°C et par petites quantités 5 g (53 mmoles)de chlorhydrate de d'acétamidine. On laisse agiter à température ambiante pendant une vingtaine de minutes, puis on ajoute goutte à goutte 8,3 ml de méthylmalonate de diéthyle. On maintient l'agitation pendant 3 heures. Puis le méthanol est condensée sous pression réduite (2 kPa). Le brut obtenu est repris avec un minimum d'eau, refroidit à 0°C puis acidifié avec de l'acide acétique pur jusqu'à un pH entre 4 et 5. Le précipité blanc formé est filtré, rincé à l'eau, éther éthylique et pentane. Puis le produit blanc est séché sur $P_2O_5$ sous pression réduite (0,2 kPa). On obtient 3,3 g (Rdt = 49 %) de produit attendu.
**CCM :** Rf = 0,2 (Silicagel, éluant : dichlorométhane-méthanol-eau-acide acétique 85-15-2-2).
**1H-RMN (DMSOd6) :** δ 1,68 (s, 3H, OH-CH=C-CH3); 2,18 (s, 3H, N=C-CH3). Synthèse de la 2,5-diméthyl-4,6-dichloro pyrimidine :

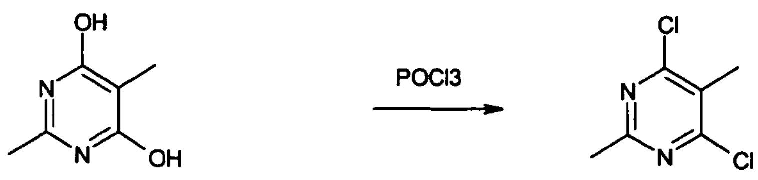


Un mélange de 3,3 g (23,5 mmoles) de 2,5-diméthyl-4,6-dihydroxy-pyrimidine et 15 ml d'oxychlorure de phosphore, est porté au reflux pendant 8 heures. Après retour à température ambiante, le milieu réactionnel est versé lentement sur un mélange de glace et d'eau. Cette phase aqueuse est extraite à l'acétate d'éthyle. La phase organique est lavée avec une solution saturée de bicarbonate de sodium puis séchée sur du sulfate de magnésium et évaporée à sec sous pression réduite (2 kPa). On obtient 3,39 g (Rdt = 81 %) de produit attendu.
**CCM :** Rf = 0,9 (Silicagel, éluant : acétate d'éthyle 100 %)
**1H-RMN (CDCl3) :** δ 2,46 (s, 3H, Cl-CH=C-CH3); 2,68 (s, 3H, N=C-CH3) **SM** : 177/179 (MH+).

Synthèse de la 6-[4-(1,2,3,4-tetrahydro-1,8-naphthyridin-7-yl)-1-pipéridinyl]-2,5-diméthyl-4-chloro-pyrimidine :

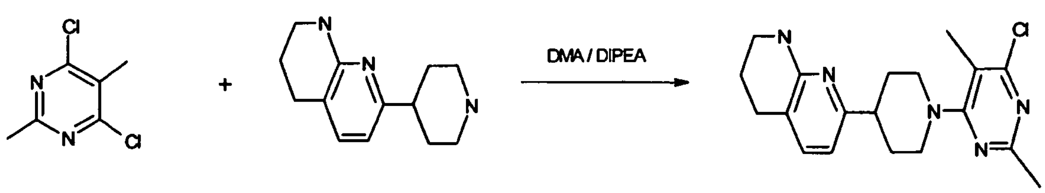


Dans un monocol contenant 2,95 g (13,5 mmoles) de 4-(1,2,3,4-tetrahydro-1,8-naphthyridin-7-yl)-1-pipéridine libérée de son sel, on ajoute 2 g (11,3 mmoles) de 4,6-dichloro-2,5-diméthyl-pyrimidine mis en solution dans 25 ml de

diméthylacétamide et 5 ml de diisopropyléthylamine. Ce mélange est chauffé à 130°C pendant 4 heures puis concentré à sec sous vide. Le résidu obtenu est repris par un mélange d'eau, d'acétate d'éthyle et d'une solution de bicarbonate de sodium saturée. La phase organique est séparée et la phase acqueuse réextraite par de l'acétate d'éthyle. Les phases organiques regroupées sont séchées sur sulfate de magnésium puis le solvant évaporé sous pression réduite (2 kPa). Le résidu est chromatographié sur silicagel en éluant avec un gradient d'acétate d'éthyle (100 %) puis d'acétate d'éthyle-méthanol (95-5). On obtient 2,2 g (Rdt = 55 %) de produit attendu.

Préparation de la naphtyridine sous forme d'amine libre :

8,3 g de naphtyridine est déplacée de son sel par 6 équivalents massiques de résine basique amberlyst A21 (résine de type $R-NMe_2$ ) dans un mélange $CH_2Cl_2$ / MeOH /AcOEt 1/1/1 sous agitation pendant 30 minutes. On lave préalablement la résine et on la laisse gonfler 20 minutes dans ce mélange de solvant. Cette opération doit être répetée 3 fois pour que le déplacement du sel soit total.

Après filtration de la résine et évaporation des solvants, on obtient 2,95 g (13,5 mmoles) de naphtyridine libre.
**CCM :** Rf = 0,15 [silicagel, éluant : dichlorométhane-méthanol 95-5]
**1H-RMN (CDCl3) :** δ 1,90 et 2,01 (2m, 6H, NH-CH2-CH2-CH2, N-CH2-CH2-CH-CH2-CH2): 2,26 (s, 3H, CH3); 2,51 (s, 3H, N=C-CH3); 2,72 (m, 3H, NH-CH2-CH2-CH2, N-CH2-CH2-CH-CH2-CH2); 2,97 et 3,97 (2m, 4H, CH2-CH2-N-CH2-CH2); 3,42 (m, 2H, NH-CH2-CH2-CH2); 6,41 et 7,16 (2d, 2H, CH=CH naphthyridine).
**SM :** 358(MH+).

Synthèse du 3-[[6-[4-(1,2,3,4-tetrahydro-1,8-naphthyridin-7-yl)-1-pipéridinyl]-2,5-diméthyl-4-pyrimidinyl]amino]-N-[(phénylméthoxy)carbonyl]alaninate de (1,1-diméthyléthyle).

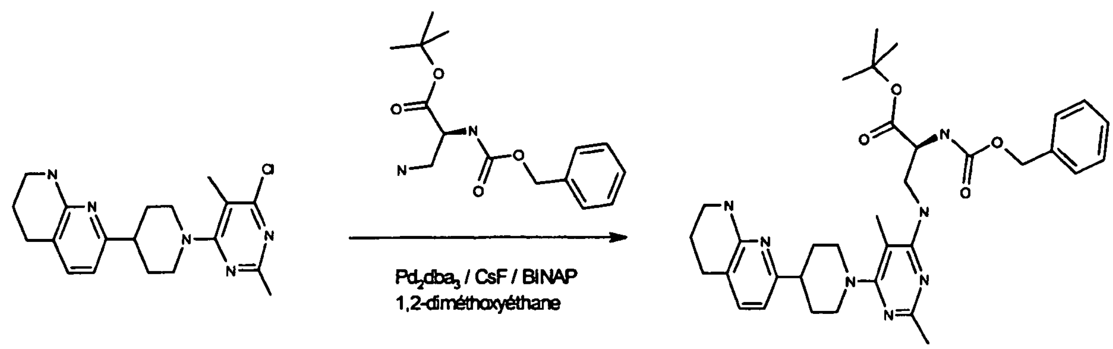


Un mélange de 2,2 g (6,15 mmoles) de 6-[4-(1,2,3,4-tetrahydro-1,8-naphthyridin-7-yl)-1-pipéridinyl]-2,5-diméthyl-4-chloro-pyrimidine et de 2,17 g (7,38.mmoles) de 3-amino-N-[(phénylméthoxy)carbonyl] alaninate de (1,1-diméthyléthyle) (préparé selon J. Med. Chem. (2001), 44(8), 1158-1176), en présence de 1,31 g (8,61 mmoles) de fluorure de cesium, de 383 mg (0,615 mmoles) de (2,2'-bis(diphénylphosphino)-1,1'-binaphthyl et de 281 mg (0,307 mmoles) de tris-dibenzylidèneacétone)dipalladium(o), dans 55 ml de 1,2-diméthoxyéthane est chauffé au reflux pendant 24 heures. Le mélange réactionnel est ensuite ramené à température ambiante, on ajoute alors 281 mg (0,307 mmoles) de tris-dibenzylidèneacétone)dipalladium(o), puis le mélange réactionnel est porté au reflux pendant encore 24 heures. Après refroidissement la solution est concentrée à sec sous pression réduite (2 kPa) puis reprise par un mélange d'eau, d'acétate d'éthyle et d'une solution saturée de bicarbonate de sodium. La phase organique est décantée et la phase aqueuse est extraite à l'acétate d'éthyle. Les phases organiques regroupées sont séchées sur du sulfate de magnésium et évaporées à sec sous pression réduite (2 kPa). Le résidu est chromatographié sur alumine avec un gradient d'éther isopropylique/acétate d'éthyle (50/50)-dichlorométhane (50-50). Les fractions contenant le produit attendu sont rassemblées pour une seconde purification sur silicagel avec un gradient acétate d'éthyle-heptane-méthanol 50-50-0 à 90-0-10. On obtient 550 mg (Rdt = 15 %) de produit attendu.
**CCM :** Rf = 0.3 (silicagel, éluant : dichlorométhane-méthanol 90-10)
**1H-RMN (CDCl3) :** δ 1,46 (s, 9H, tBu); 1,92 (m, 9H, NH-CH2-CH2-CH2, N-CH2-CH2-CH-CH2-CH2, C=C-CH3); 2.91 (s, 3H, N=C-CH3); 2.70 (m, 3H, NH-CH2-CH2-CH2, N-CH2-CH2-CH-CH2-CH2); 2.91 et 3.66 (2m, 4H, CH2-CH2-N-CH2-CH2); 3.44 (m, 2H, NH-CH2-CH2-CH2); 3,90 (m, 2H, NH-CH2-CH-NH); 4.38 (m, 1H, NH-CH2-CH-NH); 5,13 (s, 2H, O-CH2-Ph); 6.42 et 7,16 (2d, 2H, CH=CH naphthyridine); 7,35 (m, 5H, Ph).
**SM :** 616(MH+)

Synthèse de l'acide correspondant :

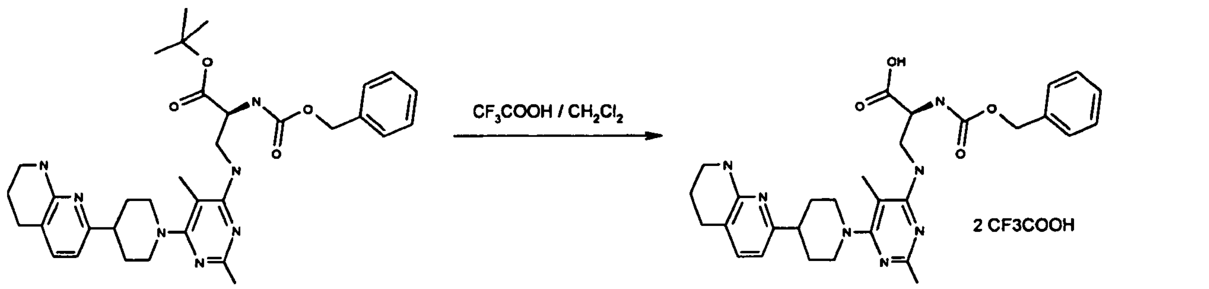


On agite à température ambiante un mélange de 500 mg (0,81 mmoles) de la 3-[[6-[4-(1,2,3,4-tetrahydro-1,8-naphthyridin-7-yl)-1-pipéridinyl]-2,5-diméthyl-4-pyrimidinyl]amino]-N-[(phénylméthoxy)carbonyl]alaninate de (1,1-diméthyléthyle) et de 5 ml d'acide trifluoroacétique dans 30 ml de dichlorométhane pendant 24 heures. On additionne alors du toluène et évapore à sec le mélange. Le résidu est solubilisé dans le minimum de dichlorométhane avec un peu de méthanol puis versé sur de l'éther diisopropylique. Le précipité est filtré. On obtient 485 mg (Rdt = 76 % exprimé en ditrifluoroacétate) de produit attendu.

**CCM :** Rf = 0,3 (silicagel, éluant : dichlorométhane-méthanol-eau-acide acétique 90-10-1-1).

**1H-RMN (CDCl3) :** δ 1,97 (m, 9H, NH-CH2-CH2-CH2, N-CH2-CH2-CH-CH2-CH2, C=C-CH3); 2,54 (s, 3H, N=C-CH3); 2,78 (m, 2H, NH-CH2-CH2-CH2); 2,98 (m, 1H, N-CH2-CH2-CH-CH2-CH2); 3,22 et 3.80 à 4,07 (2m, 6H, N-CH2-CH2-CH-CH2-CH2, NH-CH2-CH-NH); 3,51 (m, 2H, NH-CH2-CH2-CH2); 4,45 (m, 1H, NH-CH2-CH-NH); 5,10 (s, 2H, O-CH2-Ph); 6,92 et 7,37 (2m, 3H, CH=CH naphthyridine, NH mobile); 7,32 (m, 5H, Ph).

**SM :** 560 (MH+); 426 [MH+ -(COOCH2Ph)]+.

**Test pharmacologique** de référence: **Test ELISA Kistrine/Récepteur Vitronectine ($\alpha_v\beta_3$)**

Protocole :

Des plaques 96 puits MaxiSorp sont coatées une nuit à 40°C avec 100 μl de Kistrine à 1 μg/ml (dilution en tampon de coating : carbonate 0,05 M/NaOH pH 9,6). Le lendemain, les puits sont vidés et les ligands (kistrine) sont ensuite fixés (tampons de fixation : PBS contenant 0,5 % de BSA (pH = 7,4)) pendant 1 heure à température ambiante avec une agitation douce de 125 rpm. Les puits sont lavés six fois (tampon de lavage : PBS contenant 0,05 % de Tween 20 (pH 7,7) puis on ajoute par puits et dans cet ordre :

- 40 μl de tampon d'incubation
- 10 μl de la dilution du produit à tester (les produits sont dilués dans un mélange 50:50 DMSO/Eau)
- 50 μl de récepteur $\alpha_v\beta_3$ humain (cf Pytella et al. Methods Enzymol. (1987) 144 (Dilution en tampon d'incubation, à adapter suivant le lot de récepteur et selon le ligand). Le ligand, le récepteur $\alpha_v\beta_3$ et les produits à étudier sont co-incubés pendant 3 heures à température ambiante avec une agitation douce de 125 rpm.

Les puits sont à nouveau lavés six fois, puis incubés pendant 2 heures à température ambiante avec une agitation douce de 125 rpm, en présence de 100 μl d'anticorps anti-récepteur couplé à une peroxydase (L'anticorps 4B12-HRP est dilué en tampon d'incubation (50 mM TRIS pH 7,4; 0,5 % BSA; 0,05 % Tween 20; 1 mM $MnCl_2$; 50 μM $CaCl_2$; 50 μM $MgCl_2$; 100 mM NaCl) . La dilution est à adapter suivant le lot de récepteur.

Les puits sont ensuite lavés six fois avant la mesure de la liaison ligand-récepteur faite par l'intermédiaire d'un kit révélateur de peroxydase (TBM Microwell Peroxidase Substrate System Kirkegaard; Ref cat 50-76-00).

Ce kit contient un flacon A de substrat (3,3',5,5'-tétraméthylebenzidine à 0,4 g/l) et un flacon B ($H_2O_2$ à 0,02 % en tampon Citrate/Acide citrique). Extemporanément, un volume de A est mélangé à un volume de B, puis le mélange réactionnel est distribué à raison de 100 μl/puits.

La réaction enzymatique se développe entre 6 à 10 minutes pour Kistrine/$\alpha_v\beta_3$ puis son évolution est stoppée par l'addition de 100 μl d'acide phosphorique 1M. La densité optique est déterminée à 450 nm.

## Expression des résultats de référence

On trace la courbe suivante : le pourcentage de liaison en fonction du logarithme de chaque concentration du produit testé.
Pour chaque produit, on détermine l'IC50 suivant la formule suivante :

$$IC50 = (B0+ Bmin)/2$$

B0 = Maximum de liaison en l'absence de tout produit
Bmin = Minimum de liaison en présence de la concentration la plus élevée de produit.

| EXEMPLE | K/VnR $IC_{50}$ (nM) |
|---|---|
| 1 | 3 |
| 3 | 3,1 |

## Activité in vivo

### Hypercalcémie induite par l'hormone parathyroïdienne (PTH) dans un modèle de rat thyroparathyroïdectomisés (TPXT)

La stimulation de la résorption osseuse est induite chez des rats TPXT par perfusion de PTH et les variations de la résorption osseuse sont suivies par la concentration en calcium dans le sérum.
Des rats mâles Sprague Dawley pesant 150-200 g sont thyroparathyroïdectomisés. Les rats sont soumis à un régime standard contenant 7 g Ca/kg (UAR) et de l'eau de Volvic. L'efficacité de la thyroparathyroïdectomie est testée en mesurant les concentrations en Ca dans le sérum 8 jours après l'opération chez des animaux à jeun depuis la veille. Les rats sont considérés comme thyroparathyroïdectomisés lorsque les taux de Ca dans le sérum sont inférieurs à 80 mg/l. La PTH (1-34)de rat (Bachem) est dissoute dans 0,15M de NaCl Cys.HCl 2 % et délivré par des minipompes osmotiques (ALZET 2001D) à la dose de 200 pmol/kg/h. Les minipompes sont introduites dans les cavités intrapéritonéales sous anesthésie (kétamine - 75 mg/kg et acépromazine - 2,5 mg/kg) chez des rats TPXT à jeun depuis la veille. Les rats TPXT contrôles reçoivent les minipompes remplies avec le véhicule de la PTH.
Soit le produit à tester soit le véhicule (contrôles et rats traités par la PTH) sont administrés 2 fois par voie sous-cutanée (2 ml/kg de poids corporel) aux temps 0 et 3 h après le début de l'infusion de PTH. Le test est poursuivi pendant les 6 heures suivantes. A la fin du traitement, la totalité du sang est collectée après décapitation. Les échantillons de sang sont centrifugés à 3000 tpm pendant 15 mn (CR422 Jouan) pour obtenir le sérum.
Les concentrations totales de Ca dans le sérum sont mesurées par colorimétrie (Ciba-Corning) en utilisant un système de lecture de microplaques IEMS Labsystems, à 540 nm.
La différence entre les valeurs moyennes de calcémie des rats traités et des groupes contrôles est analysée en variance et par le test de Dunnett.
L'activité d'un produit est calculée par la formule suivante :

$$\text{effet \%} = \frac{\text{Calcémie (produit) - calcémie (PTH)}}{\text{Calcémie (PTH) - calcémie (contrôle)}} \times 100$$

Les produits des exemples 6, 9, 13 et 15 à 19 testés dans la méthode décrite ci-avant se sont montrés actifs à des doses allant de 2 fois 1 mg/kg à 2 fois 10 mg/kg par voie sous cutanée chez le rat.

## Revendications

**1.** Un composé de formule (I) :

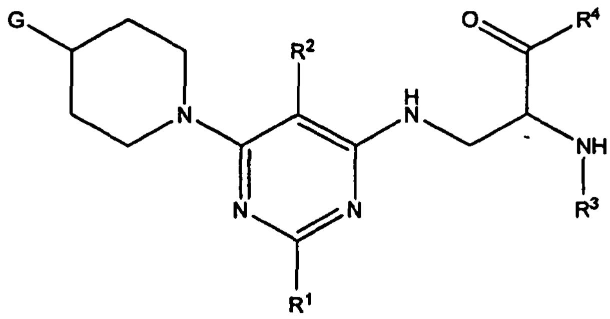

sous toutes ses formes isomères, seules ou en mélange, ainsi que ses sels d'addition physiologiquement acceptables et ainsi que ses solvates, dans laquelle:

- G représente 1,2,3,4-tétrahydro-1,8-naphtyridin-7-yle,
- $R^1$ représente méthyle,
- $R^2$ représente méthyle,
- $R^3$ représente benzyloxycarbonyle et
- $R^4$ représente OH ou t.butoxy.

**2.** Un composé selon la revendication 1, ayant la formule:

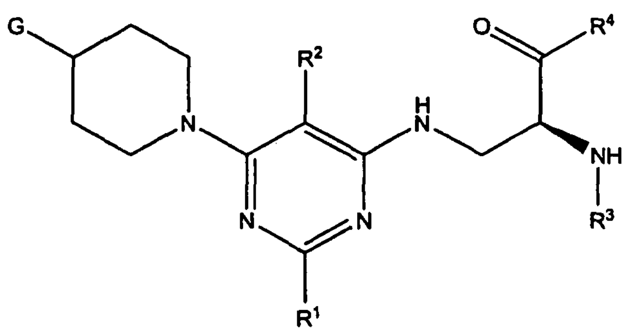

**3.** Un composé de formule (I) selon la revendication 1 ou 2 dans laquelle $R^4$ représente OH.

**4.** Un composé de formule (I) selon la revendication 1 ou 2 dans laquelle $R^4$ représente *t*.butoxy.

**5.** A titre de médicament, un composé de formule (I) et/ou ses sels physiologiquement acceptables tel que défini selon l'une quelconque des revendications 1 à 4.

**6.** Une composition pharmaceutique comprenant un médicament tel que défini à la revendication 5 ainsi qu'un ou plusieurs excipients.

**7.** A titre de médicament, ayant une activité antagoniste du récepteur de la vitronectine, un composé de formule (I) et/ou ses sels physiologiquement acceptables tel que défini selon l'une quelconque des revendications 1 à 4.

**8.** A titre de médicament, ayant une activité inhibitrice de la résorption osseuse ou pour le traitement ou la prévention de l'ostéoporose, un composé de formule (I) et/ou ses sels physiologiquement acceptables tel que défini selon l'une quelconque des revendications 1 à 4.

**9.** A titre de médicament, ayant une activité inhibitrice de la croissance tumorale ou des métastases cancéreuses, un composé de formule (I) et/ou ses sels physiologiquement acceptables tel que défini selon l'une quelconque des

revendications 1 à 4.

**10.** A titre de médicament, ayant une activité anti-inflammatoire ou pour le traitement ou la prévention des désordres cardiovasculaires, la resténose, l'artériosclérose, les néphropathies ou rétinopathies, un composé de formule (I) et/ou ses sels physiologiquement acceptables et/ou ses prodrugs tel que défini selon l'une quelconque des revendications 1 à 4.

**11.** Utilisation d'un composé de formule (I) et/ou ses sels physiologiquement acceptables tel que défini selon l'une quelconque des revendications 1 à 4 pour la préparation de médicaments destinés à la prévention ou au traitement de l'ostéoporose.

**12.** Utilisation d'un composé de formule (I) et/ou ses sels physiologiquement acceptables tel que défini selon l'une quelconque des revendications 1 à 4 pour la préparation de médicaments destinés à inhiber la croissance tumorale ou les métastases cancéreuses.

**13.** Utilisation d'un composé de formule (I) et/ou ses sels physiologiquement acceptables tel que défini selon l'une quelconque des revendications 1 à 4 pour la préparation de médicaments destinés à la prévention ou au traitement des désordres cardiovasculaires, de la resténose, de l'artériosclérose, des néphropathies ou des rétinopathies.

**14.** Un procédé de préparation des composés de formule (I) selon l'une des revendications 1 à 4, dans lequel:

a) on fait réagir un produit de formule générale (IIa):

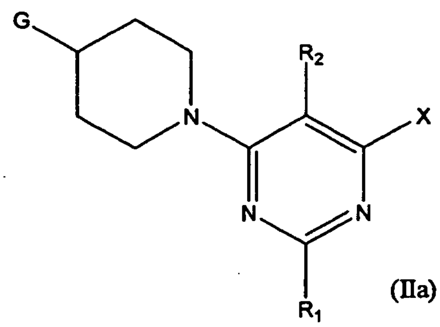

(IIa)

dans laquelle $R^1$, $R^2$ et G sont tels que définis précédemment, et X répresente un atome d'halogène, avec un produit de formule (VI)

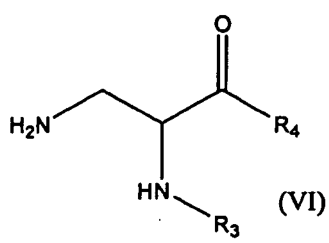

(VI)

dans laquelle $R^3$ et $R^4$ sont définis comme précédemment, soit en présence d'une base forte, soit par catalyse au palladium, pour obtenir un produit de formule (I)

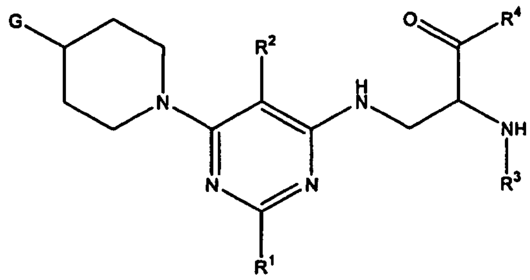

b) puis le produit de formule (I) est soumis éventuellement à l'hydrolyse et/ou à la salification.

**15.** Un procédé selon la revendication 14 dans lequel X répresente un atome de chlore.

## Claims

**1.** Compound of formula (I)

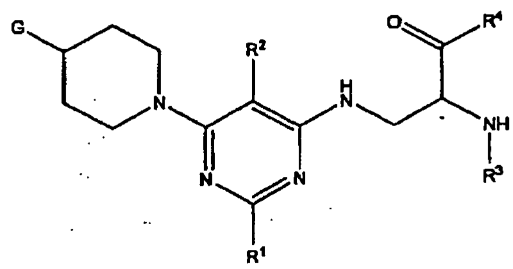

in all its isomeric forms, alone or in mixture, as well as its physiologically acceptable addition salts, and its solvates, wherein:

- G represents 1,2,3,4-tetrahydro-1,8-naphthyridin-7-yl,
- $R^1$ represents methyl,
- $R^2$ represents methyl,
- $R^3$ represents benzyloxycarbonyl and
- $R^4$ represents OH or t-butoxy.

**2.** Compound according to claim 1 having the formula:

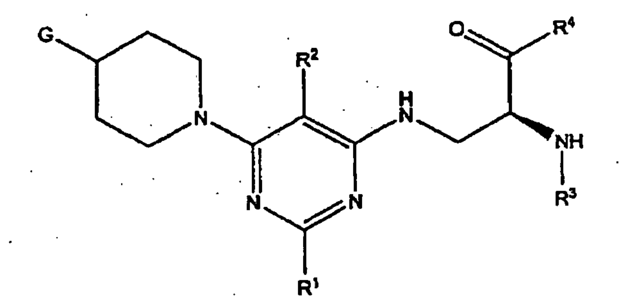

**3.** Compound of formula (I) according to either claim 1 or claim 2, wherein $R^4$ represents OH.

**4.** Compound of formula (I) according to either claim 1 or claim 2, wherein $R^4$ represents t-butoxy.

**5.** As a medicament, a compound of formula (I) and/or its physiologically acceptable salts as defined according to any one of claims 1 to 4.

**6.** Pharmaceutical composition comprising a medicament as defined in claim 5 as well as one or more excipients.

**7.** As a medicament, having an antagonist activity on the vitronectin receptor, a compound of formula (I) and/or its physiologically acceptable salts as defined according to any one of claims 1 to 4.

**8.** As a medicament, having an inhibitory activity on bone resorption or for the treatment or prevention of osteoporosis, a compound of formula (I) and/or its physiologically acceptable salts as defined according to any one of claims 1 to 4.

**9.** As a medicament, having an inhibitory activity on tumorous growth or cancerous metastases, a compound of formula (I) and/or its physiologically acceptable salts as defined according to any one of claims 1 to 4.

**10.** As a medicament, having an anti-inflammatory activity or for the treatment or prevention of cardiovascular disorders, restenosis, arteriosclerosis, nephropathies or retinopathies, a compound of formula (I) and/or its physiologically acceptable salts and/or its prodrugs as defined according to any one of claims 1 to 4.

**11.** Use of a compound of formula (I) and/or its physiologically acceptable salts as defined according to any one of claims 1 to 4 for the preparation of medicaments intended for the prevention or treatment of osteoporosis.

**12.** Use of a compound of formula (I) and/or its physiologically acceptable salts as defined according to any one of claims 1 to 4 for the preparation of medicaments intended to inhibit tumorous growth or cancerous metastases.

**13.** Use of a compound of formula (I) and/or its physiologically acceptable salts as defined according to any one of claims 1 to 4 for the preparation of medicaments for the prevention or treatment of cardiovascular disorders, restenosis, arteriosclerosis, nephropathies or retinopathies.

**14.** Process for the preparation of the compounds of formula (I) according to one of claims 1 to 4, wherein:

a) a product of general formula (IIa)

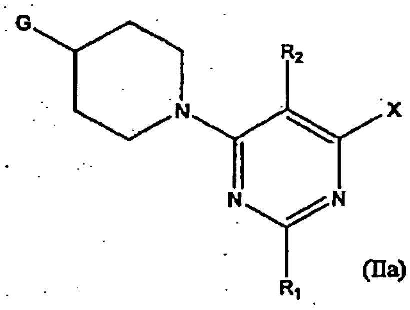

(IIa)

wherein $R^1$, $R^2$ and G are as defined previously, and X represents a halogen atom, is reacted with a product of formula (VI)

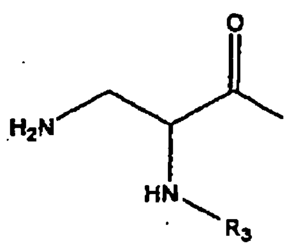

(VI)

wherein $R^3$ and $R^4$ are as defined previously, either in the presence of a strong base, or by catalysis with

palladium, to obtain a product of formula (I)

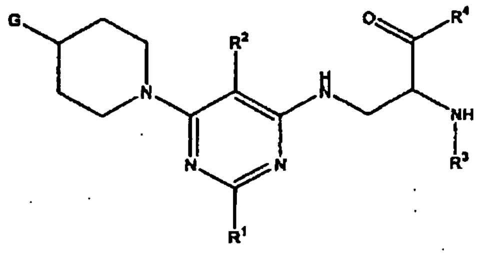

b) then the product of formula (I) is optionally subjected to hydrolysis and/or to salification.

**15.** Process according to claim 14, wherein X represents a chlorine atom.

**Patentansprüche**

**1.** Verbindung mit der Formel (I)

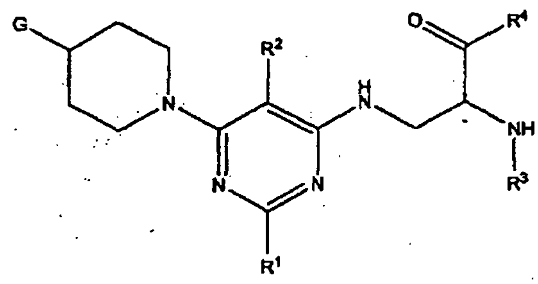

in all ihren isomeren Formen, allein oder im Gemisch, sowie ihre physiologisch akzeptierbaren Additionssalze und ihre Solvate, wobei

- G 1,2,3,4-Tetrahydro-1,8-Naphtyridin-7-yl darstellt,
- $R^1$ Methyl darstellt,
- $R^2$ Methyl darstellt,
- $R^3$ Benyloxycarbonyl darstellt, und
- $R^4$ OH oder t.butoxy darstellt.

**2.** Verbindung nach Anspruch 1 mit der Formel:

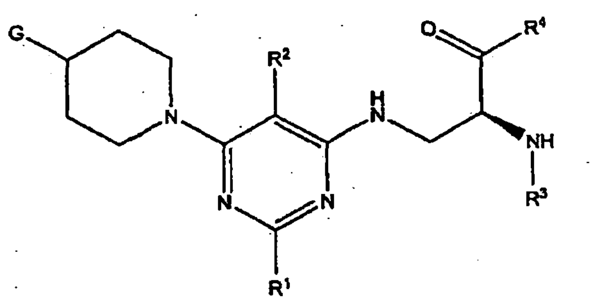

**3.** Verbindung mit der Formel (I) nach Anspruch 1 oder 2, wobei $R^4$ OH darstellt.

**4.** Verbindung mit der Formel (1) nach Anspruch 1 oder 2, wobei $R^4$ t.butoxy darstellt.

**5.** Als Medikament, Verbindung mit der Formel (I) und/oder ihre physiologisch akzeptierbaren Salze nach einem der Ansprüche 1 bis 4.

**6.** Pharmazeutische Zusammensetzung, die ein Medikament nach Anspruch 5 sowie ein oder mehrere Trägerstoffe aufweist.

**7.** Als Medikament, mit antagonistischer Wirkung auf den Vitronectin-Rezeptor, Verbindung mit der Formel (I) und/oder ihre physiologisch akzeptierbaren Salze nach einem der Ansprüche 1 bis 4.

**8.** Als Medikament, mit Hemmwirkung auf Knochenresorption oder für die Behandlung oder Verhütung von Osteoporose, Verbindung mit der Formel (I) und/oder ihre physiologisch akzeptierbaren Salze nach einem der Ansprüche 1 bis 4.

**9.** Als Medikament, mit Hemmwirkung auf Tumorwachstum oder Krebsmetastasen, Verbindung mit der Formel (I) und/oder ihre physiologisch akzeptierbaren Salze nach einem der Ansprüche 1 bis 4.

**10.** Als Medikament, mit entzündungshemmender Wirkung oder für die Behandlung oder Verhütung von Herz-Kreislauf-Störungen, Restenose, Arteriosklerose, Nephropathien oder Retinopathien, Verbindung mit der Formel (I) und/oder ihre physiologisch akzeptierbaren Salze und/oder ihre Prodrugs nach einem der Ansprüche 1 bis 4.

**11.** Verwendung einer Verbindung mit der Formel (I) und/oder ihrer physiologisch akzeptierbaren Salzen nach einem der Ansprüche 1 bis 4 für die Herstellung von Medikamenten, die für die Verhütung oder Behandlung von Osteoporose vorgesehen sind.

**12.** Verwendung einer Verbindung mit der Formel (I) und/oder ihrer physiologisch akzeptierbaren Salzen nach einem der Ansprüche 1 bis 4 für die Herstellung von Medikamenten, die für die Hemmung von Tumorwachstum oder Krebsmetastasen vorgesehen sind

**13.** Verwendung einer Verbindung mit der Formel (I) und/oder ihrer physiologisch akzeptierbaren Salzen nach einem der Ansprüche 1 bis 4 für die Herstellung von Medikamenten, die für die Verhütung oder Behandlung von Herz-Kreislauf-Störungen, Restenose, Arteriosklerose, Nephropathien oder Retinopathien vorgesehen sind.

**14.** Verfahren zur Herstellung der Verbindungen mit der Formel (I), nach einem der Ansprüche 1 bis 4 wobei:

a) ein Produkt mit der allgemeinen Formel (IIa) zur Reaktion gebracht wird:

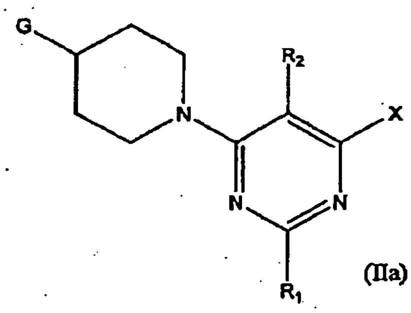

(IIa)

wobei $R^1$, $R^2$ und G den weiter oben gegebenen Definitionen entsprechen und X ein Halogenatom darstellt, mit einem Produkt mit der Formel (VI):

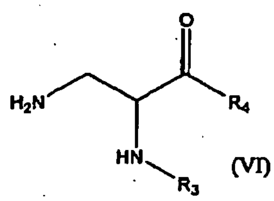

(VI)

wobei $R^3$ und $R^4$ den weiter oben gegebenen Definitionen entsprechen, entweder in Gegenwart einer starken Base oder durch Katalyse mit Palladium, um Produkt mit der Formel (I) zu erhalten:

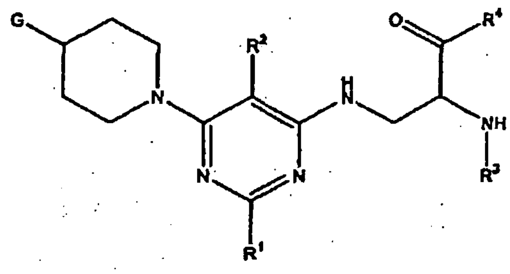

b) dann das Produkt mit der Formel (I) gegebenenfalls einer Hydrolyse und/oder Salzbildung ausgesetzt wird.

**15.** Verfahren nach Anspruch 14, wobei X ein Chloratom darstellt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 03799611 A
- WO 9412181 A
- WO 9408577 A
- EP 528586 A
- EP 528587 A
- WO 9532710 A
- WO 9600574 A
- WO 9600730 A
- WO 9800395 A
- WO 993245 A
- WO 993762 A
- EP 0820991 A
- EP 1065207 A
- WO 0078317 A


### Littérature non-brevet citée dans la description


- **Horton et al.** *Exp. Cell. Res.,* 1991, vol. 195, 368
- **Sato et al.** *J. Cell. Biol.,* 1990, vol. 111, 1713
- **Fisher et al.** *Endocrinology,* 1993, vol. 132, 1411
- **Brown et al.** *cardiovascular Res.,* 1994, vol. 28, 1815
- **Brook et al.** *Cell,* 1994, vol. 79, 1157
- **Cheresh et al.** *Science,* 1995, vol. 270, 1500
- **Greene ; Wuts.** protective Group in Organic Synthesis. Wiley, 1991
- Drug targeting, voir Targeted Drug Delivery. **R. C. Juliano et al.** Handbook of Experimental Pharmacology. Springer Verlag, vol. 100
- *J. Med. Chem.,* 2001, vol. 44 (8), 1158-1176
- **Pytella et al.** *Methods Enzymol.,* 1987, 144